# EUROPEAN PATENT APPLICATION

(11) **EP 4 755 909 A1**
(43) Date of publication of application: **10.06.2026**
(21) Application number: 24852230.2
(22) Date of filing: 02.08.2024
(51) Int. Cl.: C07K 16/22, C07K 16/28, A61P 35/00, A61K 39/00

(54) **CONJUGATE OF MODIFIED FUSION PROTEIN AND SCFV, AND USE THEREOF**

(30) Priority: 04.08.2023 KR 20230102074
(71) Applicant: Panolos Bioscience, Inc., Hwaseong-si Gyeonggi-do 18471 (KR)
(72) Inventor: LIM, Hyeseong, Seongnam-si, Gyeonggi-do 13567 (KR); YANG, Hyun Gul, Asan-si, Chungcheongnam-do 31579 (KR)
(74) Representative: Schulz Junghans Patentanwälte PartGmbB
(86) International application number: PCT/KR2024/011435
(87) International publication number: WO 2025/033876

(57) **Abstract**

The present disclosure relates to a conjugate of a multi-specific modified fusion protein and scFv that binds to an antigen of a binding scFv, a vascular endothelial growth factor, and a placental growth factor, and to a use thereof. The conjugate according to an embodiment of the present disclosure can also demonstrate excellent productivity and stability. The conjugate according to an embodiment of the present disclosure can exhibit excellent blood stability. The conjugate according to an embodiment of the present disclosure, can exhibit excellent antitumor, anti-fibrosis, and/or immune cell activation effects.

## Description

### TECHNICAL FIELD

The present disclosure relates to a conjugate of a modified fusion protein and a single-chain variable fragment (scFv) that binds to vascular endothelial growth factor, placenta growth factor, and an antigen to which the scFv binds, and uses thereof.

### BACKGROUND

Vascular endothelial growth factor (VEGF) is a signaling protein that plays an important role in vasculogenesis and angiogenesis. Its general functions include formation of new blood vessels during embryonic development, in muscles after injury or exercise, and new blood vessels that bypass blocked blood vessels; however, abnormal increases in VEGF are closely related to tumor development and metastasis (Carmeliet, P., Jain, R.K., 2000. Nature 407: pp. 249-257).

VEGF is classified into five types: VEGF-A, -B, -C, -D, and placenta growth factor (PlGF), which are known to contribute to angiogenesis (Carmeliet, P., Jain, R.K., 2000. Nature 407: pp. 249-257; Kuwano M, et al., 2001. Intern Med 40: pp. 565-572). On the other hand, there are three types of VEGF receptors, VEGF R1, R2, and R3, and binding affinity of ligands differ depending on the type of receptor. Among the receptors, VEGFR-1/-2 are known to be involved in angiogenesis (Veikkola et al., 2000. Cancer Research 60: pp. 203-212).

Continued efforts have been directed at developing drugs targeting angiogenesis inhibition through removal of vascular endothelial growth factor, which is known to be a key factor in tumorigenesis. Bevacizumab, a drug in the form of a monoclonal antibody-type drug targeting VEGF-A (Stacker et al., 2013. Chinese Journal of Cancer Research 32(6): pp. 297-302; Kazazi-Hyseni et al., 2010. Oncologist 15(8): pp. 819-825; DrugBank Accession Number: DB00112) is representative. The mechanism of action of monoclonal antibody drugs involves binding to a single vascular endothelial growth factor, and since multiple growth factors are involved in canceration, monoclonal antibodies were not effective drugs to show a remarkable efficacy. Bevacizumab actually induced increased expression of other types of growth factors that were not targeted, by intracellular compensatory mechanisms, resulting in the side effect of resistance (Bagley et al., 2011. Clinical Cancer Research 17(5): pp. 976-988; Lieu et al., 2013. PLoS One 8: e77117; Cutsem et al., 2020. Clinical Cancer Research 26(3): pp. 717-725). For these reasons, the need for development of drugs that can simultaneously multi-target angiogenic growth factors associated with canceration while also possessing targeting functions for cancer cells has gradually increased.

To overcome the shortcomings of monoclonal antibody drugs, drugs that target multiple vascular endothelial growth factors have been developed. Representative protein drugs are VEGF-Trap (Ciombor et al., 2013. Clinical Cancer Research 19(8): pp. 1920-1925; DrugBank Accession Number: DB08885) and VEGF-Grab (Lee et al., 2015. Mol. Cancer. Ther. 14(2): pp. 470-479). Both of these drugs mimic part of the decoy receptor, wherein VEGF-Trap is a heterologous protein in the form of VEGFR1 D2-VEGFR2 D3, and VEGF-Grab is a homologous protein in the form of VEGFR1 D2-D3, each being a recombinant fusion protein fused with the Fc of an immunoglobulin. Efforts to improve physicochemical properties of such fusion proteins targeting multiple vascular endothelial growth factors have continued.

It may be considered to bind an additional antigen-targeting single-chain variable fragment (scFv) to such fusion proteins, and among these, an scFv targeting programmed cell death protein ligand 1 (PD-L1), which is overexpressed in many cancer tissues, may be considered (Dong et al., 2002. Nature Medicine 8: 787-789). The programmed cell death protein 1 (PD-1) receptor and its ligand, programmed cell death ligand 1 (PD-L1), are immune checkpoint proteins implicated in suppression of immune system responses associated with chronic infection, pregnancy, tissue allografts, autoimmune diseases, and cancer. PD-L1 regulates immune responses by binding to the inhibitory receptor PD-1 expressed on the surface of T cells, B cells, and monocytes. PD-L1 also negatively regulates T-cell function through interaction with another receptor, B7-1. Formation of PD-L1/PD-1 and PD-L1/B7-1 complexes negatively regulates T-cell receptor signaling, resulting in subsequent downregulation of T-cell activation and suppression of anti-tumor immune activity.

Cancer tissues that overexpress PD-L1 include melanoma, cutaneous squamous cell carcinoma (CSCC), head and neck squamous cell carcinoma (HNSCC), non-small cell lung cancer, renal cancer, head and neck cancer, thyroid cancer, colon cancer, liver cancer, ovarian cancer, breast cancer, and pancreatic cancer.

Accordingly, there has been a need for development of a conjugate that exhibits excellent anticancer effects while enabling selective targeting based on high binding affinity to target substances within cancer cells, possessing an increased in vivo residence time, and satisfying high productivity at the level of protein therapeutics in terms of commercial-scale production, as well as having physical stability and chemical stability.

### DISCLOSURE

### TECHNICAL PROBLEM

Improved physicochemical properties of a protein can maximize production efficiency by enhancing the physical and chemical stability of the protein during expression and purification, and reduce non-specific binding, aggregation, and degradation when the protein is introduced into the body, thereby achieving high persistence in body. In the case of anticancer agents, such improvements increase selective targeting of cancer cells, resulting in excellent anticancer effects.

The inventors of the present disclosure developed modified fusion proteins by introducing, into a fusion protein containing VEGFR1 domain D2 and domain D3, an amino acid variation in a specific region of domain D3, length adjustment and variation of a linker, or a disulfide bond. Further, the present inventors developed a conjugate in which a PD-L1-targeting scFv is bound to the modified fusion protein, and confirmed that such a conjugate exhibits excellent productivity, stability, and anticancer efficacy.

### TECHNICAL SOLUTION

1. An embodiment of the present disclosure may be a conjugate in which a PD-L1-targeting scFv (anti-PD-L1 scFv) is bound to a VEGFR domain. The conjugate may bind, via the scFv, to a target expressed on the surface of cancer cells while simultaneously binding to VEGF and PlGF in the vicinity of the cancer cells, thereby inhibiting differentiation of vascular endothelial cells. This leads to inhibition of cancer growth by selectively suppressing angiogenesis around the cancer cells. The conjugate according to an embodiment of the present disclosure is a conjugate including a PD-L1-targeting scFv bound to a fusion protein comprising a VEGFR1 (vascular endothelial growth factor receptor 1) extracellular domain, a linker, and a multimerization domain, and may be characterized in that the PD-L1-targeting scFv is fused to the N-terminus of the fusion protein. In an embodiment of the present disclosure, the PD-L1-targeting scFv may specifically be an atezolizumab scFv, which is an anti-PD-L1 scFv, or a separately developed anti-PD-L1 scFv (A167 scFv; SEQ ID NO: 40).
2. An embodiment of the "fusion protein" of the present disclosure may relate to a modified fusion protein comprising a VEGFR1 extracellular domain, a linker, and a multimerization domain, wherein the VEGFR1 extracellular domain comprises an immunoglobulin (Ig)-like domain D2 and an Ig-like domain D3 of VEGFR1, the linker is located between the Ig-like domain D3 and the multimerization domain, and the modified fusion protein has one or more of the following characteristics (a) to (c): (a) one or more amino acid substitutions selected from K241E, L243S, R244V, and H246E on the β1-β2 loop of domain D3; amino acid substitution of L258A, L258S, or L258D on the β2-β3 loop of domain D3; and one or more amino acid substitutions selected from K300G, Q302T, and K304S on the β5-β6 loop of domain D3, wherein the β1-β2 loop of domain D3 includes amino acid residues T236 to T247 of the VEGFR1 amino acid sequence (e.g., the VEGFR1 amino acid sequence of Table 2; SEQ ID NO: 41), the β2-β3 loop includes amino acid residues T256 to V262 of the VEGFR1 amino acid sequence, and the β5-β6 loop includes amino acid residues D299 to L308 of the VEGFR1 amino acid sequence; (b) the linker has a length of about 14 to about 35 amino acids; and (c) a disulfide bond is present in the fusion protein, and among the amino acid residues on the β1-β2 loop of domain D3, L243 and one of amino acid residues located at positions -2, -1, 0, +1, +2, and +3 relative to Y329 of domain D3, particularly the residue 331 which is located at the +2 position, are substituted with cysteine.
3. In one embodiment, characteristic (a) of the modified fusion protein may comprise amino acid substitutions of K241E, L243S, R244V, and H246E on the β1-β2 loop of domain D3; amino acid substitution of L258A, L258S, or L258D on the β2-β3 loop of domain D3; and amino acid substitutions of K300G, Q302T, and K304S on the β5-β6 loop of domain D3. With respect to characteristic (a), the substitutions may be substitutions with amino acids that decrease the net pI of the protein compared to the residues before substitution, amino acids having negatively charged side chains, or amino acids whose side chains acquire electrostatic negative charges.
4. In one embodiment, the modified fusion protein may have an N-terminus of domain D2 starting with the amino acid sequence EF.
5. In one embodiment, with respect to characteristic (b), the linker of the modified fusion protein may comprise a GS repeat sequence, and the GS repeat sequence may be an amino acid sequence having a length of 2 to 35 consisting only of G and S.
6. In one embodiment, one or more glycine (G) residues of the GS repeat sequence included in the linker of the modified fusion protein may be substituted with cysteine (C).
7. In one embodiment, with respect to characteristic (b), the linker of the modified fusion protein may include an amino acid sequence of a hinge region derived from an immunoglobulin, and the amino acid sequence of the hinge region may be modified.
8. In one embodiment, the hinge region derived from an immunoglobulin may be derived from human IgD and/or IgG.
9. In one embodiment, with respect to characteristic (b), the modified fusion protein may have an amino acid variation at a papain recognition site or a glycosylation site present in the hinge region derived from an immunoglobulin. Specifically, the amino acid variation at the papain recognition site may be substitution of one or more amino acid residues present at the papain recognition site with alanine, serine, tyrosine, proline, or threonine, or insertion of an amino acid sequence of 1 to 10 amino acids including at least one amino acid having an aromatic carbon or cyclic carbon in its side chain at the papain recognition site. The amino acid variation at the glycosylation site may be (i) deletion of serine or threonine present in the hinge region derived from an immunoglobulin, or (ii) substitution thereof with an amino acid other than serine, asparagine, or threonine.
10. In one embodiment, with respect to characteristic (b), the linker of the modified fusion protein may sequentially include, from the N-terminus toward the C-terminus, (i) an amino acid sequence selected from the group consisting of CS, CSSG, CS(GGGGS), CS(GGGGS)3, C(GSSG)2, GS, GSSG, (GSSG)2, GGGGS, (GGGGS)4, GS(GGGGS), and GS(GGGGS)3, and (ii) a hinge region derived from an immunoglobulin selected from the group consisting of human IgG1, IgG4, or IgD/G1, wherein the amino acid sequence of the hinge region may be modified.
11. In one embodiment, with respect to characteristic (b), the linker of the modified fusion protein may be an amino acid sequence selected from the group consisting of CSSGDATPTSPPSP (SEQ ID NO: 68), CSKVDKKVEPKSSDTPPTCPPCP (SEQ ID NO: 69), CSGGGGSAEPKAGDATPPTCPPCP (SEQ ID NO: 70), CSGGGGSGGGGSGGGGSAESKYGPPCPPCP (SEQ ID NO: 71), CSNTGSGGEEKKKEKEKEEQEERSSDTPPTCPPCP (SEQ ID NO: 72), CSNTGSGGEEKKKEKEKEEQEERSCDTPPTCPPCP (SEQ ID NO: 73), CGSSGGSSGEPKSDATPTCPPCP (SEQ ID NO: 74), and CSKVDKKVEPKSSDKTYTCPPCP (SEQ ID NO: 75).
12. In one embodiment, the modified fusion protein may include (a) amino acid substitutions of K241E, L243S, R244V, and H246E on the β1-β2 loop of domain D3, amino acid substitution of L258A, L258S, or L258D on the β2-β3 loop of domain D3, and amino acid substitutions of K300G, Q302T, and K304S on the β5-β6 loop of domain D3; (b) a linker that is an amino acid sequence selected from the group consisting of CSSGDATPTSPPSP, CSKVDKKVEPKSSDTPPTCPPCP, CSGGGGSAEPKAGDATPPTCPPCP, CSGGGGSGGGGSGGGGSAESKYGPPCPPCP, CSNTGSGGEEKKKEKEKEEQEERSSDTPPTCPPCP, CSNTGSGGEEKKKEKEKEEQEERSCDTPPTCPPCP, CGSSGGSSGEPKSDATPTCPPCP, and CSKVDKKVEPKSSDKTYTCPPCP; and (c) substitution of L243 and the amino acid residue located at the +2 position relative to Y329 of domain D3 with cysteine.
13. In one embodiment, the modified fusion protein may be composed of an amino acid sequence selected from the group consisting of the amino acid sequences of C61 to C75 of Table 2 (SEQ ID NOS: 44 to 58).
14. In one embodiment, the multimerization domain may be (a) an Fc region of an immunoglobulin; (b) a CH3 region of IgG1 or IgG4; (c) CH2 and CH3 regions of IgG1 or IgG4; (d) an Fc region of an immunoglobulin including an amino acid sequence having at least 85% identity with the amino acid sequence of C88 of Table 2 (SEQ ID NO: 59); or (e) an Fc region of an immunoglobulin including an amino acid sequence of an Fc region (e.g., SEQ ID NO: 60).
15. In one embodiment, the multimerization domain may include or consist of an Fc region of IgG1 composed of the amino acid sequence of SEQ ID NO: 60. The multimerization domain may have one or more selected from (i) T20Q amino acid substitution, D126E amino acid substitution, L128M amino acid substitution, M198L amino acid substitution, and deletion of K217 relative to SEQ ID NO: 60, or (ii) L4A amino acid substitution, L5A amino acid substitution, H38Q amino acid substitution, K44Q amino acid substitution, Y66F amino acid substitution, A97G amino acid substitution, A100S amino acid substitution, P101S amino acid substitution, R125Q amino acid substitution, D126E amino acid substitution, L128M amino acid substitution, K179R amino acid substitution, Q189E amino acid substitution, P215L amino acid substitution, and deletion of K217.
16. In one embodiment, the modified fusion protein may be in dimer or multimer form.
17. An embodiment of the present disclosure relates to a conjugate of the modified fusion protein and scFv according to an embodiment of the present disclosure, wherein the scFv may be an atezolizumab scFv or an A167 scFv (Table 9; SEQ ID NO: 39 or 40) that binds to PD-L1.
18. An embodiment of the present disclosure may relate to a pharmaceutical composition for preventing or treating chronic infection, tissue allograft, autoimmune disease, inflammatory disease, neoplastic disease, cancer, angiogenesis-related disease, or ocular disease, comprising, as an active ingredient, the conjugate of the modified fusion protein and scFv according to an embodiment of the present disclosure.
19. An embodiment of the present disclosure may relate to a nucleic acid molecule encoding the conjugate of the modified fusion protein and scFv according to an embodiment of the present disclosure.
20. An embodiment of the present disclosure may relate to a host cell including a nucleotide sequence encoding the conjugate of the modified fusion protein and scFv according to an embodiment of the present disclosure.
21. An embodiment of the present disclosure may relate to a vector including a nucleotide sequence encoding the conjugate of the modified fusion protein and scFv according to an embodiment of the present disclosure.
22. In one embodiment, the vector may be a recombinant viral vector.
24. An embodiment of the present disclosure may relate to a pharmaceutical composition for delivering a viral vector to a subject, comprising a recombinant viral vector according to an embodiment of the present disclosure, wherein the conjugate of the fusion protein and scFv encoded by the recombinant viral vector is expressed in the subject, for preventing or treating chronic infection, tissue allograft, autoimmune disease, inflammatory disease, neoplastic disease, cancer, angiogenesis-related disease, or ocular disease.
25. In one embodiment, the recombinant viral vector may be a recombinant adeno-associated virus vector.
26. An embodiment of the present disclosure may relate to a method for preventing or treating one or more selected from the group consisting of chronic infection, tissue allograft, autoimmune disease, inflammatory disease, neoplastic disease, cancer, angiogenesis-related disease, or ocular disease, by administering, to a subject in need thereof, the conjugate of the modified fusion protein and scFv according to an embodiment of the present disclosure, and/or a recombinant viral vector including a nucleotide sequence encoding the conjugate of the modified fusion protein and scFv according to an embodiment of the present disclosure.
27. An embodiment of the present disclosure may relate to a use of the conjugate of the modified fusion protein and scFv according to an embodiment of the present disclosure, and/or a recombinant viral vector including a nucleotide sequence encoding the conjugate of the modified fusion protein and scFv according to an embodiment of the present disclosure, for preventing or treating one or more selected from the group consisting of chronic infection, tissue allograft, autoimmune disease, inflammatory disease, neoplastic disease, cancer, angiogenesis-related disease, or ocular disease.

### ADVANTAGEOUS EFFECTS

The conjugate of the modified fusion protein and the anti-PD-L1 scFv according to an embodiment of the present disclosure may exhibit excellent productivity, physical stability, and chemical stability.

The conjugate of the modified fusion protein and the anti-PD-L1 scFv according to an embodiment of the present disclosure may exhibit excellent blood stability.

The conjugate of the modified fusion protein and the anti-PD-L1 scFv according to an embodiment of the present disclosure may exhibit excellent binding affinity to VEGF, PlGF, and PD-L1.

The conjugate of the modified fusion protein and the anti-PD-L1 scFv according to an embodiment of the present disclosure may exhibit excellent simultaneous binding affinity to the antigens of the modified fusion protein and the scFv, respectively.

The conjugate of the modified fusion protein and the anti-PD-L1 scFv according to an embodiment of the present disclosure may exhibit excellent inhibitory effects on the respective antigens of the modified fusion protein and the scFv.

The conjugate of the modified fusion protein and the anti-PD-L1 scFv according to an embodiment of the present disclosure may have acidic properties and may exhibit an increased ratio of charge variants in the acidic and neutral regions of the pI distribution. Accordingly, the modified fusion protein can have the effect of improved non-specific interactions with a matrix and a cell surface in the body.

The conjugate of the modified fusion protein and the anti-PD-L1 scFv according to an embodiment of the present disclosure may exhibit excellent anticancer effects.

The conjugate of the modified fusion protein and the anti-PD-L1 scFv according to an embodiment of the present disclosure may inhibit activation of cancer-associated fibroblasts (CAFs) expressing CD141 by lowering PlGF levels in a tumor microenvironment (TME), thereby inhibiting fibrogenesis. This lowers vascular compression and, together with lowering VEGF-A levels, normalizes blood vessels. Normalized blood vessels and improvement of peritumoral fibrosis increase drug accessibility and increase immune cell infiltration, thereby exhibiting an effect of inhibiting tumor growth.

The conjugate of the modified fusion protein and the anti-PD-L1 scFv according to an embodiment of the present disclosure may exhibit anti-tumor, anti-fibrotic, and/or immune cell activation effects.

### DESCRIPTION OF DRAWINGS

FIG. 1 is a diagram illustrating, in a 2D structure, a form in which an anti-PD-L1 scFv is bound to a modified VEGFR1 D2-D3 domain (the modified fusion protein of the present disclosure).
FIGs. 2a to 2o are graphs showing productivity and purity of a conjugate of an anti-PD-L1 scFv and a modified fusion protein.
FIGs. 3a to 3c are graphs showing serum stability of a conjugate of an anti-PD-L1 scFv and a modified fusion protein that binds to vascular endothelial growth factor, placenta growth factor, and PD-L1 (control: a fusion protein based on VEGF-Grab (H-12C08 or prototype) and PB101 (VEGF-Grab)).
FIGs. 4a to 4k are graphs showing binding affinity to VEGF-A, PlGF, and PD-L1 of a conjugate of an anti-PD-L1 scFv and a modified fusion protein that binds to vascular endothelial growth factor, placenta growth factor, and PD-L1 (control: a prototype conjugate based on an anti-PD-L1 scFv and VEGF-Grab).
FIGs. 5a to 5f are graphs showing simultaneous binding affinity to targets of a conjugate of an anti-PD-L1 scFv and a modified fusion protein that binds to vascular endothelial growth factor, placenta growth factor, and PD-L1.
FIGs. 6a to 6o are graphs showing, at a cellular level, signal transduction inhibitory effects on VEGF-A and PD-L1 of the conjugate (PB203) of the anti-PD-L1 scFv and the modified fusion protein of the present disclosure that binds to vascular endothelial growth factor, placenta growth factor, and PD-L1 (controls: VEGF-Grab/PB101; H-12C18; H-30D01; Tecentriq; anti-PD-1 antibody; modified fusion protein/backbone/PB102).
FIGs. 7a to 7r are graphs showing an isoelectric point measurement of the conjugate according to the present disclosure (control: PB101/PD20A01).
FIGs. 8a to 8d are graphs showing thermal stability analysis of the conjugate according to the present disclosure.
FIGs. 9a and 9b are PK analysis graphs of the conjugate according to the present disclosure.
FIG. 10 is a graph showing tumor growth inhibition efficacy, in a mouse model, of the conjugate of the anti-PD-L1 scFv and the modified fusion protein of the present disclosure that binds to vascular endothelial growth factor, placenta growth factor, and PD-L1.

### MODE FOR INVENTION

The various embodiments or examples described in this document are exemplary and explanatory only and are not restrictive of the technical idea of the present disclosure. The technical idea of the present disclosure encompasses various modifications, equivalents, and alternatives of each embodiment or example described in this document as well as selective combinations of all or part of individual embodiments or examples.

Unless defined otherwise, all technical and scientific terms used herein have the same meaning as commonly understood by one of ordinary skill in the art to which the present disclosure pertains.

Use of the singular herein may include the plural unless the context dictates otherwise, and this also applies to singular expressions recited in the claims.

### I. Definitions

In the present disclosure, the term "about" may indicate a typical error range for a particular value recited, as is well known to one of ordinary skill in the art. When used in the context of numerical values or ranges stated in this disclosure, it may mean ±20%, ±15%, 10%, ±9%, ±8%, ±7%, ±6%, ±5%, ±4%, ±3%, ±2%, or ±1% of a numerical value or range that is recited in an embodiment or claimed. With respect to the length of a nucleotide or amino acid sequence, "about" can indicate that the nucleic acid or protein is not limited to the recited number of nucleotides or amino acids, and a few nucleotides or amino acids added to or removed from either terminus may be included so long as the functional activity is not impeded.

As used herein, expressions such as "comprising," "including," "having," and the like should be construed as open-ended terms that imply the possibility of inclusion of other embodiments in a similar manner to "comprising," unless stated otherwise in the phrase or sentence in which the expressions are included.

As used herein, the term "and/or" may mean, with respect to items associated with the term, any one or more of the items, any combination of the items, or all of the items.

As used herein, the term "amino acid" may refer to all naturally occurring L-α-amino acid. This definition is meant to include norleucine (Nle), ornithine, and homocysteine.

As used herein, the term "variation" or "amino acid variation" may refer to a substitution, an insertion, a deletion, or a combination thereof in an amino acid sequence as compared to a reference (e.g., native sequence) polypeptide or protein, and the term "variant" may refer to a molecule that has some differences in its amino acid sequence as compared to a reference polypeptide or protein due to such variations.

The scope of variants herein also includes proteins or fragments or derivatives thereof which exhibit the same or similar biological activity and derivatives which are differentially modified during or after translation, e.g., by glycosylation, proteolytic cleavage, linkage to an antibody molecule or other cellular ligand, and so on.

As used herein, the term "carriers" may include pharmaceutically acceptable carriers, excipients, or stabilizers which are non-toxic to cells or mammals at the dosages and concentrations employed. Often the pharmaceutically acceptable carrier is an aqueous pH buffered solution. Examples of pharmaceutically acceptable carriers include buffers such as phosphate, citrate, and other organic acids; antioxidants including ascorbic acid; low molecular weight (less than about 10 residues) polypeptides; proteins such as serum albumin, gelatin, or immunoglobulins; hydrophilic polymers such as polyvinylpyrrolidone; amino acids such as glycine, glutamine, asparagine, arginine, or lysine; monosaccharides, disaccharides, and other carbohydrates, including glucose, mannose, or dextrins; chelating agents such as EDTA; sugar alcohols such as mannitol or sorbitol; salt-forming counterions such as sodium; and/or nonionic surfactants such as TWEEN^{®}, polyethylene glycol (PEG), and PLURONICS^{®}, but are not limited thereto.

As used herein, the term "effective amount" may refer to an amount sufficient to effect beneficial or desired clinical or biochemical results. An effective amount can be administered in one or more administrations. An effective amount may refer to an amount that is sufficient to palliate, ameliorate, stabilize, slow or delay the progression of a disease state.

As used herein, the term "ligand" may refer to a molecule (e.g., VEGF-A, -B, -C, -D, and PlGF) which binds with high affinity to a modified fusion protein. In other contexts, "ligand" can mean any molecule or agent, or compound that binds specifically to a molecule such as a polypeptide or protein covalently or transiently.

As used herein, the term "individual" or "subject" may refer to a mammal. The mammal includes, but is not limited to, domestic animals (e.g., cattle, sheep, cats, dogs, and horses), primates (e.g., humans and non-human primates such as monkeys), rabbits, and rodents (e.g., mice and rats). In one embodiment, the individual or subject may be a human.

As used herein, the term "pharmaceutically acceptable carrier and/or diluent" may include, but is not limited to, any and all solvents, dispersion media, coating agents, antibacterial and antifungal agents, isotonic agents, and adsorption delaying agents and the like.

An embodiment of this disclosure described herein is to be understood to encompass "comprising," "consisting of," and/or "consisting essentially of" the embodiment.

### II. Modified fusion proteins and components of modified fusion proteins

### 1. Vascular endothelial growth factor receptor (VEGFR)

In one embodiment, a modified fusion protein of the present disclosure may comprise a VEGFR1 extracellular domain as its component. VEGFR1 is also known as fms-related tyrosine kinase (FLT-1) and is encoded by the *FLT1* gene. The amino acid sequence of VEGFR1 is presented as the VEGFR1 amino acid sequence of Table 2, which can be found in UniProtKB, #P17948. In one embodiment, the VEGFR1 is from a mammal such as a human. VEGFR1 has seven immunoglobulin (Ig)-like domains numbered 1, 2, 3, 4, 5, 6, and 7 starting from the N terminus to the C terminus of the extracellular region, as is also the case for VEGFR2 and VEGFR3.

In one embodiment, the VEGFR1 extracellular domain may comprise an Ig-like domain D2 and an Ig-like domain D3. As used herein, the term "Ig-like domain D2" of VEGFR1 refers to the second Ig-like domain found at the N terminus of the extracellular region of VEGFR1, and the "Ig-like domain D3" of VEGFR1 refers to the third Ig-like domain found at the N terminus of the extracellular region of VEGFR1, but these terms are to encompass variants as long as their functionality (e.g., binding to VEGF ligands and/or inhibiting activation of the VEGFR pathway) is maintained. Since there may be differences in the amino acid sequence of the active protein depending on the species, the Ig-like domains D2 and D3 of VEGFR1 are not limited by their origin or sequence and may include wild type or active variants thereof.

In one embodiment, the modified fusion protein of the present disclosure may further comprise other VEGFR extracellular domains in addition to the Ig-like domain D2 and Ig-like domain D3 of VEGFR1.

While not wishing to be bound by theory, it is thought that the extracellular domain of VEGFR inhibits activation of the VEGF pathway by binding to a VEGF ligand, thereby blocking the interaction between the VEGF ligand and VEGFR. Additionally, while not wishing to be bound by theory, it is thought that the extracellular domain of VEGFR may bind to VEGFR for dominant negative inhibition of the VEGF signaling pathway. In one embodiment, the extracellular domain of VEGFR is capable of binding to one or more VEGF ligands selected from the group consisting of VEGF-A, VEGF-B, VEGF-C, VEGF-D, and PlGF.

In one embodiment, the VEGFR extracellular domain may or may not comprise a signal peptide which acts as a signal sequence for secretion of the VEGFR extracellular domain or a modified fusion protein comprising the same from a host cell. This signal peptide can be operably linked to a nucleic acid encoding a protein of interest (e.g., extracellular domains of VEGFR1).

### 2. Linker

Components of a modified fusion protein (e.g., extracellular domains of VEGFR1 or a multimerization domain) may be connected by a linking moiety such as a peptide linker. The linkers increase flexibility of the fusion protein components and do not significantly interfere with the structure of each functional component within the fusion protein.

In one embodiment, a linker can be used to connect the C-terminal end of VEGFR1 extracellular domains (e.g., C-terminus of the Ig-like domain D3) to the N-terminal end of a multimerization domain (e.g., IgG1 Fc region).

In one embodiment, the linker may comprise an amino acid sequence of a hinge region derived from an immunoglobulin. The hinge region derived from an immunoglobulin may include a hinge region present in the N-terminal region of the Fc region of the immunoglobulin. In one embodiment, the hinge region derived from an immunoglobulin may be derived from human IgD or IgG.

### 3. Multimerization domain

The present disclosure provides a multimerization domain (e.g., the Fc region of an immunoglobulin) that can be a component of any modified fusion protein. A multimerization domain is a portion of a multimeric protein that promotes the association of subunits to form, for example, a dimer, a trimer, a tetramer, and the like. As used herein, the term "multimerization domain" may be used to refer to a dimerization domain, trimerization domain, tetramerization domain, and the like. A fusion protein comprising a multimerization domain can interact with another fusion protein comprising a multimerization domain to generate a fusion protein multimer (e.g., a fusion protein dimer). For example, an IgG Fc region is a dimerization domain that can be fused with the extracellular domains of VEGFR1 described herein. A fusion protein comprising extracellular domains of VEGFR1 and an IgG Fc region can dimerize with another fusion protein comprising an IgG Fc region, creating a fusion protein dimer that can simultaneously bind to a VEGF ligand and a PlGF ligand.

In one embodiment, the Fc region may be partially glycosylated or not glycosylated at all.

In one embodiment, the Fc region may have amino acid substitution T20Q, amino acid substitution D126E, amino acid substitution L128M, amino acid substitution M198L, and/or amino acid deletion of K217 based on SEQ ID NO:60. These amino acid variations can increase the half-life of the fusion protein through interaction with FcRn. Said amino acid variations may appear identically in other immunoglobulin Fc regions having a sequence corresponding to the amino acid sequence of SEQ ID NO: 60, and the modified fusion protein of the present disclosure may comprise another immunoglobulin Fc region having the amino acid variations as the multimerization domain component.

In one embodiment, the Fc region may have amino acid substitution L4A, amino acid substitution L5A, H38Q amino acid substitution, K44Q amino acid substitution, Y66F amino acid substitution, A97G amino acid substitution, A100S amino acid substitution, P101S amino acid substitution, R125Q amino acid substitution, D126E amino acid substitution, L128M amino acid substitution, K179R amino acid substitution, Q189E amino acid substitution, P215L amino acid substitution, and/or K217 amino acid deletion based on SEQ ID NO: 60. Through such amino acid variations, effector functions of the Fc region may be eliminated. The amino acid variations may likewise be present in other immunoglobulin Fc regions having sequences corresponding to the amino acid sequence of SEQ ID NO: 60, and the modified fusion protein of the present disclosure may include, as a multimerization domain component, another immunoglobulin Fc region having said amino acid variations.

Below, characteristics of a modified fusion protein according to the present disclosure are described.

### 4. Characteristics of Surface Charge Transition Variations

In one embodiment, a modified fusion protein of the present disclosure may have surface charge transition variations as a characteristic. In the present disclosure, modified fusion proteins having the characteristics of surface charge transition variations characteristic may be used interchangeably as "surface charge transition variants" or "surface charge variants."

In the characteristics of surface charge transition variations according to one embodiment, a modified fusion protein may be substituted with amino acids that decrease the net pI of the protein, amino acids having negatively charged side chains, or amino acids whose side chains acquire electrostatic negative charges, through amino acid substitutions of K241E, L243S, R244V, and H246E on the β1-β2 loop of Ig-like domain D3, amino acid substitution of L258A, L258S, or L258D on the β2-β3 loop of domain D3, and/or amino acid substitutions of K300G, Q302T, and K304S on the β5-β6 loop of domain D3. In one embodiment, an amino acid having an electronegative side chain may mean that the amino acid turns electronegative depending on the nature of adjacent amino acid residues or molecules after substitution, and may refer to a relative concept arising from the electron distribution within the molecule. For example, amino acid residues containing a hydroxy group (-OH) can turn electronegative due to the lone pair of electrons present on the oxygen atom, and this phenomenon can reduce the net pI of the protein.

In one embodiment, the β1-β2 loop of Ig-like domain D3 may comprise amino acid residues T236 to T247 of the VEGFR1 amino acid sequence of Table 2. In one embodiment, the β2-β3 loop of Ig-like domain D3 may comprise amino acid residues T256 to V262 of the VEGFR1 amino acid sequence of Table 2. In one embodiment, the β5-β6 loop of Ig-like domain D3 may comprise amino acid residues D299 to L308 of the VEGFR1 amino acid sequence of Table 2. In the present disclosure, the β1-β2 loop may be referred to as "Site 1," the β2-β3 loop may be referred to as "Site 2," and the β5-β6 loop may be referred to as "Site 3." The inventors of the present disclosure ascertained that amino acid residues present in said loops are exposed on the surface of domain D3, and have achieved surface charge stabilization and structure stabilization of modified fusion proteins through variations of amino acid residues present in the loops.

In one embodiment, the amino acid residues present in Site 1 and Site 3 affect each other through interactions between amino acid residues due to their structural positions, and accordingly, double, triple or quadruple amino acid variation (e.g. amino acid substitutions) may be introduced at each of the Sites.

In one embodiment, the three loops should be understood to encompass amino acid sequence variants that maintain their function. For example, in one embodiment, the β1-β2 loop, β2-β3 loop and β5-β6 loop may comprise an amino acid sequence having at least 85%, at least 86%, at least 87%, at least 88%, at least 89%, at least 90%, least 91%, at least 92%, at least 93%, at least 94%, at least 95%, at least 96%, at least 97%, at least 98%, or at least 99% sequence identity to the amino acid sequences TPRPVKLLRGHT, TPLNTRV and DKMQNKDKGL, respectively.

In the linker variation characteristic according to one embodiment, a modified fusion protein may have one or more amino acid residues on a β1-β2 loop of Ig-like domain D3 and one or more amino acid residues on a β5-β6 loop of domain D3 substituted with an amino acid that reduces the net pI of a protein, an amino acid having a negatively charged side chain, or an amino acid having an electronegative side chain.

In one embodiment, the N terminus of Ig-like domain D2 of a modified fusion protein may be further modified. A modified fusion protein of the present disclosure can be produced by inserting the nucleic acid sequence or nucleic acid molecule encoding the same into vectors of various origins. In this case, depending on the type or origin of the vector into which the nucleic acid sequence or nucleic acid molecule is inserted, variations may occur in the amino acid sequence at the N terminus the Ig-like domain D2, with the N terminus starting from an amino acid sequence derived from the vector (e.g., amino acid sequence EF) rather than beginning with G132. However, such variation of the N-terminal sequence by a vector-derived amino acid sequence may not affect the productivity or physicochemical properties of a modified fusion protein of the present disclosure.

### 5. Linker variation characteristic

In one embodiment, a modified fusion protein of the present disclosure may have linker variations as a characteristic. In the present disclosure, modified fusion proteins having the linker variation characteristic may be used interchangeably as "linker variants." Due to the linker variation characteristic, (i) a modified fusion protein of the present disclosure can closely mimic the structure in which domain D2 and domain D3 among the native VEGFR1 extracellular domains bind to a ligand, (ii) domain D3 among the native VEGFR1 extracellular domains can have a length spanning to the dimer formation site of domain D4 as a structural characteristic to be secured for smooth ligand binding, and (iii) the linker itself can have excellent structural stability.

In the linker variation characteristic according to one embodiment, a linker may have a length of about 14 to about 35 amino acids.

In the linker variation characteristic according to one embodiment, a linker may comprise a GS repeat sequence.

In the linker variation characteristic according to one embodiment, the GS repeat sequence may be selected from the group consisting of GS, GSSG, (GSSG)₂, GGGGS, (GGGGS)₃, GS(GGGGS), and GS(GGGGS)₃, but is not limited thereto.

In the linker variation characteristic according to one embodiment, a linker may comprise an amino acid sequence of a hinge region derived from an immunoglobulin. In one embodiment, the amino acid sequence of the hinge region may be modified.

In the present disclosure, a linker is located between the C-terminus of VEGFR1 domain D3 and the Fc region of a human immunoglobulin produced by papain digestion and may refer to the entire region extending between P243 located at the N-terminus of the CH2 region (A244-K360) and the C-terminus of domain D3. The positions of amino acid residues in the Fc region and the CH2 region included in the Fc region follow the Kabat numbering scheme (Kabat et. al., Sequences of Proteins of Immunological Interest 5th Ed., US Department of Health and Human Services, NIH Publication No. 91-3242, 1991). In one embodiment, the linker may include some region derived from a human immunoglobulin, and the region may be the amino acid sequence of CH1 (K218-V223) or the hinge region (E226-P243), or a portion of such amino acid sequence.

In the present disclosure, when a linker comprises the amino acid sequence of a hinge region derived from immunoglobulin, characteristics of the included hinge region are retained in the linker. For example, the hinge region of an Fc region has cleavage sites for various proteases and can cause oligomerization after being introduced into a fusion protein, through O-glycosylation at Ser/Thr amino acids (Song et. al., 2020. Comput Struct Biotechnol J. 18: pp. 3925-3935). In a modified fusion protein having the linker variation characteristic of the present disclosure, the amino acid sequence of the hinge region derived from an immunoglobulin was modified to achieve excellent structural stability of the linker itself and improve physicochemical properties.

In the linker variation characteristic according to one embodiment, the hinge region derived from an immunoglobulin may comprise a sequence derived from the CH1 region, an upper hinge, and/or a core hinge. In one embodiment, some amino acid sequence derived from the CH1 region of an immunoglobulin may form a beta-strand or beta-sheet. In one embodiment, the hinge region derived from an immunoglobulin may be derived from human IgD, IgG, or a combination thereof.

In one embodiment, when the hinge region is derived from IgG1, the amino acid sequence KTHT of the papain recognition site having a variation may have been modified into an amino acid sequence selected from the group consisting of KTYT, TPP, ATPT, and ATPPTCP.

In the linker variation characteristic according to one embodiment, a linker may comprise, from the N-terminus toward the C-terminus, (i) an amino acid sequence selected from the group consisting of GS, GSSG, (GSSG)₂, GGGGS, GS(GGGGS) and GS(GGGGS)₃; and (ii) a hinge region derived from an immunoglobulin selected from the group consisting of human IgG1, IgG4, or IgD/G1, and the amino acid sequence of the hinge region may be modified.

In the present disclosure, a hinge region derived from IgD/G1 may refer to a combination of the hinge sequences of IgD and IgG1. Specifically, a hinge region derived from IgD/G1 may be NTGSGGEEKKKEKEKEEQEERSSDKTHTCPPCP (SEQ ID NO: 76). In one embodiment, the hinge sequence of IgD may be NTGSGGEEKKKEKEKEEQEERSS (SEQ ID NO: 77), NTGRGGEEKKKEKEKEEQEER (SEQ ID NO: 78), or a variant thereof. In one embodiment, the hinge sequence of IgG1 may be DKTHTCPPCP (SEQ ID NO: 79) or a variant thereof.

In the linker variation characteristic according to one embodiment, a linker may be an amino acid sequence selected from the group consisting of CSSGDATPTSPPSP (SEQ ID NO: 68), CSKVDKKVEPKSSDTPPTCPPCP (SEQ ID NO: 69), CSGGGGSAEPKAGDATPPTCPPCP (SEQ ID NO: 70), CSGGGGSGGGGSGGGGSAESKYGPPCPPCP (SEQ ID NO: 71), CSNTGSGGEEKKKEKEKEEQEERSSDTPPTCPPCP (SEQ ID NO: 72), CSNTGSGGEEKKKEKEKEEQEERSCDTPPTCPPCP (SEQ ID NO: 73), CGSSGGSSGEPKSDATPTCPPCP (SEQ ID NO: 74), and CSGGGGSAEPKAGDATPPTCPPCPPCP (SEQ ID NO: 75).

### 6. Disulfide bond variation characteristic

In one embodiment, a modified fusion protein of the present disclosure may have disulfide bond variations as a characteristic. In the present disclosure, modified fusion proteins having the disulfide bond variation characteristic may be used interchangeably as "disulfide bond variants." Since a modified fusion protein of the present disclosure is a fusion of heterologous proteins, stability is expected to be reduced at the site where different types of proteins are connected. Accordingly, the disulfide bond variation characteristic was introduced to prevent physical cleavage of the modified fusion protein and improve its stability.

To introduce the disulfide bond variation characteristic, candidate amino acid residues were selected using a tertiary structure energy prediction program (FoldX in YASARA, Schymkowitz et al., 2005, Nucleic Acids Research. 33: W382-388), and these amino acid residues were substituted with cysteine to ascertain that the formation of a disulfide bond leads to improved properties (e.g., productivity, physicochemical properties, ligand binding) of modified fusion proteins (*see* Example 2).

In one embodiment, for the disulfide bond variation characteristic, (i) one of the amino acid residues on the β1-β2 loop of Ig-like domain D3 or one of the amino acid residues on the β5-β6 loop of Ig-like domain D3, and (ii) an amino acid residue at position +2 relative to Y329 of Ig-like domain D3 may be substituted with cysteine. By such substitutions, a modified fusion protein of the present disclosure may have a disulfide bond in the protein. In one embodiment, the amino acid residues present at the positions described in (i) and (ii) above for introduction of the disulfide bond variation characteristic may be selected according to the above criteria (1) to (3).

### 7. Fusion protein

In one embodiment of the present disclosure, a modified fusion protein capable of simultaneously binding a VEGF ligand and a PlGF ligand can be provided. In one embodiment, the modified fusion protein can have a first binding specificity for a VEGF ligand and a second binding specificity for a PlGF ligand. Here, the VEGF ligand may be one or more of VEGF-A, VEGF-B, VEGF-C, and VEGF-D, and may specifically be VEGF-A.

In the present disclosure, a fusion protein including D2 and D3 of VEGFR1 based on a VEGFR1 wild-type protein used as a control may be referred to as PB101 (a fusion protein including D2 of VEGFR1 and D3 glycosylated with 1 to 3 amino acids, based on a VEGFR1 wild-type protein) or VEGF-Grab.

In one embodiment, the modified fusion protein may comprise a VEGFR1 extracellular domain, a linker, and a multimerization domain. In one embodiment, the modified fusion protein may comprise a VEGFR1 extracellular domain, a linker, and a multimerization domain, as components arranged in that order from the N terminus to the C terminus. In one embodiment, the VEGFR1 extracellular domain may comprise Ig-like domain D2 and Ig-like domain D3 of VEGFR1.

In one embodiment, a modified fusion protein may have (a) surface charge transition characteristic, (b) linker variation characteristic, and (c) disulfide bond variation characteristics.
(a) amino acid substitutions of K241E, L243S, R244V, and H246E on the β1-β2 loop of domain D3, amino acid substitution of L258A, L258S, or L258D on the β2-β3 loop of domain D3, and/or amino acid substitutions of K300G, Q302T, and K304S on the β5-β6 loop of domain D3 are introduced;
(b) the linker is an amino acid sequence selected from the group consisting of CSSGDATPTSPPSP (SEQ ID NO: 68), CSKVDKKVEPKSSDTPPTCPPCP (SEQ ID NO: 69), CSGGGGSAEPKAGDATPPTCPPCP (SEQ ID NO: 70), CSGGGGSGGGGSGGGGSAESKYGPPCPPCP (SEQ ID NO: 71), CSNTGSGGEEKKKEKEKEEQEERSSDTPPTCPPCP (SEQ ID NO: 72), CSNTGSGGEEKKKEKEKEEQEERSCDTPPTCPPCP (SEQ ID NO: 73), CGSSGGSSGEPKSDATPTCPPCP (SEQ ID NO: 74), and CSKVDKKVEPKSSDKTYTCPPCP (SEQ ID NO: 75); and
(c) L243 amino acid residue on the β1-β2 loop of domain D3 is substituted with cysteine, and an amino acid residue at position +2 relative to Y329 of domain D3 is substituted with cysteine.

In one embodiment, the modified fusion protein may comprise an amino acid sequence selected from the group consisting of the amino acid sequences of C61 to C75 of Table 2. In one embodiment, the modified fusion protein may consist of an amino acid sequence selected from the group consisting of the amino acid sequences of C61 to C75 of Table 2.

In one embodiment, the modified fusion protein may have at least 85%, at least 86%, at least 87%, at least 88%, at least 89%, at least 90%, least 91%, at least 92%, at least 93%, at least 94%, at least 95%, at least 96%, at least 97%, at least 98%, or at least 99% sequence identity to an amino acid sequence selected from the group consisting of the amino acid sequences of C61 to C75 of Table 2. Such variants may comprise amino acid variations (e.g., conservative amino acid substitutions) in additional amino acid sequences while retaining or maintaining the amino acid variations defined in the characteristics of a modified fusion protein of the present disclosure.

In one embodiment, the components of a modified fusion protein of the present disclosure (a VEGFR1 extracellular domain, a linker, and a multimerization domain) may carry post-translational modifications including, for example, glycosylation, sialylation, acetylation, and phosphorylation.

In one embodiment, a modified fusion protein of the present disclosure may contain conservative amino acid substitutions at positions other than those of the amino acid variations defined in the characteristics described in the present disclosure. These conservative amino acid substitutions can be introduced into any of the individual components of a modified fusion protein of the present disclosure (e.g., a VEGFR1 extracellular domain, a linker, and a multimerization domain).

In one embodiment, conservative substitutions that may be included in the modified fusion protein of the present disclosure may be as shown in Table 0 below. Through such conservative substitutions, desired activity, for example, maintained/improved binding affinity to a target, reduced immunogenicity, or improved serum stability/thermal stability, may be obtained for the modified fusion protein of the present disclosure.

**[TABLE 0]**

| Original residue | Exemplary conservative substitution residues |
|---|---|
| Ala (A) | Val; Leu; Ile |
| Arg (R) | Lys; Gln; Asn |
| Asn (N) | Gln; Lys; Asp; Lys; Arg |
| Asp (D) | Glu; Asn |
| Cys (C) | Ser; Ala |
| Gln (Q) | Asn; Glu |
| Glu (E) | Asp; Gln |
| Gly (G) | Ala |
| His (H) | Asn; Gln; Lys; Arg |
| Ile (I) | Leu; Val; Met; Ala; Phe; Nle |
| Leu (L) | Nle; Ile; Val; Met; Ala; Phe |
| Lys (K) | Arg; Gln; Asn |
| Met (M) | Leu; Phe; Ile |
| Phe (F) | Trp; Leu; Val; Ile; Ala; Tyr |
| Pro (P) | Ala |
| Ser (S) | Thr |
| Thr (T) | Val; Ser |
| Trp (W) | Tyr; Phe |
| Tyr (Y) | Trp; Phe; Thr; Ser |
| Val (V) | Ile; Leu; Met; Phe; Ala; Nle |

In one embodiment, a modified fusion protein of the present disclosure may be as disclosed in C61 to C75 of Table 2.

### 8. Conjugate of Fusion Protein and scFv

In one embodiment of the present disclosure, a conjugate of the modified fusion protein and an scFv according to an embodiment of the present disclosure may be provided. In one embodiment, the scFv may be an scFv targeting PD-L1, and may specifically be an atezolizumab scFv or an A167 scFv binding to PD-L1 as disclosed in Table 9. In the present disclosure, a conjugate of the PD-L1-targeting scFv and the modified fusion protein according to an embodiment of the present disclosure may be referred to as PB203.

In one embodiment, a conjugate of the modified fusion protein and scFv of the present disclosure may be as disclosed in SEQ ID NOS: 1 to 32 of Table 9.

In one embodiment, a conjugate may have at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity to an amino acid sequence selected from the group consisting of the amino acid sequences of Table 9. In such variants, while including or maintaining the amino acid variations shown in the characteristics of the conjugate of the present disclosure, amino acid variations (e.g., conservative amino acid substitutions) in other amino acid sequences may be included.

In one embodiment, components of a conjugate of the present disclosure (scFv, VEGFR1 extracellular domain, linker, and multimerization domain) may have post-translational modifications including, for example, glycosylation, sialylation, acetylation, and phosphorylation.

In one embodiment, a conjugate of the present disclosure may include conservative amino acid substitutions at positions other than the amino acid variation positions described herein. Such conservative amino acid substitutions may be introduced into any of the components of the modified fusion protein of the present disclosure (e.g., scFv, extracellular domain of VEGFR1, linker, and multimerization domain).

In one embodiment, a conjugate of the present disclosure may comprise a signal peptide or signal sequence for secretion of the protein from a cell. For example, a conjugate of the present disclosure may further comprise a peptide of heterologous origin, specifically, a signal sequence or another peptide having a specific cleavage site at the N terminus of the mature fusion protein. In one embodiment, the heterologous signal sequence may be one that is recognized and processed (i.e., cleaved by a signal peptide hydrolase) by a eukaryotic host cell. Information regarding signal peptides is well known in the field and is presented, for example, in Korean Patent No. 10-2228921, but is not limited thereto. In one embodiment, the signal peptide may be a human interleukin signal sequence. In one embodiment, the signal peptide may consist of the amino acid sequence MVSYWDTGVLLCALLSCLLLTGSSSG (tPA), MEFGLSWVFLVALFRGVQC (H7), MKWVTFISLLFLFSSAYS (human serum albumin), MGWSCIILFLVATATGVHS (mouse Ig heavy chain), MDWTWRVFCLLAVAPGAHS (human Ig heavy chain), or MYRMQLLSCIALSLALVTNS (human interleukin-2), but any signal peptide that a person of ordinary skill in the art can use for the production of a fusion protein may be used without limitation.

In one embodiment, a conjugate of the present disclosure may have an IC₅₀ of less than or equal to about 1 mg/ml, 500 ng/ml, 300 ng/ml, 100 ng/ml 70 ng/ml, 50 ng/ml, 45 ng/ml, 40 ng/ml, 35 ng/ml, 30 ng/ml, 25 ng/ml, 20 ng/ml, 10 ng/ml, 5 ng/ml, 1 ng/mL, 100 pg/mL, 70 pg/mL, 50 pg/mL, 45 pg/mL, 40 pg/mL, 35 pg/mL, 30 pg/mL, 25 pg/mL, 20 pg/mL, 10 pg/mL, 5 pg/mL, or 1 pg/mL (inclusive, any value between these numbers included) for inhibition of ligand activity (e.g., inhibition of VEGF activity, PD-L1 activity, or PlGF activity).

In one embodiment, a conjugate of the present disclosure may have a Kd that is less than any one of 1.0 mM, 500 µM, 100 µM, 50 µM, 25 µM, 10 µM, 5 µM, 1 µM, 900 nM, 800 nM, 700 nM, 600 nM, 500 nM, 400 nM, 350 nM, 300 nM, 250 nM, 200 nM, 150 nM, 100 nM, 95 nM, 90 nM, 85 nM, 80 nM, 75 nM, 70 nM, 65 nM, 60 nM, 55 nM, 50 nM, 45 nM, 40 nM, 35 nM, 30 nM, 25 nM, 20 nM, 15 nM, 10 nM, 5 nM, 1 nM, 900 pM, 800 pM, 700 pM, 600 pM, 500 pM, 400 pM, 300 pM, 200 pM, 100 pM, 50 pM, 25 pM, 12.5 pM, 6.25 pM, 5 pM, 4 pM, 3 pM, 1 pM, 0.5 pM, 0.1 pM, 0.05 pM, or 0.01 pM (inclusive, including any values between these numbers) with respect to a binding partner (e.g., VEGF, PD-L1, and/or PlGF).

### III. Nucleic acids, vectors, and host cells

### Nucleic acids

In one embodiment of the present disclosure, an isolated nucleic acid encoding any component of the modified fusion protein, scFv, and conjugate of the modified fusion protein and scFv of the present disclosure, such as a anti PD-L1 scFv, VEGFR1 extracellular domain and/or a multimerization domain, can be provided. Nucleic acids encoding mammalian VEGFRs have been described for all receptor types. Exemplary nucleic acid sequences can be found in US Patent No. 7,928,072 and WO 2006/113277 but are not limited thereto. mRNAs encoding human VEGFR1 and VEGFR2 can be found under GenBank accession numbers NM_002019.4 and NM_002253.2, respectively.

Isolated nucleic acids encoding a multimerization domain (e.g., an Fc region) can be provided in the present disclosure.

An isolated nucleic acid encoding the modified fusion protein of the present disclosure may be provided.

An isolated nucleic acid sequence encoding a modified fusion protein of the present disclosure or a component of the fusion protein (e.g., an extracellular domain of VEGFR1 or a multimerization domain) may further comprise a nucleic acid sequence encoding a linker.

In one embodiment, an isolated nucleic acid may further comprise a sequence encoding a signal peptide which serves as a signal sequence for secretion of a modified fusion protein from a host cell. In one embodiment, an isolated nucleic acid may not include a sequence encoding a signal peptide.

An isolated nucleic acid molecule encoding a modified fusion protein of the present disclosure or a component of the fusion protein (e.g., an extracellular domain of VEGFR1, a linker, or a multimerization domain) may be in the form of RNA, e.g., mRNA, hnRNA, tRNA, or any other form, or in the form of DNA including, but not limited to, cDNA and genomic DNA obtained by cloning or produced synthetically, or any combination thereof. Such isolated nucleic acid molecules can be prepared by various methods known in the art (*see* Molecular Cloning: A Laboratory Manual (Sambrook et al.,4thed., Cold Spring Harbor Laboratory Press, Cold Spring Harbor, NY, 2012 ) and Current Protocols in Molecular Biology (F.M. Ausubel, et al. eds., 2003).

### Vectors

In one embodiment, the present disclosure relates to a nucleic acid delivery vehicle or use thereof for introducing one or more nucleic acid sequences encoding a conjugate or a conjugate component into a cell for expression of the protein. The nucleic acid sequence may be the sequence of the isolated nucleic acid described above.

In one embodiment, examples of nucleic acid delivery vehicles include liposomes, biocompatible polymers (including natural and synthetic polymers); lipoproteins; polypeptides; polysaccharides; lipopolysaccharide; artificial viral envelopes; metal particles; and bacteria, viruses such as baculoviruses, adenoviruses and retroviruses, bacteriophages, cosmids, plasmids, fungal vectors and other recombinant vehicles commonly used in the art which have been described for expression in various eukaryotic and prokaryotic hosts. In one embodiment, the nucleic acid delivery vehicle may be an expression vector such as a plasmid. In one embodiment, the nucleic acid sequence included in an expression vector may be operably linked to an expression control sequence.

In one embodiment, the vector may include any element to establish a conventional function of an expression vector, such as a promoter, ribosome binding element, terminator, enhancer, selection marker, and an origin of replication. The promoter can be a constitutive, inducible or repressible promoter. An exemplary promoter is provided in Korean Patent No. 10-0659477 but is not limited thereto.

A number of expression vectors capable of delivering nucleic acids to a cell (e.g., derived from bacterial cell, yeast cell, plant cell, or mammalian cell) are known in the art and may be used herein for production of a conjugate or conjugate component in the cell. For example, *E. coli* can be used to produce a conjugate if transformed with a plasmid, such as pBR322 (Mandel et al., J. Mol. Biol., 1970, 53:154), engineered to contain a nucleic acid encoding the conjugate. Expressed conjugates or conjugate components can be harvested from the cells and purified according to conventional techniques known in the art and as described in this disclosure.

In one embodiment, an expression vector comprising a nucleic acid encoding a conjugate described above and capable of being replicated in a bacterial or eukaryotic host can be used to transfect the host and thereby direct expression of the nucleic acid to produce the conjugate which may then be recovered in a biologically active form. As used herein, a biologically active form includes a form capable of binding a VEGF ligand, PD-L1 ligand, or a PlGF ligand.

In one embodiment, an expression vector containing a nucleic acid molecule encoding a conjugate or conjugate component can be identified by, without limitation, at least three general methods: (a) DNA-DNA hybridization, (b) presence or absence of "marker" gene functions, and (c) expression of the inserted sequence. Details of the above three methods are provided in Korean Patent Nos. 10-0659477 or 10-2228921, but are not limited thereto.

### Host cells

In one embodiment of the present disclosure, a host cell containing the expression vector encoding a conjugate of the present disclosure can be provided. Such host cells are suitable for expression of a conjugate of the present disclosure and may correspond to a host-vector system for producing the conjugate. In one embodiment, host cells can be used to produce viral particles (e.g., recombinant viral vectors).

As used herein, the term "host cell" may include a cell which has been or can be a recipient for a vector(s) of this disclosure and progeny thereof. The progeny may not necessarily be completely identical (in morphology or in genomic of total DNA complement) to the original parent cell due to natural, accidental, or deliberate mutation. In one embodiment, the host cell may be a bacterial cell such as *E. coli*, a yeast cell such as *Pichia pastoris*, an insect cell such as *Spodoptera frugiperda*, or a mammalian cell such as COS, HEK or CHO cell, but is not limited thereto.

In one embodiment, a conjugate of the present disclosure may be expressed in a host cell transiently, constitutively or permanently.

In one embodiment of the present disclosure, a method of producing a conjugate of the present disclosure can be provided which comprises growing host cells or cells of a host-vector system described above under conditions permitting production of the conjugate and then recovering the conjugate so produced. A conjugate useful for practicing the present disclosure can be produced by expression in a prokaryotic or eukaryotic expression system. Methods for culturing host cells or producing a conjugate from host cells are well known in the field and are disclosed, for example, in Korean Patent No. 10-2228921, but are not limited thereto.

In one embodiment, a conjugate of the present disclosure produced from the host cells described above can be purified and identified by a variety of methods. Methods for purifying and identifying a conjugate produced from host cells are well known in the art and are provided, for example, in Korean Patent No. 10-2228921, but are not limited thereto.. For further purification of the conjugate, any number of purification methods may be used, including but not limited to conventional ion exchange chromatography, affinity chromatography, different sugar chromatography, hydrophobic interaction chromatography, reverse phase chromatography or gel filtration.

### IV. Viral particles and methods for producing viral particles

In one embodiment of the present disclosure, viral particles (or virions) comprising a nucleic acid encoding a conjugate of the present disclosure can be provided.

Viral vectors can be used to deliver a nucleic acid encoding a conjugate or conjugate component for expression of the protein in target cells within a particular target tissue (e.g., a diseased tissue). Many species of virus are known, and many have been studied for purposes of delivering nucleic acids to target cells. An exogenous nucleic acid can be inserted into a vector, such as adenovirus, partially deleted adenovirus, fully deleted adenovirus, adeno-associated virus (AAV), retrovirus, lentivirus, etc., for delivery to a cell.

In one embodiment, the cell is within an individual, and the virus can be delivered via intravenous, intramuscular, intraportal or other route of administration. In one embodiment, viral vectors may include those derived from adenoviruses, adeno-associated viruses (AAV), and retroviruses (including lentiviruses, such as human immunodeficiency virus (HIV)). For exemplary viral vectors, see, without limitation, US Patent No. 7,928,072 and WO 2006/113277, which are incorporated herein by reference in their entirety. Viral particles containing a nucleic acid encoding a conjugate and methods for producing them are well known in the art and are disclosed, for example, in Korean Patent No. 10-2228921, but are not limited thereto.

### V. Methods of treatment using conjugates and viral particles

In one embodiment, the present disclosure relates to a pharmaceutical composition for use in the prevention, amelioration, or treatment of a VEGF ligand-, VEGF receptor-, or PD-L1-related disease or at least one of the symptoms of the disease, comprising conjugate of the present disclosure as an active ingredient.

In one embodiment, the present disclosure relates to a method for preventing, ameliorating or treating a VEGF ligand-, VEGF receptor-, or PD-L1-related disease or at least one of the symptoms of the disease, the method comprising administering a conjugate of the present disclosure.

In one embodiment, the present disclosure relates to the use of a conjugate of the present disclosure for preventing, ameliorating or treating a VEGF ligand-, VEGF receptor-, or PD-L1-related disease or at least one of the symptoms of the disease.

In one embodiment, the present disclosure relates to a conjugate of the present disclosure or a pharmaceutical composition comprising the same for use in a method for preventing, ameliorating or treating a VEGF ligand-, VEGF receptor-, or PD-L1-related disease or at least one of the symptoms of the disease.

In one embodiment, a conjugate of the present disclosure, in the form of a nucleic acid encoding the same, can be used in a nucleic acid delivery vehicle or viral particles (e.g., a recombinant viral vector) for the prevention, amelioration or treatment of a VEGF ligand-, VEGF receptor-, or PD-L1-related disease or at least one of the symptoms of the disease. In one embodiment, the nucleic acid delivery vehicle or viral particles may be applied in gene therapy for use in the prevention, amelioration or treatment of a VEGF ligand-, VEGF receptor-, or PD-L1-related disease or at least one of the symptoms of the disease. In one embodiment, . any gene therapy method available in the art can be used according to the present disclosure. In one embodiment, the nucleic acid is introduced into a cell prior to administration *in vivo* of the resulting recombinant cell. Cells into which a nucleic acid is introduced for gene therapy purposes encompass all available cell types and include, for example, epithelial cells, endothelial cells, keratinocytes, fibroblasts, muscle cells, hepatocytes, blood cells (e.g., T-lymphocytes, B-lymphocytes, monocytes, macrophages, neutrophils, eosinophils, megakaryocytes, or granulocytes), various stem cells or progenitor cells, such as hematopoietic stem or progenitor cells obtained from bone marrow, umbilical cord blood, peripheral blood, or fetal liver. etc., but are not limited thereto. In one embodiment, the cells used for gene therapy may be autologous cells of the patient.

In one embodiment, a conjugate can bind a VEGF protein, PlGF and/or a PD-L1 protein. In one embodiment, a conjugate may have one or more of the following characteristics: (a) bind one or more proteins of the VEGF family, e.g., VEGF-A, VEGF-B, VEGF-C, and VEGF-D, PlGF, and PD-L1; (b) block the binding of a VEGF family protein to a VEGF receptor and/or the binding of a PD-L1 protein to a PD-1 receptor; (c) inhibit activation of the VEGF signaling pathway and/or PD-L1 signaling pathway; (d) prevent, ameliorate and/or treat a disease such as an chronic infection, tissue allograft, ocular disease, autoimmune disease, inflammatory disease, neoplastic disease, or cancer; (e) attenuate, contain or prevent growth of neoplasm or cancer; (f) inhibit metastasis of neoplasm or cancer. The activity of a conjugate can be measured *in vivo* and/or *in vitro.*

In one embodiment, a pharmaceutical composition may include a pharmaceutically acceptable carrier and/or diluent. In one embodiment, pharmaceutically acceptable carriers can be sterile liquids, such as water and oils, including those of petroleum, animal, plant, or synthetic origin, such as peanut oil, soybean oil, mineral oil, etc. Saline and aqueous dextrose, polyethylene glycol (PEG) and glycerol solutions can also be used as liquid carriers, particularly for injectable solutions. Such pharmaceutical compositions may further include additional ingredients, for example preservatives, buffers, isotonic agents, antioxidants and stabilizers, nonionic wetting or clarifying agents, thickening agents, etc. A pharmaceutical composition of the present disclosure may be packaged in single unit dosages or in multidosage forms. These compositions are generally formulated as sterile and substantially isotonic solutions. Additionally, compositions may be formulated to have osmotic values compatible with the aqueous humor of the eye and ophthalmic tissues. Such osmotic values can generally be in the range of from about 200 to 400 milliomoles per kilogram of water ("mOsm/kg") or about 300 mOsm/kg. The retina is considered to have an osmotic value of approximately 283 mOsm/kg.

In one embodiment, a pharmaceutical composition may be a parenteral composition. It may be advantageous to formulate parenteral compositions in a dosage unit form for uniformity of dosage and ease of administration.

### Diseases or disorders

In one embodiment, a VEGF ligand-,VEGF receptor-, or PD-L1-related disease may include a chronic infection, a tissue allograft, an autoimmune disease (e.g., rheumatoid arthritis, multiple sclerosis, habitual abortion due to immune response, or systemic lupus erythematosus), an inflammatory disease (inflammatory arthritis, osteoarthritis, or psoriasis), a neoplastic disease, cancer (e.g., breast cancer, lung cancer, stomach cancer, pancreatic cancer, or leukemia), an angiogenesis-related disease (e.g., atherosclerosis), or an ocular disease (e.g., age-related macular degeneration, choroidal neovascularization, or uveitis), but is not limited thereto.

In one embodiment, cancer may include prostate cancer, urethral cancer, penile cancer, breast cancer, lung cancer, esophageal cancer, small intestine cancer, colorectal cancer, rectal cancer, colon cancer, liver cancer, urinary tract cancer (e.g. bladder cancer), kidney cancer, lung cancer (e.g. non-small cell lung cancer), ovarian cancer, cervical cancer, endometrial cancer, vaginal carcinoma, vulvar carcinoma, pancreatic cancer, stomach cancer, endocrine cancer, head and neck cancer, thyroid cancer, parathyroid cancer, thyroid gland cancer, adrenal cancer, skin cancer (e.g. melanoma, cutaneous squamous cell carcinoma (CSCC), head and neck squamous cell carcinoma (HNSCC)), Hodgkin's disease, bone cancer, hematopoietic cancers of lymphoid or myeloid lineage, chronic or acute leukemia, head and neck cancer, nasopharyngeal carcinoma (NPC), glioblastoma, teratocarcinoma, neuroblastoma, adenocarcinoma, cancer of mesenchymal origin (e.g. fibrosarcoma or rhabdomyosarcoma), soft tissue sarcomas and carcinomas, choriocarcinoma, hepatoblastoma, central nervous system (CNS) tumor, primary central nervous system lymphoma, spinal cord tumor, brainstem glioma, pituitary adenoma, Kaposi's sarcoma, or Wilm's tumor, but is not limited thereto. A conjugate of the present disclosure can act as anti-PD-L1 and anti-angiogenic agent or anti-VEGF and anti-PD-L1 agent to ameliorate, prevent and/or treat cancer or symptoms of cancer.

### Methods of administration and dosages

In one embodiment of the present disclosure, a method of delivering an effective amount of a conjugate to a subject can be provided. The conjugate can be delivered to a subject in a composition. The conjugate can also be delivered to a subject by a nucleic acid delivery vehicle or viral particle (e.g., a recombinant viral vector) containing a nucleic acid encoding the conjugate. In one embodiment of the present disclosure, a composition comprising a conjugate or a nucleic acid delivery vehicle or viral particles (e.g., a recombinant viral vector) comprising a nucleic acid encoding a conjugate can be provided.

In one embodiment, a composition of the present disclosure can be administered to an individual via any route including, but not limited to: intravenous (e.g., by infusion pumps), intraperitoneal, intraocular, intraarterial, intrapulmonary, oral, inhalational, intravesicular, intramuscular, intratracheal, subcutaneous, intraocular, intrathecal, transdermal, transpleural, intraarterial, topical, inhalational (e.g., as mists of sprays), mucosal (e.g., via nasal mucosa), subcutaneous, transdermal, gastrointestinal, intra-articular, intracisternal, intraventricular, intracranial, intraurethral, intrahepatic and intratumoral. In one embodiment, a composition of the present disclosure can be administered by any conventional route, for example, by infusion or bolus injection, by absorption through the epithelial or mucocutaneous linings (e.g., oral mucosa, rectal mucosa, intestinal mucosa, etc.) and can also be administered together with other biologically active ingredients. Administration can be systemic or local. In one embodiment, a composition of the present disclosure may be introduced into the central nervous system by any suitable route, including intraventricular injection and intrathecal injection, wherein intraventricular injection may be administered via an intraventricular catheter, for example, attached to a reservoir, such as an Ommaya reservoir.

In one embodiment, the optimal effective amount of a conjugate, a nucleic acid delivery vehicle encoding the same, or a composition comprising them can be determined empirically and may depend on the type and severity of the disease, the route of administration, disease progression and health, the weight and body area of the individual. Such determinations are within the skill of one of ordinary skill in the art.

In one embodiment, a conjugate may be administered, for example, at about 0.05 ng to about 20 mg per kg of body weight per day. In one embodiment, the dosage of the conjugate may be at least about 0.1 mg/kg, at least about 0.5 mg/kg, at least about 1.0 mg/kg, at least about 1.5 mg/kg, at least about 2.0 mg/kg, at least about 2.5 mg/kg, at least about 3.0 mg/kg, at least about 3.5 mg/kg, at least about 4.0 mg/kg, at least about 4.5 mg/kg, at least about 5.0 mg/kg, at least about 6.0 mg/kg, at least about 8.0 mg /kg, at least about 10.0 mg/kg, at least about 15.0 mg/kg, or about 20.0 mg/kg or less, about 17.0 mg/kg or less, about 14.0 mg/kg or less, about 11.0 mg/kg or less, about 9.0 mg /kg or less, about 7.0 mg/kg or less, about 5.5 mg/kg or less, about 5.0 mg/kg or less, about 4.5 mg/kg or less, about 4.0 mg/kg or less, about 3.5 mg/kg or less, about 3.0 mg/ kg or less, about 2.5 mg/kg or less, about 2.0 mg/kg or less, about 1.5 mg/kg or less, or about 1.0 mg/kg or less. In one embodiment, the dosage of the conjugate may be about 1.0 mg/kg to about 10.0 mg/kg or about 1.0 mg/kg to about 5.0 mg/kg.

In one embodiment, the amount of a nucleic acid delivery vehicle (e.g., a recombinant viral vector) comprising a nucleic acid encoding a conjugate of the present disclosure can be administered to an individual at a DNAse particle resistance (drp) titer of about 10⁴ to about 10¹⁴ drps per dose. In one embodiment, the amount of a nucleic acid delivery vehicle comprising a nucleic acid encoding the conjugate can be administered to an individual at about 10⁵ to about 10¹³, about 10⁶ to about 10¹², about 10⁷ to about 10¹¹, about 10⁸ to about 10¹⁰, about 10⁹ to about 10¹⁰, about 10¹⁰ to about 10¹¹, or about 10¹¹ to about 10¹² drp per dose.

In one embodiment, a composition comprising a conjugate of the present disclosure or a nucleic acid delivery vehicle encoding the same may be administered in a single daily dose, or the total daily dose may be administered in divided dosages of 2, 3, or 4 times daily. In one embodiment, a composition comprising a conjugate of the present disclosure can be administered 6 times a week, 5 times a week, 4 times a week, 3 times a week, 2 times a week, once a week, once every 2 weeks, once every 3 weeks, once a month, once every 2 months, once every 3 months, once every 6 months, once every 9 months, or once every year. In one embodiment, a composition comprising a nucleic acid delivery vehicle comprising a nucleic acid encoding a conjugate of the present disclosure can be administered less frequently, for example, once every 3 months, once every 4 months, once every 5 months, once every 6 months, once every 7 months, once every 8 months, once every 9 months, once every 10 months, once every 11 months, or once every year.

### Diagnosis or detection

A conjugate of the present disclosure may be labeled with a detectable label such as a radioisotope, a fluorescent label, a toxin label, an enzyme label, a chemiluminescent label, or a nuclear magnetic resonance contrast agent to confirm the ligand-receptor binding interaction. Assay systems applied to chimeric molecules may also be considered. Such detectable labels are well known in the art.

### VI. Articles of manufacture and kits

In one embodiment, the present disclosure relates to an article of manufacture or kit containing a conjugate of the present disclosure, a recombinant viral vector expressing the same, or a composition containing the same in suitable packaging. In one embodiment, the suitable packaging is well known in the art and includes, for example, vials (e.g., sealed vials), vessels, ampoules, bottles, jars, flexible packaging (e.g., sealed Mylar or plastic bags), etc., but is not limited thereto. These articles of manufacture may further be sterilized and/or sealed.

In one embodiment, a kit may further include instructions on how to use the composition for the uses described herein. In one embodiment, the kit may further include other components desirable from a commercial and user standpoint, such as other buffers, diluents, filters, needles, syringes, and package inserts (PIs) with instructions for performing any methods described herein. For example, in one embodiment, a kit may comprise (i) a conjugate protein described herein and/or a recombinant viral vector encoding a conjugate protein described herein, (ii) a pharmaceutically acceptable carrier, and (iii) any one or more of: a buffer, a diluent, a filter, a needle, a syringe, and a package insert with instructions for performing administration.

Hereinafter, technical features and effects of the present disclosure will be described in more detail referring to working examples. However, these examples are provided only for illustrative purposes to aid understanding of the present disclosure, and the scope of the present disclosure is not limited by the following examples.

### EXAMPLE 1 - Construction of Expression Vectors

The coding sequence (CDS) region of a conjugate designed in the examples below was subjected to codon optimization to be in a form suitable for CHO production cells, and the conjugate was prepared in a final pcDNA3.1(+) vector (Invitrogen) via gene synthesis (Geneuniversal).

### EXAMPLE 2 - Design of Modified Fusion Proteins

### Example 2.1 - Preparation and Characterization of Surface Charge Transition Variants

In order to secure a fusion protein including a VEGFR receptor component with enhanced physicochemical properties, the inventors of the present disclosure used a structure containing the entire extracellular domain of VEGFR1 (PDB entry. 5t89) as a template to analyz the surface charge distribution of the pretein and deduced areas with dense positive charges and positions with hydrophobic surface charges.

Through this analysis, it was demonstrated that in VEGFR1 domain D3, side chains of amino acid residues present in the β1-β2 loop corresponding to amino acid residues T236 to T247 (named Site 1), the β2-β3 loop corresponding to amino acid residues T256 to V262 (named Site 2), and the β5-β6 loop corresponding to amino acid residues D299 to L308 (named Site 3) are exposed on the surface of the fusion protein. An attempt was made to stabilize the surface charge and structure of fusion proteins through variations in amino acid residues located in the flexible loops exposed on the surface. For example, an attempt was made to substitute amino acid residues exposed on the surface with amino acid residues present at the homologous position of VEGFR2. In addition, amino acid residues of Site 2 are exposed on the protein surface on the side that binds to ligands (VEGF or PlGF), and variations at these positions affect binding affinity to the ligands or solubility due to surface residue exposure.

### Example 2.2 - Preparation and Characterization of Linker Variants

A human VEGF receptor binds to the ligand VEGF or PlGF through interaction with the immunoglobulin-like domains D2 and D3. In the case of a fusion proteins using these domains D2 and D3, the domains D2 and D3 have an open structure before binding to a ligand and then form a closed structure upon binding to the ligand. If the structures of domains D2 and D3 cannot secure enough space to bind to a ligand depending on the binding length of domain D3 and the multimerization domain or interference occurs between the constructs, binding affinity of the fusion protein to a ligand may decrease. Therefore, the binding length and binding mode of domain D3 and the multimerization domain in the fusion protein can affect the structure of the fusion protein that binds to a ligand and its binding affinity to the ligand.

Therefore, the present inventors modified the length and type of the linker in the modified fusion proteins of the present disclosure in order to emulate and secure the structural characteristics of a native human VEGF receptor. Specifically, linkers comprising polypeptides of Gly-Ser repeat sequences, amino acid sequences of a hinge region derived from IgG1, IgG4, or IgD, and a variant sequences of such hinges were introduced. Such linkers were intended to increase physical stability by reducing the tension generated in the loop (linker) formed between domain D3 and the multimerization domain (Fc region) upon ligand binding.

In addition, amino acid sequences derived from immunoglobulin hinge regions inherently have cleavage sites for various proteases due to its own characteristics (Vlasak and Ionescu, 2011, MAbs 3: pp. 253-263), and upon introduction into fusion proteins, glycosylation (e.g., O-glycosylation) may occur, resulting in oligomerization (Song et al., 2020, Computational and Structural Biotechnology Journal 18: pp. 3925-3935). To improve in vivo stability of the modified fusion protein of the present disclosure, the present inventors further modified amino acid sequences at protease cleavage sites, for example, cleavage sites for papain, and attempted to modify amino acid residues Ser or Thr where glycosylation occurs. In addition, to increase the structural flexibility of the linker, attempts were made to substitute cysteines present in sequences derived from the core hinge of the Fc hinge region (e.g., CPPCP). The modified fusion protein according to an embodiment of the present disclosure exhibits excellent productivity and excellent physicochemical properties, as well as superior binding affinity to ligands.

### Example 2.3 - Preparation and Characterization of Disulfide Bond Variants

Since a fusion protein is a conjugate of heterologous proteins, stability is expected to be decreased due to non-specific cleavage at the linking site. The terminal region of domain D3 forms a loop and may lack secondary structure, and is expected to have weak stability because it is the site where connection to the multimerization domain, a heterologous protein, begins. By introducing a covalent disulfide bond, the inventors aimed to induce inter-domain linkage near the site where the heterologous proteins are connected, thereby preventing physical cleavage of the protein and improve stability.

Positions where a disulfide bond can be introduced in the fusion protein were identified through three-dimensional structure-based analysis. Among residues located in variable loops adjacent to the terminal region of domain D3, residues whose side-chain orientation was directed toward the linker and whose distance between the terminal atom of the side chain and the Y329 Cα atom was within 6 Å were selected as variation residues for disulfide bonding. After securing the three-dimensional structure of the fusion protein through a structural modeling program (Phyre2, Swiss-Model), a structural model with an introduced disulfide bond was obtained through in silico variation at the introduction sites. Thereafter, changes in the energy level of the fusion protein due to introduction of the disulfide bond were predicted using a three-dimensional structure energy prediction program (foldX in YASARA; Schymkowitz et al., 2005, Nucleic Acids Research 33: W382-388).

As positions that are physically proximal to the terminal region of domain D3 and capable of forming a disulfide bond, amino acid residues on the β1-β2 loop or the β5-β6 loop of domain D3 were selected, and for the terminal region of domain D3, an amino acid residue at the +2 position relative to Y329 was selected.

### Example 2.4 - Preparation and Characterization of Combination Variants

Combination variants of fusion proteins having combined characteristics of the variants exhibiting superior properties in Examples 2.1 to 2.3 were prepared as shown in Table 2, and the characteristics of each variant are shown in Table 1.

**[TABLE 1]**

| Protein code | Surface charge variant position | Linker sequence | Origin of Fc hinge for linker sequence | Amino acid variation for disulfide bond |
|---|---|---|---|---|
| C08 (control: VEGF-Grab) | - | DKTHTCPPCP | IgG1 | - |
| C61 | K241E/R244V/H246E/L25 8A/K300G/Q302T/K304S | CSSGDATPTSPPSP | IgG1 | L243C/331C |
| C62 | K241E/R244V/H246E/L25 8A/K300G/Q302T/K304S | CSKVDKKVEPKSSD TPPTCPPCP | IgG1 | L243C/331C |
| C63 | K241E/R244V/H246E/L25 8A/K300G/Q302T/K304S | CSGGGGSAEPKAG DATPPTCPPCP | IgG1 | L243C/331C |
| C64 | K241E/R244V/H246E/L25 8A/K300G/Q302T/K304S | CSGGGGSGGGGSG GGGSAESKYGPPCP PCP | IgG4 | L243C/331C |
| C65 | K241E/R244V/H246E/L25 8A/K300G/Q302T/K304S | CSNTGSGGEEKKKE KEKEEQEERSSDTP PTCPPCP | IgD/G1 | L243C/331C |
| C66 | K241E/R244V/H246E/L25 8A/K300G/Q302T/K304S | CGSSGGSSGEPKSD ATPTCPPCP | IgG1 | L243C/331C |
| C67 | K241E/R244V/H246E/L25 8A/K300G/Q302T/K304S | CSKVDKKVEPKSSD KTYTCPPCP | IgG1 | L243C/331C |
| C68 | K241E/R244V/H246E/L25 8S/K300G/Q302T/K304S | CSKVDKKVEPKSSD TPPTCPPCP | IgG1 | L243C/331C |
| C69 | K241E/R244V/H246E/L25 8D/K300G/Q302T/K304S | CSKVDKKVEPKSSD TPPTCPPCP | IgG1 | L243C/331C |
| C70 | K241E/R244V/H246E/L25 8S/K300G/Q302T/K304S | CSGGGGSGGGGSG GGGSAESKYGPPCP PCP | IgG4 | L243C/331C |
| C71 | K241E/R244V/H246E/L25 8D/K300G/Q302T/K304S | CSGGGGSGGGGSG GGGSAESKYGPPCP PCP | IgG4 | L243C/331C |
| C72 | K241E/R244V/H246E/L25 8S/K300G/Q302T/K304S | CGSSGGSSGEPKSD ATPTCPPCP | IgG1 | L243C/331C |
| C73 | K241E/R244V/H246E/L25 8D/K300G/Q302T/K304S | CGSSGGSSGEPKSD ATPTCPPCP | IgG1 | L243C/331C |
| C74 | K241E/R244V/H246E/L25 8D/K300G/Q302T/K304S | CSSGDATPTSPPSP | IgG1 | L243C/331C |
| C75 | K241E/R244V/H246E/L25 | CSNTGSGGEEKKKE | IgD/G1 | L243C/331C |
| | 8D/K300G/Q302T/K304S | KEKEEQEERSSDTP PTCPPCP | | |
| C94 | L258A | CGSSGGSSGEPKSD ATPTCPPCP | IgG1 | - |
| C95 | K300G/Q302T/K304S/K30 6Q | CGSSGGSSGEPKSD ATPTCPPCP | | - |
| C96 | L243S/R244V/L258A | CSNTGSGGEEKKKE KEKEEQEERSCDTP PTCPPCP | IgG1 | - |
| C97 | L258A/K300G/Q302T/K304S | CSGGGGSGGGGSG GGGSAESKYGPPCP PCP | IgG1 | - |
| C99 | K241E/L243S/R244V/H24 SE/K300G/Q302T/K304S/ K306Q | CGSSGGSSGEPKSD ATPTCPPCP | IgG1 | - |

Given in Table 1 are identification codes, amino acid sequences, and SEQ ID NOS of the proteins described in the present disclosure.

**[TABLE 2]**

| Protein code | Amino acid sequence |
|---|---|
| VEGFR1(FLT1) | DLAARNILLSENNVVKICDFGLARDIYKNPDYVRKGDTRLPLK WMAPESIFDKIYSTKSDVWSYGVLLWEIFSLGGSPYPGVQMDE DFCSRLREGMRMRAPEYSTPEIYQIMLDCWHRDPKERPRFAELV EKLGDLLQANVQQDGKDYIPINAILTGNSGFTYSTPAFSEDFFKE SISAPKFNSGSSDDVRYVNAFKFMSLERIKTFEELLPNATSMFDD YQGDSSTLLASPMLKRFTWTDSKPKASLKIDLRVTSKSKESGLS DVSRPSFCHSSCGHVSEGKRRFTYDHAELERKIACCSPPPDYNS VVLYSTPPI |
| SEQ ID NO: 41 | |
| VEGF-Grab1(fusion protein including D2 of VEGFR1 and D3 glycosylated with 1 to 3 amino acids, based on VEGFR1 wild-type protein) SEQ ID NO: 42 | EFGRPFVEMYSEIPEIIHMTEGRELVIPCRVTSPNITVTLKKFPLDT LIPDGKRIIWDSRKGFIISNATYKEIGLLTCEATVNGHLYKTNYL THRQTNTIIDVQISTPSPVTLLRGHTLVLNCTATTPLNTRVQMTW SYPDEKNKRASVRRRIDQSNSHANIFYSVLTIDKMQNKDKGLYT CRVRSGPSFKSVNTSVHIYDKALEDKTHTCPPCPAPELLGGPSVF LFPPKPKDTLMISRTPEVTCVVVDVSHEDPEVKFNWYVDGVEV HNAKTKPREEQYNSTYRVVSVLTVLHQDWLNGKEYKCKVSNK ALPAPIEKTISKAKGQPREPQVYTLPPSREEMTKNQVSLTCLVKG FYPSDIAVEWESNGQPENNYKTTPPVLDSDGSFFLYSKLTVDKS RWQQGNVFSCSVMHEALHNHYTQKSLSLSPGK |
| VEGF-Grab3 (fusion protein including D2 of VEGFR1 and D3 glycosylated with 3 amino acids, based on a VEGFR1) SEQ ID NO: 43 | EFGRPFVEMYSEIPEIIHMTEGRELVIPCRVTSPNITVTLKKFPLDT LIPDGKRIIWDSRKGFIISNATYKEIGLLTCEATVNGHLYKTNYL THRQTNTIIDVQISTPSPVTLLRGHTLVLNCTATTPLNTRVQMTW SYPDEKNKNASVRRRIDQSNSHANIFYSVLTIDKMQNKDKGLYT CRVRSGPSFKSVNTSVHIYDKALEDKTHTCPPCPAPELLGGPSVF LFPPKPKDTLMISRTPEVTCVVVDVSHEDPEVKFNWYVDGVEV HNAKTKPREEQYNSTYRVVSVLTVLHQDWLNGKEYKCKVSNK ALPAPIEKTISKAKGQPREPQVYTLPPSREEMTKNQVSLTCLVKG FYPSDIAVEWESNGQPENNYKTTPPVLDSDGSFFLYSKLTVDKS RWQQGNVFSCSVMHEALHNHYTQKSLSLSPGK |
| C61 | EFGRPFVEMYSEIPEIIHMTEGRELVIPCRVTSPNITVTLKKFPLDT LIPDGKRIIWDSRKGFIISNATYKEIGLLTCEATVNGHLYKTNYL THRQTNTIIDVQISTPSPVELCVGETLVLNCTATTPANTRVQMT WSYPDEKNKNASVRRRIDQSNSHANIFYSVLTIDGMTNSDKGL YTCRVRSGPSFKSVNTSVHIYDCSSGDATPTSPPSPAPELLGGPS VFLFPPKPKDTLMISRTPEVTCVVVDVSHEDPEVKFNWYVDGV EVHNAKTKPREEQYNSTYRVVSVLTVLHQDWLNGKEYKCKVS NKALPAPIEKTISKAKGQPREPQVYTLPPSRDELTKNQVSLTCLV KGFYPSDIAVEWESNGQPENNYKTTPPVLDSDGSFFLYSKLTVD KSRWQQGNVFSCSVMHEALHNHYTQKSLSLSPG |
| SEQ ID NO: 44 | |
| C62 | EFGRPFVEMYSEIPEIIHMTEGRELVIPCRVTSPNITVTLKKFPLDT LIPDGKRIIWDSRKGFIISNATYKEIGLLTCEATVNGHLYKTNYL THRQTNTIIDVQISTPSPVELCVGETLVLNCTATTPANTRVQMT WSYPDEKNKNASVRRRIDQSNSHANIFYSVLTIDGMTNSDKGL YTCRVRSGPSFKSVNTSVHIYDCSKVDKKVEPKSSDTPPTCPPCP APELLGGPSVFLFPPKPKDTLMISRTPEVTCVVVDVSHEDPEVKF NWYVDGVEVHNAKTKPREEQYNSTYRVVSVLTVLHQDWLNG KEYKCKVSNKALPAPIEKTISKAKGQPREPQVYTLPPSRDELTK NQVSLTCLVKGFYPSDIAVEWESNGQPENNYKTTPPVLDSDGSF FLYSKLTVDKSRWQQGNVFSCSVMHEALHNHYTQKSLSLSPG |
| SEQ ID NO: 45 | |
| C63 | EFGRPFVEMYSEIPEIIHMTEGRELVIPCRVTSPNITVTLKKFPLDT LIPDGKRIIWDSRKGFIISNATYKEIGLLTCEATVNGHLYKTNYL THRQTNTIIDVQISTPSPVELCVGETLVLNCTATTPANTRVQMT WSYPDEKNKNASVRRRIDQSNSHANIFYSVLTIDGMTNSDKGL YTCRVRSGPSFKSVNTSVHIYDCSGGGGSAEPKAGDATPPTCPP CPPCPAPELLGGPSVFLFPPKPKDTLMISRTPEVTCVVVDVSHED PEVKFNWYVDGVEVHNAKTKPREEQYNSTYRVVSVLTVLHQD WLNGKEYKCKVSNKALPAPIEKTISKAKGQPREPQVYTLPPSRD ELTKNQVSLTCLVKGFYPSDIAVEWESNGQPENNYKTTPPVLDS DGSFFLYSKLTVDKSRWQQGNVFSCSVMHEALHNHYTQKSLSL SPG |
| SEQ ID NO: 46 | |
| C64 | EFGRPFVEMYSEIPEIIHMTEGRELVIPCRVTSPNITVTLKKFPLDT LIPDGKRIIWDSRKGFIISNATYKEIGLLTCEATVNGHLYKTNYL THRQTNTIIDVQISTPSPVELCVGETLVLNCTATTPANTRVQMT WSYPDEKNKNASVRRRIDQSNSHANIFYSVLTIDGMTNSDKGL YTCRVRSGPSFKSVNTSVHIYDCSGGGGSGGGGSGGGGSAESK YGPPCPPCPAPELLGGPSVFLFPPKPKDTLMISRTPEVTCVVVDV SHEDPEVKFNWYVDGVEVHNAKTKPREEQYNSTYRVVSVLTV LHQDWLNGKEYKCKVSNKALPAPIEKTISKAKGQPREPQVYTL PPSRDELTKNQVSLTCLVKGFYPSDIAVEWESNGQPENNYKTTP PVLDSDGSFFLYSKLTVDKSRWQQGNVFSCSVMHEALHNHYTQ KSLSLSPG |
| SEQ ID NO: 47 | |
| C65 | EFGRPFVEMYSEIPEIIHMTEGRELVIPCRVTSPNITVTLKKFPLDT LIPDGKRIIWDSRKGFIISNATYKEIGLLTCEATVNGHLYKTNYL THRQTNTIIDVQISTPSPVELCVGETLVLNCTATTPANTRVQMT WSYPDEKNKNASVRRRIDQSNSHANIFYSVLTIDGMTNSDKGL YTCRVRSGPSFKSVNTSVHIYDCSNTGSGGEEKKKEKEKEEQEE RSSDTPPTCPPCPAPELLGGPSVFLFPPKPKDTLMISRTPEVTCVV VDVSHEDPEVKFNWYVDGVEVHNAKTKPREEQYNSTYRVVSV LTVLHQDWLNGKEYKCKVSNKALPAPIEKTISKAKGQPREPQV YTLPPSRDELTKNQVSLTCLVKGFYPSDIAVEWESNGQPENNYK TTPPVLDSDGSFFLYSKLTVDKSRWQQGNVFSCSVMHEALHNH YTQKSLSLSPG |
| SEQ ID NO: 48 | |
| C66 | EFGRPFVEMYSEIPEIIHMTEGRELVIPCRVTSPNITVTLKKFPLDT LIPDGKRIIWDSRKGFIISNATYKEIGLLTCEATVNGHLYKTNYL THRQTNTIIDVQISTPSPVELCVGETLVLNCTATTPANTRVQMT WSYPDEKNKNASVRRRIDQSNSHANIFYSVLTIDGMTNSDKGL YTCRVRSGPSFKSVNTSVHIYDCGSSGGSSGEPKSDATPTCPPCP APELLGGPSVFLFPPKPKDTLMISRTPEVTCVVVDVSHEDPEVKF NWYVDGVEVHNAKTKPREEQYNSTYRVVSVLTVLHQDWLNG KEYKCKVSNKALPAPIEKTISKAKGQPREPQVYTLPPSRDELTK NQVSLTCLVKGFYPSDIAVEWESNGQPENNYKTTPPVLDSDGSF FLYSKLTVDKSRWQQGNVFSCSVMHEALHNHYTQKSLSLSPG |
| SEQ ID NO: 49 | |
| C67 | EFGRPFVEMYSEIPEIIHMTEGRELVIPCRVTSPNITVTLKKFPLDT LIPDGKRIIWDSRKGFIISNATYKEIGLLTCEATVNGHLYKTNYL THRQTNTIIDVQISTPSPVELCVGETLVLNCTATTPANTRVQMT WSYPDEKNKNASVRRRIDQSNSHANIFYSVLTIDGMTNSDKGL YTCRVRSGPSFKSVNTSVHIYDCSKVDKKVEPKSSDKTYTCPPC PAPELLGGPSVFLFPPKPKDTLMISRTPEVTCVVVDVSHEDPEVK FNWYVDGVEVHNAKTKPREEQYNSTYRVVSVLTVLHQDWLN GKEYKCKVSNKALPAPIEKTISKAKGQPREPQVYTLPPSRDELT KNQVSLTCLVKGFYPSDIAVEWESNGQPENNYKTTPPVLDSDGS FFLYSKLTVDKSRWQQGNVFSCSVMHEALHNHYTQKSLSLSPG |
| SEQ ID NO: 50 | |
| C68 | EFGRPFVEMYSEIPEIIHMTEGRELVIPCRVTSPNITVTLKKFPLDT LIPDGKRIIWDSRKGFIISNATYKEIGLLTCEATVNGHLYKTNYL THRQTNTIIDVQISTPSPVELCVGETLVLNCTATTPSNTRVQMTW SYPDEKNKNASVRRRIDQSNSHANIFYSVLTIDGMTNSDKGLYT CRVRSGPSFKSVNTSVHIYDCSKVDKKVEPKSSDTPPTCPPCPAP ELLGGPSVFLFPPKPKDTLMISRTPEVTCVVVDVSHEDPEVKFN WYVDGVEVHNAKTKPREEQYNSTYRVVSVLTVLHQDWLNGK EYKCKVSNKALPAPIEKTISKAKGQPREPQVYTLPPSRDELTKN QVSLTCLVKGFYPSDIAVEWESNGQPENNYKTTPPVLDSDGSFF LYSKLTVDKSRWQQGNVFSCSVMHEALHNHYTQKSLSLSPG |
| SEQ ID NO: 51 | |
| C69 | EFGRPFVEMYSEIPEIIHMTEGRELVIPCRVTSPNITVTLKKFPLDT LIPDGKRIIWDSRKGFIISNATYKEIGLLTCEATVNGHLYKTNYL THRQTNTIIDVQISTPSPVELCVGETLVLNCTATTPDNTRVQMT WSYPDEKNKNASVRRRIDQSNSHANIFYSVLTIDGMTNSDKGL YTCRVRSGPSFKSVNTSVHIYDCSKVDKKVEPKSSDTPPTCPPCP APELLGGPSVFLFPPKPKDTLMISRTPEVTCVVVDVSHEDPEVKF NWYVDGVEVHNAKTKPREEQYNSTYRVVSVLTVLHQDWLNG KEYKCKVSNKALPAPIEKTISKAKGQPREPQVYTLPPSRDELTK NQVSLTCLVKGFYPSDIAVEWESNGQPENNYKTTPPVLDSDGSF FLYSKLTVDKSRWQQGNVFSCSVMHEALHNHYTQKSLSLSPG |
| SEQ ID NO: 52 | |
| C70 | EFGRPFVEMYSEIPEIIHMTEGRELVIPCRVTSPNITVTLKKFPLDT LIPDGKRIIWDSRKGFIISNATYKEIGLLTCEATVNGHLYKTNYL THRQTNTIIDVQISTPSPVELCVGETLVLNCTATTPSNTRVQMTW SYPDEKNKNASVRRRIDQSNSHANIFYSVLTIDGMTNSDKGLYT CRVRSGPSFKSVNTSVHIYDCSGGGGSGGGGSGGGGSAESKYG PPCPPCPAPELLGGPSVFLFPPKPKDTLMISRTPEVTCVVVDVSH EDPEVKFNWYVDGVEVHNAKTKPREEQYNSTYRVVSVLTVLH QDWLNGKEYKCKVSNKALPAPIEKTISKAKGQPREPQVYTLPPS RDELTKNQVSLTCLVKGFYPSDIAVEWESNGQPENNYKTTPPVL DSDGSFFLYSKLTVDKSRWQQGNVFSCSVMHEALHNHYTQKSL SLSPG |
| SEQ ID NO: 53 | |
| C71 | EFGRPFVEMYSEIPEIIHMTEGRELVIPCRVTSPNITVTLKKFPLDT LIPDGKRIIWDSRKGFIISNATYKEIGLLTCEATVNGHLYKTNYL THRQTNTIIDVQISTPSPVELCVGETLVLNCTATTPDNTRVQMT WSYPDEKNKNASVRRRIDQSNSHANIFYSVLTIDGMTNSDKGL YTCRVRSGPSFKSVNTSVHIYDCSGGGGSGGGGSGGGGSAESK YGPPCPPCPAPELLGGPSVFLFPPKPKDTLMISRTPEVTCVVVDV SHEDPEVKFNWYVDGVEVHNAKTKPREEQYNSTYRVVSVLTV LHQDWLNGKEYKCKVSNKALPAPIEKTISKAKGQPREPQVYTL PPSRDELTKNQVSLTCLVKGFYPSDIAVEWESNGQPENNYKTTP PVLDSDGSFFLYSKLTVDKSRWQQGNVFSCSVMHEALHNHYTQ KSLSLSPG |
| SEQ ID NO: 54 | |
| C72 | EFGRPFVEMYSEIPEIIHMTEGRELVIPCRVTSPNITVTLKKFPLDT LIPDGKRIIWDSRKGFIISNATYKEIGLLTCEATVNGHLYKTNYL THRQTNTIIDVQISTPSPVELCVGETLVLNCTATTPSNTRVQMTW SYPDEKNKNASVRRRIDQSNSHANIFYSVLTIDGMTNSDKGLYT CRVRSGPSFKSVNTSVHIYDCGSSGGSSGEPKSDATPTCPPCPAP ELLGGPSVFLFPPKPKDTLMISRTPEVTCVVVDVSHEDPEVKFN WYVDGVEVHNAKTKPREEQYNSTYRVVSVLTVLHQDWLNGK EYKCKVSNKALPAPIEKTISKAKGQPREPQVYTLPPSRDELTKN QVSLTCLVKGFYPSDIAVEWESNGQPENNYKTTPPVLDSDGSFF LYSKLTVDKSRWQQGNVFSCSVMHEALHNHYTQKSLSLSPG |
| SEQ ID NO: 55 | |
| C73 | EFGRPFVEMYSEIPEIIHMTEGRELVIPCRVTSPNITVTLKKFPLDT LIPDGKRIIWDSRKGFIISNATYKEIGLLTCEATVNGHLYKTNYL THRQTNTIIDVQISTPSPVELCVGETLVLNCTATTPDNTRVQMT WSYPDEKNKNASVRRRIDQSNSHANIFYSVLTIDGMTNSDKGL YTCRVRSGPSFKSVNTSVHIYDCGSSGGSSGEPKSDATPTCPPCP APELLGGPSVFLFPPKPKDTLMISRTPEVTCVVVDVSHEDPEVKF NWYVDGVEVHNAKTKPREEQYNSTYRVVSVLTVLHQDWLNG KEYKCKVSNKALPAPIEKTISKAKGQPREPQVYTLPPSRDELTK NQVSLTCLVKGFYPSDIAVEWESNGQPENNYKTTPPVLDSDGSF FLYSKLTVDKSRWQQGNVFSCSVMHEALHNHYTQKSLSLSPG |
| SEQ ID NO: 56 | |
| C74 | EFGRPFVEMYSEIPEIIHMTEGRELVIPCRVTSPNITVTLKKFPLDT LIPDGKRIIWDSRKGFIISNATYKEIGLLTCEATVNGHLYKTNYL THRQTNTIIDVQISTPSPVELCVGETLVLNCTATTPDNTRVQMT WSYPDEKNKNASVRRRIDQSNSHANIFYSVLTIDGMTNSDKGL YTCRVRSGPSFKSVNTSVHIYDCSSGDATPTSPPSPAPELLGGPS VFLFPPKPKDTLMISRTPEVTCVVVDVSHEDPEVKFNWYVDGV EVHNAKTKPREEQYNSTYRVVSVLTVLHQDWLNGKEYKCKVS NKALPAPIEKTISKAKGQPREPQVYTLPPSRDELTKNQVSLTCLV KGFYPSDIAVEWESNGQPENNYKTTPPVLDSDGSFFLYSKLTVD KSRWQQGNVFSCSVMHEALHNHYTQKSLSLSPG |
| SEQ ID NO: 57 | |
| C75 | EFGRPFVEMYSEIPEIIHMTEGRELVIPCRVTSPNITVTLKKFPLDT LIPDGKRIIWDSRKGFIISNATYKEIGLLTCEATVNGHLYKTNYL THRQTNTIIDVQISTPSPVELCVGETLVLNCTATTPDNTRVQMT WSYPDEKNKNASVRRRIDQSNSHANIFYSVLTIDGMTNSDKGL YTCRVRSGPSFKSVNTSVHIYDCSNTGSGGEEKKKEKEKEEQEE RSSDTPPTCPPCPAPELLGGPSVFLFPPKPKDTLMISRTPEVTCVV VDVSHEDPEVKFNWYVDGVEVHNAKTKPREEQYNSTYRVVSV LTVLHQDWLNGKEYKCKVSNKALPAPIEKTISKAKGQPREPQV YTLPPSRDELTKNQVSLTCLVKGFYPSDIAVEWESNGQPENNYK TTPPVLDSDGSFFLYSKLTVDKSRWQQGNVFSCSVMHEALHNH YTQKSLSLSPG |
| SEQ ID NO: 58 | |
| C88 | EFGRPFVEMYSEIPEIIHMTEGRELVIPCRVTSPNITVTLKKFPLDT LIPDGKRIIWDSRKGFIISNATYKEIGLLTCEATVNGHLYKTNYL THRQTNTIIDVQISTPRPVKLLRGHTLVLNCTATTPLNTRVQMT WSYPDEKNKRASVRRRIDQSNSHANIFYSVLTIDKMQNKDKGL YTCRVRSGPSFKSVNTSVHIYDKALEDKTHTCPPCPAPELLGGPS VFLFPPKPKDTLMISRTPEVTCVVVDVSHEDPEVKFNWYVDGV EVHNAKTKPREEQYNSTYRVVSVLTVLHQDWLNGKEYKCKVS NKALPAPIEKTISKAKGQPREPQVYTLPPSRDELTKNQVSLTCLV KGFYPSDIAVEWESNGQPENNYKTTPPVLDSDGSFFLYSKLTVD KSRWQQGNVFSCSVMHEALHNHYTQKSLSLSPG |
| SEQ ID NO: 59 | |
| Fc region | APELLGGPSVFLFPPKPKDTLMISRTPEVTCVVVDVSHEDPEVKF NWYVDGVEVHNAKTKPREEQYNSTYRVVSVLTVLHQDWLNG KEYKCKVSNKALPAPIEKTISKAKGQPREPQVYTLPPSRDELTK NQVSLTCLVKGFYPSDIAVEWESNGQPENNYKTTPPVLDSDGSF FLYSKLTVDKSRWQQGNVFSCSVMHEALHNHYTQKSLSLSPGK |
| SEQ ID NO: 60 | |
| Ig-like domains D2 and D3 of VEGFR1 SEQ ID NO: 61 | GRPFVEMYSEIPEIIHMTEGRELVIPCRVTSPNITVTLKKFPLDTLI PDGKRIIWDSRKGFIISNATYKEIGLLTCEATVNGHLYKTNYLTH RQTNTIIDVQISTPRPVKLLRGHTLVLNCTATTPLNTRVQMTWS YPDEKNKRASVRRRIDQSNSHANIFYSVLTIDKMQNKDKGLYT CRVRSGPSFKSVNTSVHIYDKA |
| C08 (control: VEGF-Grab) | GRPFVEMYSEIPEIIHMTEGRELVIPCRVTSPNITVTLKKFPLDTLI PDGKRIIWDSRKGFIISNATYKEIGLLTCEATVNGHLYKTNYLTH RQTNTIIDVQISTPSPVTLLRGHTLVLNCTATTPLNTRVQMTWSY PDEKNKNASVRRRIDQSNSHANIFYSVLTIDKMQNKDKGLYTC RVRSGPSFKSVNTSVHIYDKDKTHTCPPCPAPELLGGPSVFLFPP KPKDTLMISRTPEVTCVVVDVSHEDPEVKFNWYVDGVEVHNA KTKPREEQYNSTYRVVSVLTVLHQDWLNGKEYKCKVSNKALP APIEKTISKAKGQPREPQVYTLPPSREEMTKNQVSLTCLVKGFYP SDIAVEWESNGQPENNYKTTPPVLDSDGSFFLYSKLTVDKSRW QQGNVFSCSVMHEALHNHYTQKSLSLSPG |
| SEQ ID NO: 62 | |
| C94 | GRPFVEMYSEIPEIIHMTEGRELVIPCRVTSPNITVTLKKFPLDTLI PDGKRIIWDSRKGFIISNATYKEIGLLTCEATVNGHLYKTNYLTH RQTNTIIDVQISTPSPVELCVGETLVLNCTATTPDNTRVQMTWS YPDEKNKNASVRRRIDQSNSHANIFYSVLTIDGMTNSDKGLYTC RVRSGPSFKSVNTSVHIYDCGSSGGSSGEPKSDATPTCPPCPAPE LLGGPSVFLFPPKPKDTLMISRTPEVTCVVVDVSHEDPEVKFNW YVDGVEVHNAKTKPREEQYNSTYRVVSVLTVLHQDWLNGKEY KCKVSNKALPAPIEKTISKAKGQPREPQVYTLPPSRDELTKNQVS LTCLVKGFYPSDIAVEWESNGQPENNYKTTPPVLDSDGSFFLYS KLTVDKSRWQQGNVFSCSVMHEALHNHYTQKSLSLSPG |
| SEQ ID NO: 63 | |
| C95 | GRPFVEMYSEIPEIIHMTEGRELVIPCRVTSPNITVTLKKFPLDTLI PDGKRIIWDSRKGFIISNATYKEIGLLTCEATVNGHLYKTNYLTH RQTNTIIDVQISTPSPVELCVGETLVLNCTATTPDNTRVQMTWS YPDEKNKNASVRRRIDQSNSHANIFYSVLTIDGMTNSDKGLYTC RVRSGPSFKSVNTSVHIYDCGSSGGSSGEPKSDATPTCPPCPAPE LLGGPSVFLFPPKPKDTLMISRTPEVTCVVVDVSHEDPEVKFNW YVDGVEVHNAKTKPREEQYNSTYRVVSVLTVLHQDWLNGKEY KCKVSNKALPAPIEKTISKAKGQPREPQVYTLPPSRDELTKNQVS LTCLVKGFYPSDIAVEWESNGQPENNYKTTPPVLDSDGSFFLYS KLTVDKSRWQQGNVFSCSVMHEALHNHYTQKSLSLSPG |
| SEQ ID NO: 64 | |
| C96 | GRPFVEMYSEIPEIIHMTEGRELVIPCRVTSPNITVTLKKFPLDTLI PDGKRIIWDSRKGFIISNATYKEIGLLTCEATVNGHLYKTNYLTH RQTNTIIDVQISTPSPVELCVGETLVLNCTATTPDNTRVQMTWS YPDEKNKNASVRRRIDQSNSHANIFYSVLTIDGMTNSDKGLYTC RVRSGPSFKSVNTSVHIYDCSNTGSGGEEKKKEKEKEEQEERS C DTPPTCPPCPAPELLGGPSVFLFPPKPKDTLMISRTPEVTCVVVD VSHEDPEVKFNWYVDGVEVHNAKTKPREEQYNSTYRVVSVLT VLHQDWLNGKEYKCKVSNKALPAPIEKTISKAKGQPREPQVYT LPPSRDELTKNQVSLTCLVKGFYPSDIAVEWESNGQPENNYKTT PPVLDSDGSFFLYSKLTVDKSRWQQGNVFSCSVMHEALHNHYT QKSLSLSPG |
| SEQ ID NO: 65 | |
| C97 | GRPFVEMYSEIPEIIHMTEGRELVIPCRVTSPNITVTLKKFPLDTLI PDGKRIIWDSRKGFIISNATYKEIGLLTCEATVNGHLYKTNYLTH RQTNTIIDVQISTPSPVELCVGETLVLNCTATTPDNTRVQMTWS YPDEKNKNASVRRRIDQSNSHANIFYSVLTIDGMTNSDKGLYTC RVRSGPSFKSVNTSVHIYDCSGGGGSGGGGSGGGGSAESKYGPP CPPCPAPELLGGPSVFLFPPKPKDTLMISRTPEVTCVVVDVSHED PEVKFNWYVDGVEVHNAKTKPREEQYNSTYRVVSVLTVLHQD WLNGKEYKCKVSNKALPAPIEKTISKAKGQPREPQVYTLPPSRD ELTKNQVSLTCLVKGFYPSDIAVEWESNGQPENNYKTTPPVLDS DGSFFLYSKLTVDKSRWQQGNVFSCSVMHEALHNHYTQKSLSL SPG |
| SEQ ID NO: 66 | |
| C99 | GRPFVEMYSEIPEIIHMTEGRELVIPCRVTSPNITVTLKKFPLDTLI PDGKRIIWDSRKGFIISNATYKEIGLLTCEATVNGHLYKTNYLTH RQTNTIIDVQISTPSPVELCVGETLVLNCTATTPDNTRVQMTWS YPDEKNKNASVRRRIDQSNSHANIFYSVLTIDGMTNSDKGLYTC RVRSGPSFKSVNTSVHIYDCGSSGGSSGEPKSDATPTCPPCPAPE LLGGPSVFLFPPKPKDTLMISRTPEVTCVVVDVSHEDPEVKFNW YVDGVEVHNAKTKPREEQYNSTYRVVSVLTVLHQDWLNGKEY KCKVSNKALPAPIEKTISKAKGQPREPQVYTLPPSRDELTKNQVS LTCLVKGFYPSDIAVEWESNGQPENNYKTTPPVLDSDGSFFLYS KLTVDKSRWQQGNVFSCSVMHEALHNHYTQKSLSLSPG |
| SEQ ID NO: 67 | |

The surface charge variation positions or disulfide bond positions described in the following Examples or Tables refer to the varied positions and amino acid residues based on the amino acid sequence of VEGF-Grab3 in Table 2. The physicochemical properties and binding affinities of combination variants prepared with variations introduced at different positions as shown in Table 2 were evaluated. Results of physicochemical property evaluation for variants prepared based on VEGFR wild-type protein and the wild-type protein are shown in Table 3, and binding affinity evaluation results are shown in Tables 4 to 5. Physicochemical property evaluation results of variants prepared based on VEGF-Grab3 are shown in Table 6, and binding affinity evaluation results are shown in Tables 7 and 8, wherein Table 7 shows relative binding affinity evaluation results with respect to VEGF-Grab3 (manufactured by Panolos Bioscience), and Table 8 shows relative binding affinity evaluation results with respect to VEGF-Grab3 (manufactured by Samsung Biologics). In the case of relative binding affinity evaluation with respect to VEGF-Grab3 (manufactured by Panolos Bioscience), comparisons were made with products produced under the same conditions and batch as the variants.

**[TABLE 3]**

| Protein code | Titer (g/L) | Elution (mg) | Step yield (%) | HMWS (%) | of functional monomer (%) | LMWS (%) |
|---|---|---|---|---|---|---|
| C65 | 0.185 | 6.9 | 123.1 | 36.1 | 62.9 | 1.1 |
| C73 | 0.143 | 5.0 | 116.4 | 20.2 | 79.8 | 0.0 |

**[TABLE 4]**

| Protein code | Binding affinity to VEGF-A | | | Binding affinity to VEGF-A relative to VEGFR-wild type protein C88 (increase fold) | | |
|---|---|---|---|---|---|---|
| | KD(M) | kon(1/Ms) | kdis(l/s) | fold(KD) | fold(kon) | fold(kdis) |
| C65 | 8.46E-11 | 5.16E+05 | 4.37E-05 | 0.72 | 0.89 | 0.80 |
| C73 | 1.53E-13 | 6.52E+05 | <1.0E-07 | > 60.86 | 1.13 | > 100 |

**[TABLE 5]**

| Protein code | Binding affinity to PlGF | | | Binding affinity to PlGF relative to VEGFR- wild type protein C88 (increase fold) | | |
|---|---|---|---|---|---|---|
| | KD(M) | kon(1/Ms) | kdis(l/s) | fold(KD) | fold(kon) | fold(kdis) |
| C65 | 1.37E-10 | 2.41E+06 | 3.31E-04 | 1.49 | 1.99 | 0.75 |
| C73 | 1.68E-10 | 2.22E+06 | 3.72E-04 | 1.22 | 1.83 | 0.67 |

**[TABLE 6]**

| Protein code | Titer (g/L) | Elution (mg) | Step yield (%) | HMWS (%) | Content of functional monomer (%) | LMWS (%) |
|---|---|---|---|---|---|---|
| VEGF-Grab1 | 0.527 | 8.6 | 46.7 | 33.1 | 43.0 | 23.8 |
| VEGF-Grab3 | 0.359 | 5.4 | 50.5 | 53.9 | 44.3 | 1.7 |
| C61 | 0.537 | 10 | 62.2 | 10.2 | 89.8 | 0.0 |
| C62 | 0.231 | 5.9 | 85.7 | 11.2 | 88.8 | 0.0 |
| C63 | 0.505 | 9.6 | 63.4 | 9.5 | 90.5 | 0.0 |
| C64 | 0.171 | 6.2 | 121.6 | 13.7 | 86.3 | 0.0 |
| C65 | 0.66 | 8.5 | 42.7 | 9.7 | 90.3 | 0.0 |
| C66 | 0.212 | 6.2 | 98.1 | 20.7 | 79.4 | 0.0 |
| C67 | 0.437 | 5.8 | 43.9 | 5 | 95.1 | 0.0 |
| C68 | 0.153 | 6.3 | 137.3 | 45 | 55.0 | 0.0 |
| C69 | 0.378 | 5 | 44.4 | 2.6 | 97.4 | 0.0 |
| C70 | 0.61 | 8 | 43.9 | 13.2 | 86.9 | 0.0 |
| C71 | 0.698 | 7.1 | 33.8 | 4.6 | 95.4 | 0.0 |
| C72 | 0.451 | 7.3 | 54.1 | 9.5 | 90.5 | 0.0 |
| C73 | 0.408 | 7.1 | 58.3 | 3.1 | 96.9 | 0.0 |
| C74 | 0.535 | 5.8 | 35.9 | 6.7 | 93.3 | 0.0 |
| C75 | 0.295 | 8.2 | 92.2 | 0.4 | 99.6 | 0.0 |

**[TABLE 7]**

| Protein code | Binding affinity to VEGF-A (increase fold) | | | Binding affinity to PlGF (increase fold) | | |
|---|---|---|---|---|---|---|
| | fold(KD) | fold(kon) | fold(kdis) | fold(KD) | fold(kon) | fold(kdis) |
| C61 | 1.35 | 0.74 | 1.83 | 1.24 | 0.76 | 1.62 |
| C62 | > 100 | 1.27 | > 100 | 1.93 | 1.70 | 1.13 |
| C63 | 1.54 | 0.70 | 2.21 | 1.38 | 0.89 | 1.56 |
| C64 | 2.54 | 1.39 | 1.83 | 1.82 | 1.70 | 1.07 |
| C65 | 2.12 | 0.72 | 2.93 | 1.83 | 0.91 | 2.00 |
| C66 | 1.46 | 1.24 | 1.18 | 3.77 | 1.25 | 3.01 |
| C67 | 1.95 | 0.94 | 2.06 | 1.03 | 0.96 | 1.07 |
| C68 | > 100 | 1.83 | > 100 | 1.21 | 2.43 | 0.50 |
| C69 | > 100 | 1.37 | > 100 | 0.83 | 1.24 | 0.67 |
| C70 | 3.01 | 1.20 | 2.52 | 1.38 | 1.30 | 1.06 |
| C71 | 3.06 | 0.99 | 3.10 | 1.43 | 0.82 | 1.75 |
| C72 | 0.95 | 0.82 | 1.15 | 1.48 | 0.70 | 2.11 |
| C73 | > 100 | 1.29 | > 100 | 2.10 | 0.66 | 3.18 |
| C74 | > 100 | 2.09 | > 100 | 1.07 | 2.06 | 0.52 |
| C75 | 0.27 | 1.38 | 0.20 | 0.88 | 2.07 | 0.42 |

**[TABLE 8]**

| Protein code | Binding affinity to VEGF-A (increase fold) | | | Binding affinity to PlGF (increase fold) | | |
|---|---|---|---|---|---|---|
| | fold(KD) | fold(kon) | fold(kdis) | fold(KD) | fold(kon) | fold(kdis) |
| C61 | < 0.01 | 0.94 | < 0.01 | 1.53 | 1.14 | 1.35 |
| C62 | 1.83 | 0.55 | 1.00 | 2.39 | 2.54 | 0.94 |
| C63 | < 0.01 | 0.99 | < 0.01 | 1.71 | 1.32 | 1.30 |
| C64 | 0.01 | 0.50 | < 0.01 | 2.26 | 2.54 | 0.89 |
| C65 | < 0.01 | 0.96 | < 0.01 | 2.26 | 1.36 | 1.66 |
| C66 | < 0.01 | 0.56 | < 0.01 | 4.67 | 1.87 | 2.50 |
| C67 | < 0.01 | 0.73 | < 0.01 | 1.27 | 1.43 | 0.89 |
| C68 | 2.65 | 0.38 | 1.00 | 1.49 | 3.63 | 0.41 |
| C69 | 0.42 | 0.51 | 0.21 | 1.03 | 1.86 | 0.56 |
| C70 | 0.01 | 0.58 | < 0.01 | 1.70 | 1.93 | 0.88 |
| C71 | 0.01 | 0.70 | < 0.01 | 1.77 | 1.22 | 1.45 |
| C72 | < 0.01 | 0.84 | < 0.01 | 1.84 | 1.05 | 1.75 |
| C73 | 1.87 | 0.54 | 1.00 | 2.60 | 0.99 | 2.64 |
| C74 | 1.17 | 1.17 | 1.00 | 1.47 | 2.54 | 0.58 |
| C75 | < 0.01 | 0.77 | < 0.01 | 1.20 | 2.56 | 0.47 |

According to the results of Tables 3 to 8, it can be confirmed that the modified fusion protein according to an embodiment of the present disclosure exhibits excellent productivity based on titer and protein elution results, and exhibits excellent physicochemical properties based on functional monomer content results. In addition, for example, based on results for kon and the like, it was confirmed that the modified fusion protein also exhibits superior binding affinity to ligands. From these results, it was confirmed that the characteristics introduced during structural design of the modified fusion protein in Examples 2.1 to 2.3 were effectively manifested and that these characteristics acted in combination to exhibit a synergistic effect.

### EXAMPLE 3 - Production of Conjugates

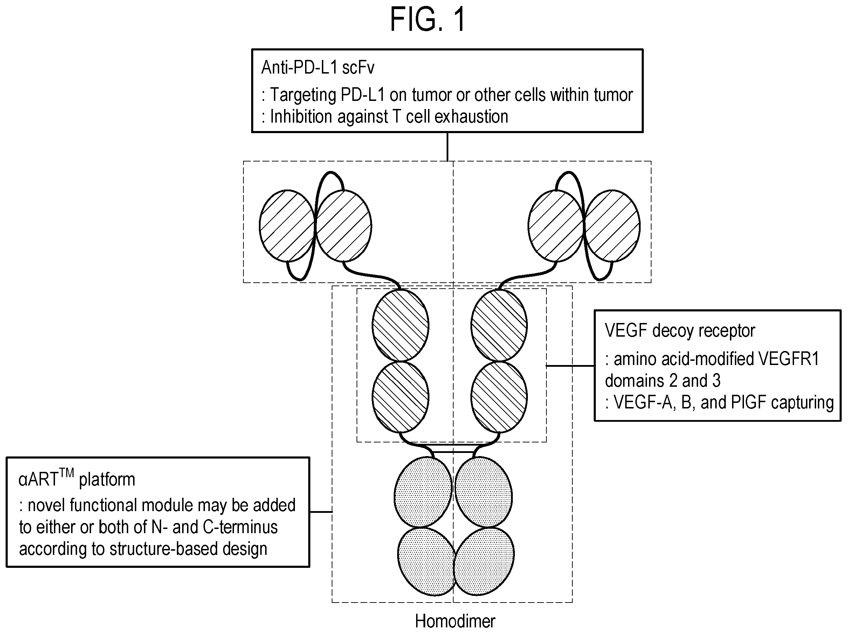

Conjugates were designed by coupling the fusion proteins C61 to C75 prepared in Example 2 above with atezolizumab and A167 scFvs that bind to PD-L1 (FIG. 1). As can be seen in FIG. 1, the fusion protein of the present disclosure includes a VEGF decoy receptor comprising engineered VEGFR1 domains 2 and 3 capable of trapping VEGF-A and PlGF, and a novel functional module may be linked to the N-terminus or the C-terminus thereof. The anti-PD-L1 scFv was linked to the N-terminus of the fusion protein of the present disclosure to prepare the conjugate according to the present disclosure.

**[TABLE 9]**

| Configuration | Sequence |
|---|---|
| Anti-PD-L1 scFv (atezolizumab) | EVQLVESGGGLVQPGGSLRLSCAASGFTFSDSWIHWVRQAPGKGL EWVAWISPYGGSTYYADSVKGRFTISADTSKNTAYLQMNSLRAED TAVYYCARRHWPGGFDYWGQGTLVTVSSGGGGSGGGGSGGGGS GGGGSDIQMTQSPSSLSASVGDRVTITCRASQDVSTAVAWYQQKP GKAPKLLIYSASFLYSGVPSRFSGSGSGTDFTLTISSLQPEDFATYYC QQYLYHPATFGQGTKVEI |
| SEQ ID NO: 39 | |
| Anti-PD-L1 scFv(A167 scFv) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKR |
| SEQ ID NO: 40 | |
| Code Name (Configuration Description) | Sequence |
| H-12C08 (control: A167HG4SL-VEGF-Grab) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSGGGGSEIVLTQSPDTLSVTPKEKVTLTCRASQSIGTNIHWFQ QKPGQSPKLLIKYASESISGVPSRFSGSGSGTDFTLTINSVEAEDAAT YYCQQSNSWPYTFGQGTKLEIKRTVGGGGSEFGRPFVEMYSEIPEII HMTEGRELVIPCRVTSPNITVTLKKFPLDTLIPDGKRIIWDSRKGFIIS NATYKEIGLLTCEATVNGHLYKTNYLTHRQTNTIIDVQISTPSPVTL LRGHTLVLNCTATTPLNTRVQMTWSYPDEKNKNASVRRRIDQSNS HANIFYSVLTIDKMQNKDKGLYTCRVRSGPSFKSVNTSVHIYDKAL EDKTHTCPPCPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVYTLPPSRE EMTKNQVSLTCLVKGFYPSDIAVEWESNGQPENNYKTTPPVLDSD GSFFLYSKLTVDKSRWQQGNVFSCSVMHEALHNHYTQKSLSLSPG K EIVLTQSPDTLSVTPKEKVTLTCRASQSIGTNIHWFQQKPGQSPKLL IKYASESISGVPSRFSGSGSGTDFTLTINSVEAEDAATYYCQQSNSW PYTFGQGTKLEIKRTVGGGGSGGGGSGGGGSGGGGSGGGGSQVQ LQESGPGLVKPSETLSITCTVSGFSLSNYDISWIRQPPGKGLEWLGV IWTGGATNYNPALKSRLTISRDNSKNQVSLKMSSVTAADTAVYYC VRDSNYRYDEPFTYWGQGTLVTVSSGGGGSEFGRPFVEMYSEIPEI IHMTEGRELVIPCRVTSPNITVTLKKFPLDTLIPDGKRIIWDSRKGFII SNATYKEIGLLTCEATVNGHLYKTNYLTHRQTNTIIDVQISTPSPVT LLRGHTLVLNCTATTPLNTRVQMTWSYPDEKNKNASVRRRIDQSN SHANIFYSVLTIDKMQNKDKGLYTCRVRSGPSFKSVNTSVHIYDKA |
| SEQ ID NO: 1 | |
| H-12C09 (control: A167LG4SH-VEGF-Grab) | EIVLTQSPDTLSVTPKEKVTLTCRASQSIGTNIHWFQQKPGQSPKLL IKYASESISGVPSRFSGSGSGTDFTLTINSVEAEDAATYYCQQSNSW PYTFGQGTKLEIKRTVGGGGSGGGGSGGGGSGGGGSGGGGSQVQ LQESGPGLVKPSETLSITCTVSGFSLSNYDISWIRQPPGKGLEWLGV IWTGGATNYNPALKSRLTISRDNSKNQVSLKMSSVTAADTAVYYC VRDSNYRYDEPFTYWGQGTLVTVSSGGGGSEFGRPFVEMYSEIPEI IHMTEGRELVIPCRVTSPNITVTLKKFPLDTLIPDGKRIIWDSRKGFII SNATYKEIGLLTCEATVNGHLYKTNYLTHRQTNTIIDVQISTPSPVT LLRGHTLVLNCTATTPLNTRVQMTWSYPDEKNKNASVRRRIDQSN SHANIFYSVLTIDKMQNKDKGLYTCRVRSGPSFKSVNTSVHIYDKA LEDKTHTCPPCPAPELLGGPSVFLFPPKPKDTLMISRTPEVTCVVVD VSHEDPEVKFNWYVDGVEVHNAKTKPREEQYNSTYRVVSVLTVL HQDWLNGKEYKCKVSNKALPAPIEKTISKAKGQPREPQVYTLPPS REEMTKNQVSLTCLVKGFYPSDIAVEWESNGQPENNYKTTPPVLD SDGSFFLYSKLTVDKSRWQQGNVFSCSVMHEALHNHYTQKSLSLS PGK |
| SEQ ID NO: 2 | |
| H-12C18 (control: A167-3(G4S)-Fc) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSGGGGSEIVLTQSPDTLSVTPKEKVTLTCRASQSIGTNIHWFQ QKPGQSPKLLIKYASESISGVPSRFSGSGSGTDFTLTINSVEAEDAAT YYCQQSNSWPYTFGQGTKLEIKRTVGGGGSGGGGSGGGGSDKTH TCPPCPAPELLGGPSVFLFPPKPKDTLMISRTPEVTCVVVDVSHEDP EVKFNWYVDGVEVHNAKTKPREEQYNSTYRVVSVLTVLHQDWL NGKEYKCKVSNKALPAPIEKTISKAKGQPREPQVYTLPPSREEMTK NQVSLTCLVKGFYPSDIAVEWESNGQPENNYKTTPPVLDSDGSFFL YSKLTVDKSRWQQGNVFSCSVMHEALHNHYTQKSLSLSPGK |
| SEQ ID NO: 3 | |
| H-12C21 (control: VEGF-Grab-3(G4S)-A167) | EFGRPFVEMYSEIPEIIHMTEGRELVIPCRVTSPNITVTLKKFPLDTLI PDGKRIIWDSRKGFIISNATYKEIGLLTCEATVNGHLYKTNYLTHR QTNTIIDVQISTPSPVTLLRGHTLVLNCTATTPLNTRVQMTWSYPDE KNKNASVRRRIDQSNSHANIFYSVLTIDKMQNKDKGLYTCRVRSG PSFKSVNTSVHIYDKALEDKTHTCPPCPAPELLGGPSVFLFPPKPKD TLMISRTPEVTCVVVDVSHEDPEVKFNWYVDGVEVHNAKTKPRE EQYNSTYRVVSVLTVLHQDWLNGKEYKCKVSNKALPAPIEKTISK AKGQPREPQVYTLPPSREEMTKNQVSLTCLVKGFYPSDIAVEWES NGQPENNYKTTPPVLDSDGSFFLYSKLTVDKSRWQQGNVFSCSVM HEALHNHYTQKSLSLSPGKGGGGSGGGGSGGGGSQVQLQESGPG LVKPSETLSITCTVSGFSLSNYDISWIRQPPGKGLEWLGVIWTGGAT NYNPALKSRLTISRDNSKNQVSLKMSSVTAADTAVYYCVRDSNYR YDEPFTYWGQGTLVTVSSGGGGSGGGGSGGGGSGGGGSGGGGSE IVLTQSPDTLSVTPKEKVTLTCRASQSIGTNIHWFQQKPGQSPKLLI KYASESISGVPSRFSGSGSGTDFTLTINSVEAEDAATYYCQQSNSWP YTFGQGTKLEIKRTV |
| SEQ ID NO: 4 | |
| H-12C70 (A167-V3-C66_sur1) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGSGGGGSGGGGSGGGGSGGGGSGRPFV EMYSEIPEIIHMTEGRELVIPCRVTSPNITVTLKKFPLDTLIPDGKRII WDSRKGFIISNATYKEIGLLTCEATVNGHLYKTNYLTHRQTNTIID VQISTPSPVTLCRGHTLVLNCTATTPANTRVQMTWSYPDEKNKNA SVRRRIDQSNSHANIFYSVLTIDGMTNSDQGLYTCRVRSGPSFKSV NTSVHIYDCGSSGGSSGEPKSDATPTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPSRDELTKNQVSLTCLVKGFYPSDIAVEWE SNGQPENNYKTTPPVLDSDGSFFLYSKLTVDKSRWQQGNVFSCSV MHEALHNHYTQKSLSLSPG |
| SEQ ID NO: 5 | |
| H-12C71 (A167-V1-C66_sur1) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGGGGSGGGGSDTGRPFVEMYSEIPEIIH MTEGRELVIPCRVTSPNITVTLKKFPLDTLIPDGKRIIWDSRKGFIISN ATYKEIGLLTCEATVNGHLYKTNYLTHRQTNTIIDVQISTPSPVTLC RGHTLVLNCTATTPANTRVQMTWSYPDEKNKNASVRRRIDQSNS HANIFYSVLTIDGMTNSDQGLYTCRVRSGPSFKSVNTSVHIYDCGS SGGSSGEPKSDATPTCPPCPAPELLGGPSVFLFPPKPKDTLMISRTPE VTCVVVDVSHEDPEVKFNWYVDGVEVHNAKTKPREEQYNSTYR VVSVLTVLHQDWLNGKEYKCKVSNKALPAPIEKTISKAKGQPREP QVYTLPPSRDELTKNQVSLTCLVKGFYPSDIAVEWESNGQPENNY KTTPPVLDSDGSFFLYSKLTVDKSRWQQGNVFSCSVMHEALHNHY TQKSLSLSPG |
| SEQ ID NO: 6 | |
| H-12C72 (A167-V3-C65(Cys)_sur1) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGSGGGGSGGGGSGGGGSGGGGSGRPFV EMYSEIPEIIHMTEGRELVIPCRVTSPNITVTLKKFPLDTLIPDGKRII WDSRKGFIISNATYKEIGLLTCEATVNGHLYKTNYLTHRQTNTIID VQISTPSPVTLCRGHTLVLNCTATTPANTRVQMTWSYPDEKNKNA SVRRRIDQSNSHANIFYSVLTIDGMTNSDQGLYTCRVRSGPSFKSV NTSVHIYDCSNTGSGGEEKKKEKEKEEQEERS C DTPPTCPPCPAPE LLGGPSVFLFPPKPKDTLMISRTPEVTCVVVDVSHEDPEVKFNWYV DGVEVHNAKTKPREEQYNSTYRVVSVLTVLHQDWLNGKEYKCK VSNKALPAPIEKTISKAKGQPREPQVYTLPPSRDELTKNQVSLTCLV KGFYPSDIAVEWESNGQPENNYKTTPPVLDSDGSFFLYSKLTVDKS RWQQGNVFSCSVMHEALHNHYTQKSLSLSPG |
| SEQ ID NO: 7 | |
| H-12C73 (A167-V3-C64_sur1) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGSGGGGSGGGGSGGGGSGGGGSGRPFV EMYSEIPEIIHMTEGRELVIPCRVTSPNITVTLKKFPLDTLIPDGKRII WDSRKGFIISNATYKEIGLLTCEATVNGHLYKTNYLTHRQTNTIID VQISTPSPVTLCRGHTLVLNCTATTPANTRVQMTWSYPDEKNKNA SVRRRIDQSNSHANIFYSVLTIDGMTNSDQGLYTCRVRSGPSFKSV NTSVHIYDCSGGGGSGGGGSGGGGSAESKYGPPCPPCPAPELLGGP SVFLFPPKPKDTLMISRTPEVTCVVVDVSHEDPEVKFNWYVDGVE VHNAKTKPREEQYNSTYRVVSVLTVLHQDWLNGKEYKCKVSNK ALPAPIEKTISKAKGQPREPQVYTLPPSRDELTKNQVSLTCLVKGFY PSDIAVEWESNGQPENNYKTTPPVLDSDGSFFLYSKLTVDKSRWQ QGNVFSCSVMHEALHNHYTQKSLSLSPG |
| SEQ ID NO: 8 | |
| H-12C74 (A167-V1-C61_sur1) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGGGGSGGGGSDTGRPFVEMYSEIPEIIH MTEGRELVIPCRVTSPNITVTLKKFPLDTLIPDGKRIIWDSRKGFIISN ATYKEIGLLTCEATVNGHLYKTNYLTHRQTNTIIDVQISTPSPVTLC RGHTLVLNCTATTPANTRVQMTWSYPDEKNKNASVRRRIDQSNS HANIFYSVLTIDGMTNSDQGLYTCRVRSGPSFKSVNTSVHIYDCSS GDATPTSPPSPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVYTLPPSR DELTKNQVSLTCLVKGFYPSDIAVEWESNGQPENNYKTTPPVLDS DGSFFLYSKLTVDKSRWQQGNVFSCSVMHEALHNHYTQKSLSLSP G |
| SEQ ID NO: 9 | |
| H-12C75 (A167-V2-C66_sur1) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGSAEPKAGGGGSGGGGSGGGGSGRPFV EMYSEIPEIIHMTEGRELVIPCRVTSPNITVTLKKFPLDTLIPDGKRII WDSRKGFIISNATYKEIGLLTCEATVNGHLYKTNYLTHRQTNTIID VQISTPSPVTLCRGHTLVLNCTATTPANTRVQMTWSYPDEKNKNA SVRRRIDQSNSHANIFYSVLTIDGMTNSDQGLYTCRVRSGPSFKSV NTSVHIYDCGSSGGSSGEPKSDATPTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPSRDELTKNQVSLTCLVKGFYPSDIAVEWE SNGQPENNYKTTPPVLDSDGSFFLYSKLTVDKSRWQQGNVFSCSV MHEALHNHYTQKSLSLSPG |
| SEQ ID NO: 10 | |
| H-12C88 (A167-V3-C66) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGSGGGGSGGGGSGGGGSGGGGSGRPFV SHEDPEVKFNWYVDGVEVHNAKTKPREEQYNSTYRVVSVLTVLH QDWLNGKEYKCKVSNKALPAPIEKTISKAKGQPREPQVYTLPPSR DELTKNQVSLTCLVKGFYPSDIAVEWESNGQPENNYKTTPPVLDS DGSFFLYSKLTVDKSRWQQGNVFSCSVMHEALHNHYTQKSLSLSP G |
| SEQ ID NO: 11 | |
| H-12C89 (A167-V1-C66) | EMYSEIPEIIHMTEGRELVIPCRVTSPNITVTLKKFPLDTLIPDGKRII WDSRKGFIISNATYKEIGLLTCEATVNGHLYKTNYLTHRQTNTIID VQISTPSPVELCVGETLVLNCTATTPANTRVQMTWSYPDEKNKNA SVRRRIDQSNSHANIFYSVLTIDGMTNSDKGLYTCRVRSGPSFKSV NTSVHIYDCGSSGGSSGEPKSDATPTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPSRDELTKNQVSLTCLVKGFYPSDIAVEWE SNGQPENNYKTTPPVLDSDGSFFLYSKLTVDKSRWQQGNVFSCSV MHEALHNHYTQKSLSLSPG |
| SEQ ID NO: 12 | |
| H-12C90 (A167-V3-C65(Cys)) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGGGGSGGGGSDTGRPFVEMYSEIPEIIH MTEGRELVIPCRVTSPNITVTLKKFPLDTLIPDGKRIIWDSRKGFIISN ATYKEIGLLTCEATVNGHLYKTNYLTHRQTNTIIDVQISTPSPVELC VGETLVLNCTATTPANTRVQMTWSYPDEKNKNASVRRRIDQSNS HANIFYSVLTIDGMTNSDKGLYTCRVRSGPSFKSVNTSVHIYDCGS SGGSSGEPKSDATPTCPPCPAPELLGGPSVFLFPPKPKDTLMISRTPE VTCVVVDVSHEDPEVKFNWYVDGVEVHNAKTKPREEQYNSTYR VVSVLTVLHQDWLNGKEYKCKVSNKALPAPIEKTISKAKGQPREP QVYTLPPSRDELTKNQVSLTCLVKGFYPSDIAVEWESNGQPENNY KTTPPVLDSDGSFFLYSKLTVDKSRWQQGNVFSCSVMHEALHNHY TQKSLSLSPG |
| SEQ ID NO: 13 | LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGSGGGGSGGGGSGGGGSGGGGSGRPFV EMYSEIPEIIHMTEGRELVIPCRVTSPNITVTLKKFPLDTLIPDGKRII WDSRKGFIISNATYKEIGLLTCEATVNGHLYKTNYLTHRQTNTIID VQISTPSPVELCVGETLVLNCTATTPANTRVQMTWSYPDEKNKNA SVRRRIDQSNSHANIFYSVLTIDGMTNSDKGLYTCRVRSGPSFKSV NTSVHIYDCSNTGSGGEEKKKEKEKEEQEERS C DTPPTCPPCPAPE LLGGPSVFLFPPKPKDTLMISRTPEVTCVVVDVSHEDPEVKFNWYV DGVEVHNAKTKPREEQYNSTYRVVSVLTVLHQDWLNGKEYKCK VSNKALPAPIEKTISKAKGQPREPQVYTLPPSRDELTKNQVSLTCLV KGFYPSDIAVEWESNGQPENNYKTTPPVLDSDGSFFLYSKLTVDKS RWQQGNVFSCSVMHEALHNHYTQKSLSLSPG |
| H-12C91 (A167-V3-C64) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGSGGGGSGGGGSGGGGSGGGGSGRPFV EMYSEIPEIIHMTEGRELVIPCRVTSPNITVTLKKFPLDTLIPDGKRII WDSRKGFIISNATYKEIGLLTCEATVNGHLYKTNYLTHRQTNTIID VQISTPSPVELCVGETLVLNCTATTPANTRVQMTWSYPDEKNKNA SVRRRIDQSNSHANIFYSVLTIDGMTNSDKGLYTCRVRSGPSFKSV NTSVHIYDCSGGGGSGGGGSGGGGSAESKYGPPCPPCPAPELLGGP SVFLFPPKPKDTLMISRTPEVTCVVVDVSHEDPEVKFNWYVDGVE VHNAKTKPREEQYNSTYRVVSVLTVLHQDWLNGKEYKCKVSNK ALPAPIEKTISKAKGQPREPQVYTLPPSRDELTKNQVSLTCLVKGFY PSDIAVEWESNGQPENNYKTTPPVLDSDGSFFLYSKLTVDKSRWQ QGNVFSCSVMHEALHNHYTQKSLSLSPG |
| SEQ ID NO: 14 | |
| H-12C92 (A167-V1-C61) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGGGGSGGGGSDTGRPFVEMYSEIPEIIH MTEGRELVIPCRVTSPNITVTLKKFPLDTLIPDGKRIIWDSRKGFIISN ATYKEIGLLTCEATVNGHLYKTNYLTHRQTNTIIDVQISTPSPVELC VGETLVLNCTATTPANTRVQMTWSYPDEKNKNASVRRRIDQSNS HANIFYSVLTIDGMTNSDKGLYTCRVRSGPSFKSVNTSVHIYDCSS GDATPTSPPSPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVYTLPPSR DELTKNQVSLTCLVKGFYPSDIAVEWESNGQPENNYKTTPPVLDS DGSFFLYSKLTVDKSRWQQGNVFSCSVMHEALHNHYTQKSLSLSP G |
| SEQ ID NO: 15 | |
| H-12C93 (A167-V2-C66) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGSAEPKAGGGGSGGGGSGGGGSGRPFV EMYSEIPEIIHMTEGRELVIPCRVTSPNITVTLKKFPLDTLIPDGKRII WDSRKGFIISNATYKEIGLLTCEATVNGHLYKTNYLTHRQTNTIID VQISTPSPVELCVGETLVLNCTATTPANTRVQMTWSYPDEKNKNA SVRRRIDQSNSHANIFYSVLTIDGMTNSDKGLYTCRVRSGPSFKSV NTSVHIYDCGSSGGSSGEPKSDATPTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPSRDELTKNQVSLTCLVKGFYPSDIAVEWE SNGQPENNYKTTPPVLDSDGSFFLYSKLTVDKSRWQQGNVFSCSV MHEALHNHYTQKSLSLSPG |
| SEQ ID NO: 16 | |
| H-12C94 (A167-V3-C73) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGSGGGGSGGGGSGGGGSGGGGSGRPFV EMYSEIPEIIHMTEGRELVIPCRVTSPNITVTLKKFPLDTLIPDGKRII WDSRKGFIISNATYKEIGLLTCEATVNGHLYKTNYLTHRQTNTIID VQISTPSPVELCVGETLVLNCTATTPDNTRVQMTWSYPDEKNKNA SVRRRIDQSNSHANIFYSVLTIDGMTNSDKGLYTCRVRSGPSFKSV NTSVHIYDCGSSGGSSGEPKSDATPTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPSRDELTKNQVSLTCLVKGFYPSDIAVEWE SNGQPENNYKTTPPVLDSDGSFFLYSKLTVDKSRWQQGNVFSCSV MHEALHNHYTQKSLSLSPG |
| SEQ ID NO: 17 | |
| H-12C95 (A167-V1-C73) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGGGGSGGGGSDTGRPFVEMYSEIPEIIH MTEGRELVIPCRVTSPNITVTLKKFPLDTLIPDGKRIIWDSRKGFIISN ATYKEIGLLTCEATVNGHLYKTNYLTHRQTNTIIDVQISTPSPVELC VGETLVLNCTATTPDNTRVQMTWSYPDEKNKNASVRRRIDQSNS HANIFYSVLTIDGMTNSDKGLYTCRVRSGPSFKSVNTSVHIYDCGS SGGSSGEPKSDATPTCPPCPAPELLGGPSVFLFPPKPKDTLMISRTPE VTCVVVDVSHEDPEVKFNWYVDGVEVHNAKTKPREEQYNSTYR VVSVLTVLHQDWLNGKEYKCKVSNKALPAPIEKTISKAKGQPREP QVYTLPPSRDELTKNQVSLTCLVKGFYPSDIAVEWESNGQPENNY KTTPPVLDSDGSFFLYSKLTVDKSRWQQGNVFSCSVMHEALHNHY TQKSLSLSPG |
| H-12C96 (A167-V3-C75(Cys)) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGSGGGGSGGGGSGGGGSGGGGSGRPFV EMYSEIPEIIHMTEGRELVIPCRVTSPNITVTLKKFPLDTLIPDGKRII WDSRKGFIISNATYKEIGLLTCEATVNGHLYKTNYLTHRQTNTIID VQISTPSPVELCVGETLVLNCTATTPDNTRVQMTWSYPDEKNKNA SVRRRIDQSNSHANIFYSVLTIDGMTNSDKGLYTCRVRSGPSFKSV NTSVHIYDCSNTGSGGEEKKKEKEKEEQEERS C DTPPTCPPCPAPE LLGGPSVFLFPPKPKDTLMISRTPEVTCVVVDVSHEDPEVKFNWYV DGVEVHNAKTKPREEQYNSTYRVVSVLTVLHQDWLNGKEYKCK VSNKALPAPIEKTISKAKGQPREPQVYTLPPSRDELTKNQVSLTCLV KGFYPSDIAVEWESNGQPENNYKTTPPVLDSDGSFFLYSKLTVDKS RWQQGNVFSCSVMHEALHNHYTQKSLSLSPG |
| SEQ ID NO: 19 | |
| H-12C97 (A167-V3-C71) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGSGGGGSGGGGSGGGGSGGGGSGRPFV EMYSEIPEIIHMTEGRELVIPCRVTSPNITVTLKKFPLDTLIPDGKRII WDSRKGFIISNATYKEIGLLTCEATVNGHLYKTNYLTHRQTNTIID VQISTPSPVELCVGETLVLNCTATTPDNTRVQMTWSYPDEKNKNA SVRRRIDQSNSHANIFYSVLTIDGMTNSDKGLYTCRVRSGPSFKSV NTSVHIYDCSGGGGSGGGGSGGGGSAESKYGPPCPPCPAPELLGGP SVFLFPPKPKDTLMISRTPEVTCVVVDVSHEDPEVKFNWYVDGVE VHNAKTKPREEQYNSTYRVVSVLTVLHQDWLNGKEYKCKVSNK ALPAPIEKTISKAKGQPREPQVYTLPPSRDELTKNQVSLTCLVKGFY PSDIAVEWESNGQPENNYKTTPPVLDSDGSFFLYSKLTVDKSRWQ QGNVFSCSVMHEALHNHYTQKSLSLSPG |
| SEQ ID NO: 20 | |
| H-12C98 (A167-V1-C74) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGGGGSGGGGSDTGRPFVEMYSEIPEIIH MTEGRELVIPCRVTSPNITVTLKKFPLDTLIPDGKRIIWDSRKGFIISN ATYKEIGLLTCEATVNGHLYKTNYLTHRQTNTIIDVQISTPSPVELC VGETLVLNCTATTPDNTRVQMTWSYPDEKNKNASVRRRIDQSNS HANIFYSVLTIDGMTNSDKGLYTCRVRSGPSFKSVNTSVHIYDCSS GDATPTSPPSPAPELLGGPSVFLFPPKPKDTLMISRTPEVTCVVVDV SHEDPEVKFNWYVDGVEVHNAKTKPREEQYNSTYRVVSVLTVLH QDWLNGKEYKCKVSNKALPAPIEKTISKAKGQPREPQVYTLPPSR DELTKNQVSLTCLVKGFYPSDIAVEWESNGQPENNYKTTPPVLDS DGSFFLYSKLTVDKSRWQQGNVFSCSVMHEALHNHYTQKSLSLSP G |
| SEQ ID NO: 21 | |
| H-12C99 (A167-V2-C73) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGSAEPKAGGGGSGGGGSGGGGSGRPFV EMYSEIPEIIHMTEGRELVIPCRVTSPNITVTLKKFPLDTLIPDGKRII WDSRKGFIISNATYKEIGLLTCEATVNGHLYKTNYLTHRQTNTIID VQISTPSPVELCVGETLVLNCTATTPDNTRVQMTWSYPDEKNKNA SVRRRIDQSNSHANIFYSVLTIDGMTNSDKGLYTCRVRSGPSFKSV NTSVHIYDCGSSGGSSGEPKSDATPTCPPCPAPELLGGPSVFLFPPKP KDTLMISRTPEVTCVVVDVSHEDPEVKFNWYVDGVEVHNAKTKP REEQYNSTYRVVSVLTVLHQDWLNGKEYKCKVSNKALPAPIEKTI SKAKGQPREPQVYTLPPSRDELTKNQVSLTCLVKGFYPSDIAVEWE SNGQPENNYKTTPPVLDSDGSFFLYSKLTVDKSRWQQGNVFSCSV MHEALHNHYTQKSLSLSPG |
| SEQ ID NO: 22 | |
| H-32D01 (A167-V2-C65) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGSAEPKAGGGGSGGGGSGGGGSGRPFV EMYSEIPEIIHMTEGRELVIPCRVTSPNITVTLKKFPLDTLIPDGKRII WDSRKGFIISNATYKEIGLLTCEATVNGHLYKTNYLTHRQTNTIID VQISTPSPVELCVGETLVLNCTATTPANTRVQMTWSYPDEKNKNA SVRRRIDQSNSHANIFYSVLTIDGMTNSDKGLYTCRVRSGPSFKSV NTSVHIYDCSNTGSGGEEKKKEKEKEEQEERSSDTPPTCPPCPAPEL LGGPSVFLFPPKPKDTLMISRTPEVTCVVVDVSHEDPEVKFNWYV DGVEVHNAKTKPREEQYNSTYRVVSVLTVLHQDWLNGKEYKCK VSNKALPAPIEKTISKAKGQPREPQVYTLPPSRDELTKNQVSLTCLV KGFYPSDIAVEWESNGQPENNYKTTPPVLDSDGSFFLYSKLTVDKS RWQQGNVFSCSVMHEALHNHYTQKSLSLSPG |
| SEQ ID NO: 23 | |
| H-32D02 (A167-V3-C65) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGSGGGGSGGGGSGGGGSGGGGSGRPFV EMYSEIPEIIHMTEGRELVIPCRVTSPNITVTLKKFPLDTLIPDGKRII WDSRKGFIISNATYKEIGLLTCEATVNGHLYKTNYLTHRQTNTIID VQISTPSPVELCVGETLVLNCTATTPANTRVQMTWSYPDEKNKNA SVRRRIDQSNSHANIFYSVLTIDGMTNSDKGLYTCRVRSGPSFKSV NTSVHIYDCSNTGSGGEEKKKEKEKEEQEERSSDTPPTCPPCPAPEL LGGPSVFLFPPKPKDTLMISRTPEVTCVVVDVSHEDPEVKFNWYV DGVEVHNAKTKPREEQYNSTYRVVSVLTVLHQDWLNGKEYKCK VSNKALPAPIEKTISKAKGQPREPQVYTLPPSRDELTKNQVSLTCLV KGFYPSDIAVEWESNGQPENNYKTTPPVLDSDGSFFLYSKLTVDKS RWQQGNVFSCSVMHEALHNHYTQKSLSLSPG |
| SEQ ID NO: 24 | |
| H-32D03 (A167-V2-G4H-G4Fc) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGSAEPKAGGGGSGGGGSGGGGSGRPFV EMYSEIPEIIHMTEGRELVIPCRVTSPNITVTLKKFPLDTLIPDGKRII WDSRKGFIISNATYKEIGLLTCEATVNGHLYKTNYLTHRQTNTIID VQISTPSPVELCVGETLVLNCTATTPANTRVQMTWSYPDEKNKNA SVRRRIDQSNSHANIFYSVLTIDGMTNSDKGLYTCRVRSGPSFKSV NTSVHIYDCSGGGGSGGGGSGGGGSAESKYGPPCPPCPAPEAAGG PSVFLFPPKPKDTLMISRTPEVTCVVVDVSQEDPEVQFNWYVDGV EVHNAKTKPREEQFNSTYRVVSVLTVLHQDWLNGKEYKCKVSNK GLPSSIEKTISKAKGQPREPQVYTLPPSQEEMTKNQVSLTCLVKGFY PSDIAVEWESNGQPENNYKTTPPVLDSDGSFFLYSRLTVDKSRWQE GNVFSCSVMHEALHNHYTQKSLSLSLG |
| SEQ ID NO: 25 | |
| H-32D04 (A167-V3-G4H-G4Fc) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGSGGGGSGGGGSGGGGSGGGGSGRPFV EMYSEIPEIIHMTEGRELVIPCRVTSPNITVTLKKFPLDTLIPDGKRII WDSRKGFIISNATYKEIGLLTCEATVNGHLYKTNYLTHRQTNTIID VQISTPSPVELCVGETLVLNCTATTPANTRVQMTWSYPDEKNKNA SVRRRIDQSNSHANIFYSVLTIDGMTNSDKGLYTCRVRSGPSFKSV NTSVHIYDCSGGGGSGGGGSGGGGSAESKYGPPCPPCPAPEAAGG PSVFLFPPKPKDTLMISRTPEVTCVVVDVSQEDPEVQFNWYVDGV EVHNAKTKPREEQFNSTYRVVSVLTVLHQDWLNGKEYKCKVSNK GLPSSIEKTISKAKGQPREPQVYTLPPSQEEMTKNQVSLTCLVKGFY PSDIAVEWESNGQPENNYKTTPPVLDSDGSFFLYSRLTVDKSRWQE GNVFSCSVMHEALHNHYTQKSLSLSLG |
| SEQ ID NO: 26 | |
| H-32D05 (A167-V2-C65H-G4Fc) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGSAEPKAGGGGSGGGGSGGGGSGRPFV EMYSEIPEIIHMTEGRELVIPCRVTSPNITVTLKKFPLDTLIPDGKRII WDSRKGFIISNATYKEIGLLTCEATVNGHLYKTNYLTHRQTNTIID VQISTPSPVELCVGETLVLNCTATTPANTRVQMTWSYPDEKNKNA SVRRRIDQSNSHANIFYSVLTIDGMTNSDKGLYTCRVRSGPSFKSV NTSVHIYDCSNTGSGGEEKKKEKEKEEQEERSSDTPPTCPPCPAPEA AGGPSVFLFPPKPKDTLMISRTPEVTCVVVDVSQEDPEVQFNWYV DGVEVHNAKTKPREEQFNSTYRVVSVLTVLHQDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVMHEALHNHYTQKSLSLSLG |
| SEQ ID NO: 27 | |
| H-32D06 (A167-V3-C65H-G4Fc) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGSGGGSGGGGSGGGGSGGGGSGRPFV EMYSEIPEIIHMTEGRELVIPCRVTSPNITVTLKKFPLDTLIPDGKRII WDSRKGFIISNATYKEIGLLTCEATVNGHLYKTNYLTHRQTNTIID VQISTPSPVELCVGETLVLNCTATTPANTRVQMTWSYPDEKNKNA SVRRRIDQSNSHANIFYSVLTIDGMTNSDKGLYTCRVRSGPSFKSV NTSVHIYDCSNTGSGGEEKKKEKEKEEQEERSSDTPPTCPPCPAPEA AGGPSVFLFPPKPKDTLMISRTPEVTCVVVDVSQEDPEVQFNWYV DGVEVHNAKTKPREEQFNSTYRVVSVLTVLHQDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVMHEALHNHYTQKSLSLSLG |
| SEQ ID NO: 28 | |
| H-32D07 (A167-V2-C65H-G4Fc+QL) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGSAEPKAGGGGSGGGGSGGGGSGRPFV EMYSEIPEIIHMTEGRELVIPCRVTSPNITVTLKKFPLDTLIPDGKRII WDSRKGFIISNATYKEIGLLTCEATVNGHLYKTNYLTHRQTNTIID VQISTPSPVELCVGETLVLNCTATTPANTRVQMTWSYPDEKNKNA SVRRRIDQSNSHANIFYSVLTIDGMTNSDKGLYTCRVRSGPSFKSV NTSVHIYDCSNTGSGGEEKKKEKEKEEQEERSSDTPPTCPPCPAPEA AGGPSVFLFPPKPKDQLMISRTPEVTCVVVDVSQEDPEVQFNWYV DGVEVHNAKTKPREEQFNSTYRVVSVLTVLHQDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVLHEALHNHYTQKSLSLSLG |
| SEQ ID NO: 29 | |
| H-32D08 (A167-V3-C65H-G4Fc+QL) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSEIVLTQSPDTLSVTPKEKVTLTCRASQSIGTNIHWFQQKPGQS PKLLIKYASESISGVPSRFSGSGSGTDFTLTINSVEAEDAATYYCQQ SNSWPYTFGQGTKLEIKRGSGGGGSGGGGSGGGGSGGGGSGRPFV EMYSEIPEIIHMTEGRELVIPCRVTSPNITVTLKKFPLDTLIPDGKRII WDSRKGFIISNATYKEIGLLTCEATVNGHLYKTNYLTHRQTNTIID VQISTPSPVELCVGETLVLNCTATTPANTRVQMTWSYPDEKNKNA SVRRRIDQSNSHANIFYSVLTIDGMTNSDKGLYTCRVRSGPSFKSV NTSVHIYDCSNTGSGGEEKKKEKEKEEQEERSSDTPPTCPPCPAPEA AGGPSVFLFPPKPKDQLMISRTPEVTCVVVDVSQEDPEVQFNWYV DGVEVHNAKTKPREEQFNSTYRVVSVLTVLHQDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVLHEALHNHYTQKSLSLSLG |
| SEQ ID NO: 30 | |
| H-32D09 (Ate-V2-C65) | EVQLVESGGGLVQPGGSLRLSCAASGFTFSDSWIHWVRQAPGKGL EWVAWISPYGGSTYYADSVKGRFTISADTSKNTAYLQMNSLRAED TAVYYCARRHWPGGFDYWGQGTLVTVSSGGGGSGGGGSGGGGS GGGGSDIQMTQSPSSLSASVGDRVTITCRASQDVSTAVAWYQQKP GKAPKLLIYSASFLYSGVPSRFSGSGSGTDFTLTISSLQPEDFATYYC QQYLYHPATFGQGTKVEIKRGSAEPKAGGGGSGGGGSGGGGSGR PFVEMYSEIPEIIHMTEGRELVIPCRVTSPNITVTLKKFPLDTLIPDGK RIIWDSRKGFIISNATYKEIGLLTCEATVNGHLYKTNYLTHRQTNTII DVQISTPSPVELCVGETLVLNCTATTPANTRVQMTWSYPDEKNKN ASVRRRIDQSNSHANIFYSVLTIDGMTNSDKGLYTCRVRSGPSFKS VNTSVHIYDCSNTGSGGEEKKKEKEKEEQEERSSDTPPTCPPCPAPE LLGGPSVFLFPPKPKDTLMISRTPEVTCVVVDVSHEDPEVKFNWYV DGVEVHNAKTKPREEQYNSTYRVVSVLTVLHQDWLNGKEYKCK VSNKALPAPIEKTISKAKGQPREPQVYTLPPSRDELTKNQVSLTCLV KGFYPSDIAVEWESNGQPENNYKTTPPVLDSDGSFFLYSKLTVDKS RWQQGNVFSCSVMHEALHNHYTQKSLSLSPG |
| SEQ ID NO: 31 | |
| H-32D10 (Ate-V3-C65) | EVQLVESGGGLVQPGGSLRLSCAASGFTFSDSWIHWVRQAPGKGL EWVAWISPYGGSTYYADSVKGRFTISADTSKNTAYLQMNSLRAED TAVYYCARRHWPGGFDYWGQGTLVTVSSGGGGSGGGGSGGGGS GGGGSDIQMTQSPSSLSASVGDRVTITCRASQDVSTAVAWYQQKP GKAPKLLIYSASFLYSGVPSRFSGSGSGTDFTLTISSLQPEDFATYYC QQYLYHPATFGQGTKVEIKRGSGGGGSGGGGSGGGGSGGGGSGR PFVEMYSEIPEIIHMTEGRELVIPCRVTSPNITVTLKKFPLDTLIPDGK RIIWDSRKGFIISNATYKEIGLLTCEATVNGHLYKTNYLTHRQTNTII DVQISTPSPVELCVGETLVLNCTATTPANTRVQMTWSYPDEKNKN ASVRRRIDQSNSHANIFYSVLTIDGMTNSDKGLYTCRVRSGPSFKS VNTSVHIYDCSNTGSGGEEKKKEKEKEEQEERSSDTPPTCPPCPAPE LLGGPSVFLFPPKPKDTLMISRTPEVTCVVVDVSHEDPEVKFNWYV DGVEVHNAKTKPREEQYNSTYRVVSVLTVLHQDWLNGKEYKCK VSNKALPAPIEKTISKAKGQPREPQVYTLPPSRDELTKNQVSLTCLV KGFYPSDIAVEWESNGQPENNYKTTPPVLDSDGSFFLYSKLTVDKS RWQQGNVFSCSVMHEALHNHYTQKSLSLSPG |
| SEQ ID NO: 32 | |
| H-32D11 (Ate-V2-G4H-G4Fc+QL) | EVQLVESGGGLVQPGGSLRLSCAASGFTFSDSWIHWVRQAPGKGL EWVAWISPYGGSTYYADSVKGRFTISADTSKNTAYLQMNSLRAED TAVYYCARRHWPGGFDYWGQGTLVTVSSGGGGSGGGGSGGGGS GGGGSDIQMTQSPSSLSASVGDRVTITCRASQDVSTAVAWYQQKP GKAPKLLIYSASFLYSGVPSRFSGSGSGTDFTLTISSLQPEDFATYYC QQYLYHPATFGQGTKVEIKRGSAEPKAGGGGSGGGGSGGGGSGR PFVEMYSEIPEIIHMTEGRELVIPCRVTSPNITVTLKKFPLDTLIPDGK RIIWDSRKGFIISNATYKEIGLLTCEATVNGHLYKTNYLTHRQTNTII DVQISTPSPVELCVGETLVLNCTATTPANTRVQMTWSYPDEKNKN ASVRRRIDQSNSHANIFYSVLTIDGMTNSDKGLYTCRVRSGPSFKS VNTSVHIYDCSGGGGSGGGGSGGGGSAESKYGPPCPPCPAPEAAG GPSVFLFPPKPKDQLMISRTPEVTCVVVDVSQEDPEVQFNWYVDG VEVHNAKTKPREEQFNSTYRVVSVLTVLHQDWLNGKEYKCKVSN KGLPSSIEKTISKAKGQPREPQVYTLPPSQEEMTKNQVSLTCLVKGF YPSDIAVEWESNGQPENNYKTTPPVLDSDGSFFLYSRLTVDKSRW QEGNVFSCSVLHEALHNHYTQKSLSLSLG |
| SEQ ID NO: 33 | |
| H-32D12 (Ate-V3-G4H-G4Fc+QL) | EVQLVESGGGLVQPGGSLRLSCAASGFTFSDSWIHWVRQAPGKGL EWVAWISPYGGSTYYADSVKGRFTISADTSKNTAYLQMNSLRAED TAVYYCARRHWPGGFDYWGQGTLVTVSSGGGGSGGGGSGGGGS GGGGSDIQMTQSPSSLSASVGDRVTITCRASQDVSTAVAWYQQKP GKAPKLLIYSASFLYSGVPSRFSGSGSGTDFTLTISSLQPEDFATYYC QQYLYHPATFGQGTKVEIKRGSGGGGSGGGGSGGGGSGGGGSGR PFVEMYSEIPEIIHMTEGRELVIPCRVTSPNITVTLKKFPLDTLIPDGK RIIWDSRKGFIISNATYKEIGLLTCEATVNGHLYKTNYLTHRQTNTII DVQISTPSPVELCVGETLVLNCTATTPANTRVQMTWSYPDEKNKN ASVRRRIDQSNSHANIFYSVLTIDGMTNSDKGLYTCRVRSGPSFKS VNTSVHIYDCSGGGGSGGGGSGGGGSAESKYGPPCPPCPAPEAAG GPSVFLFPPKPKDQLMISRTPEVTCVVVDVSQEDPEVQFNWYVDG VEVHNAKTKPREEQFNSTYRVVSVLTVLHQDWLNGKEYKCKVSN KGLPSSIEKTISKAKGQPREPQVYTLPPSQEEMTKNQVSLTCLVKGF YPSDIAVEWESNGQPENNYKTTPPVLDSDGSFFLYSRLTVDKSRW QEGNVFSCSVLHEALHNHYTQKSLSLSLG |
| SEQ ID NO: 34 | |
| H-32D13 (Ate-V2-C65H-G4Fc+QL) | EVQLVESGGGLVQPGGSLRLSCAASGFTFSDSWIHWVRQAPGKGL EWVAWISPYGGSTYYADSVKGRFTISADTSKNTAYLQMNSLRAED TAVYYCARRHWPGGFDYWGQGTLVTVSSGGGGSGGGGSGGGGS GGGGSDIQMTQSPSSLSASVGDRVTITCRASQDVSTAVAWYQQKP GKAPKLLIYSASFLYSGVPSRFSGSGSGTDFTLTISSLQPEDFATYYC QQYLYHPATFGQGTKVEIKRGSAEPKAGGGGSGGGGSGGGGSGR PFVEMYSEIPEIIHMTEGRELVIPCRVTSPNITVTLKKFPLDTLIPDGK RIIWDSRKGFIISNATYKEIGLLTCEATVNGHLYKTNYLTHRQTNTII DVQISTPSPVELCVGETLVLNCTATTPANTRVQMTWSYPDEKNKN ASVRRRIDQSNSHANIFYSVLTIDGMTNSDKGLYTCRVRSGPSFKS VNTSVHIYDCSNTGSGGEEKKKEKEKEEQEERSSDTPPTCPPCPAPE AAGGPSVFLFPPKPKDQLMISRTPEVTCVVVDVSQEDPEVQFNWY VDGVEVHNAKTKPREEQFNSTYRVVSVLTVLHQDWLNGKEYKCK VSNKGLPSSIEKTISKAKGQPREPQVYTLPPSQEEMTKNQVSLTCLV KGFYPSDIAVEWESNGQPENNYKTTPPVLDSDGSFFLYSRLTVDKS RWQEGNVFSCSVLHEALHNHYTQKSLSLSLG |
| SEQ ID NO: 35 | |
| H-32D14 (Ate-V3-C65H-G4Fc+QL) | EVQLVESGGGLVQPGGSLRLSCAASGFTFSDSWIHWVRQAPGKGL EWVAWISPYGGSTYYADSVKGRFTISADTSKNTAYLQMNSLRAED TAVYYCARRHWPGGFDYWGQGTLVTVSSGGGGSGGGGSGGGGS GGGGSDIQMTQSPSSLSASVGDRVTITCRASQDVSTAVAWYQQKP GKAPKLLIYSASFLYSGVPSRFSGSGSGTDFTLTISSLQPEDFATYYC QQYLYHPATFGQGTKVEIKRGSGGGGSGGGGSGGGGSGGGGSGR PFVEMYSEIPEIIHMTEGRELVIPCRVTSPNITVTLKKFPLDTLIPDGK RIIWDSRKGFIISNATYKEIGLLTCEATVNGHLYKTNYLTHRQTNTII DVQISTPSPVELCVGETLVLNCTATTPANTRVQMTWSYPDEKNKN ASVRRRIDQSNSHANIFYSVLTIDGMTNSDKGLYTCRVRSGPSFKS VNTSVHIYDCSNTGSGGEEKKKEKEKEEQEERSSDTPPTCPPCPAPE AAGGPSVFLFPPKPKDQLMISRTPEVTCVVVDVSQEDPEVQFNWY VDGVEVHNAKTKPREEQFNSTYRVVSVLTVLHQDWLNGKEYKCK VSNKGLPSSIEKTISKAKGQPREPQVYTLPPSQEEMTKNQVSLTCLV KGFYPSDIAVEWESNGQPENNYKTTPPVLDSDGSFFLYSRLTVDKS RWQEGNVFSCSVLHEALHNHYTQKSLSLSLG |
| SEQ ID NO: 36 | |
| H-12C18 (A167-3(G4S)-Fc) | QVQLQESGPGLVKPSETLSITCTVSGFSLSNYDISWIRQPPGKGLEW LGVIWTGGATNYNPALKSRLTISRDNSKNQVSLKMSSVTAADTAV YYCVRDSNYRYDEPFTYWGQGTLVTVSSGGGGSGGGGSGGGGSG GGGSGGGGSEIVLTQSPDTLSVTPKEKVTLTCRASQSIGTNIHWFQ QKPGQSPKLLIKYASESISGVPSRFSGSGSGTDFTLTINSVEAEDAAT YYCQQSNSWPYTFGQGTKLEIKRTVGGGGSGGGGSGGGGSDKTH TCPPCPAPELLGGPSVFLFPPKPKDTLMISRTPEVTCVVVDVSHEDP EVKFNWYVDGVEVHNAKTKPREEQYNSTYRVVSVLTVLHQDWL NGKEYKCKVSNKALPAPIEKTISKAKGQPREPQVYTLPPSREEMTK NQVSLTCLVKGFYPSDIAVEWESNGQPENNYKTTPPVLDSDGSFFL YSKLTVDKSRWQQGNVFSCSVMHEALHNHYTQKSLSLSPGK |
| SEQ ID NO: 37 - scFv-Fc conjugate control but not full-length antibody | |
| H-30D01 (Ate-IgG1 Fc) | EVQLVESGGGLVQPGGSLRLSCAASGFTFSDSWIHWVRQAPGKGL EWVAWISPYGGSTYYADSVKGRFTISADTSKNTAYLQMNSLRAED TAVYYCARRHWPGGFDYWGQGTLVTVSSGGGGSGGGGSGGGGS GGGGSDIQMTQSPSSLSASVGDRVTITCRASQDVSTAVAWYQQKP GKAPKLLIYSASFLYSGVPSRFSGSGSGTDFTLTISSLQPEDFATYYC QQYLYHPATFGQGTKVEIKRCPPCPAPELLGGPSVFLFPPKPKDTL MISRTPEVTCVVVDVSHEDPEVKFNWYVDGVEVHNAKTKPREEQ YNSTYRVVSVLTVLHQDWLNGKEYKCKVSNKALPAPIEKTISKAK GQPREPQVYTLPPSRDELTKNQVSLTCLVKGFYPSDIAVEWESNGQ PENNYKTTPPVLDSDGSFFLYSKLTVDKSRWQQGNVFSCSVMHEA LHNHYTQKSLSLSPG |
| SEQ ID NO: 38 - scFv-Fc conjugate control but not full-length antibody | |

In the description of the configuration of Table 9, H and L represent the heavy chain and light chain of the scFv; Cnn (where n is an integer) corresponds to the sequence name in Table 2 above; G4S means a GGGGS linker; linkers connecting the scFv and the fusion protein are V1, V2, and V3, wherein V1 has the sequence GGGGSGGGGSDT, V2 has the sequence GSAEPKAGGGGSGGGGSGGGGS, and V3 has the sequence GSGGGGSGGGGSGGGGSGGGGS; C65H means that, among hinge portions of the fusion protein, the C65 hinge portion of Table 2 was used; G4H means that IgG4 was used instead of IgG1 among hinge portions of the fusion protein; G4Fc means an IgG4 sequence from which effector function has been removed; QL means that a variation was introduced to increase binding affinity to FcRn, which is known to increase the FcRn cycle and extend half-life; Cys means that a Cys variation was introduced in the fusion protein (Ser→Cys at position 494 for C65 and at position 493 for C75); and sur1 means a variant in which a back-variation (reverse variation) of partial surface charge variations was performed.

ExpiCHO^{™} cells (Gibco) used for production of the designed conjugates were used for transient expression at a passage number of 10 or more and 20 or less. Subculture of ExpiCHO^{™} cells was performed at intervals of 3 or 4 days; for 3-day culture, cells were subcultured at a density of 0.3 × 10⁶ cells/mL, and for 4-day culture, cells were subcultured at a density of 0.15 × 10⁶ cells/mL. Cell density and viability were measured using a Countess II (Invitrogen) instrument by trypan blue staining. Transient expression of the conjugates was performed using the Max Titer protocol culture method with Expifectamine^{™} (Gibco) provided by the manufacturer. One day prior to transfection, ExpiCHO^{™} cells were subcultured at a density of 3.5 × 10⁶ cells/mL. On the following day, cell density and viability were measured again to confirm that the cell density was at least 7.0 × 10⁶ cells/mL and the cell viability was at least 95%, and the cells were diluted by adding ExpiCHO^{™} expression medium such that the cell density became 6.0 × 10⁶ cells/mL. After dispensing 25 mL of the cell culture into a 125 mL Erlenmeyer flask, the cells were maintained in a 37°C incubator until preparation of the transfection mixture. Plasmid DNA corresponding to 20 µg was mixed well with 1 mL of OptiPro^{™} SFM medium, and 80 µL of Expifectamine^{™} CHO reagent was mixed well with 920 µL of OptiPro^{™} SFM medium. The Expifectamine-OptiPro^{™} SFM mixture was added to the pre-prepared plasmid-OptiPro^{™} SFM and mixed well, followed by incubation at room temperature for 3 minutes. The mixture was slowly added to the pre-dispensed cell culture and cultured in an incubator set to 37°C, 8% CO₂, and 130 rpm. At 18-22 hours after transfection, 150 µL of Expifectamine^{™} CHO Enhancer and 4 mL of ExpiCHO^{™} Feed were added to the flask, and cell culture was continued in an incubator set to 32°C, 5% CO₂, and 130 rpm. To produce a larger amount of conjugate, 200 mL of the cell culture was dispensed into a 1 L Erlenmeyer flask and the cells were maintained in a 37°C incubator until preparation of the transfection mixture. Plasmid DNA corresponding to 160 µg was mixed well with 8 mL of OptiPro^{™} SFM medium, and 640 µL of Expifectamine^{™} CHO reagent was mixed well with 7.4 mL of OptiPro^{™} SFM medium. The Expifectamine-OptiPro^{™} SFM mixture was added to the pre-prepared plasmid-OptiPro^{™} SFM and mixed well, followed by incubation at room temperature for 3 minutes. The mixture was slowly added to the pre-dispensed cell culture and cultured in an incubator set to 37°C, 8% CO₂, and 130 rpm. At 18-22 hours after transfection, 1200 µL of Expifectamine^{™} CHO Enhancer and 32 mL of ExpiCHO^{™} Feed were added to the flask, and cell culture was continued in an incubator set to 32°C, 5% CO₂, and 130 rpm. Cell density and viability were measured on days 2 and 5 after transfection. On day 5, after measuring the final titer using a Cedex (Roche) instrument, the culture was centrifuged at 3000 × g for 30 minutes to recover the cell culture supernatant containing the protein. The recovered supernatant was filtered through a 0.22 µm polyethersulfone (PES) filter and stored at 4°C for short-term storage or at -80°C for long-term storage.

### EXAMPLE 4 - Purification of Conjugate Proteins (Protein A Affinity Chromatography and Size-Exclusion Chromatography)

Protein purification from the culture supernatant was performed by affinity chromatography using an AKTA avant25 instrument equipped with an Amsphere a3 column. The culture supernatant filtered through a 0.22 µm bottle-top filter was loaded at a flow rate of 13.33 mL/min. To remove nonspecific binding, buffers were applied sequentially in an amount of 8 column volumes each: first, a buffer of 50 mM Na-Pi, 0.5 M NaCl, pH 7.0; and second, a buffer of 50 mM sodium acetate, pH 4.5. After removal of nonspecific binding, bound proteins were eluted by applying an elution buffer of 100 mM glycine, pH 3.0, in an amount of 10 column volumes, and the eluate was collected in a 50 mL tube. The collected protein solution was neutralized by adding 1 M Trizma^{®} base (pH 11.0) in an amount corresponding to 1% of the collected protein volume, and precipitates were removed using a 0.22 µm syringe filter. Samples before and after neutralization were quantified by a UV method, and purity was analyzed by SEC-HPLC. The primarily purified material was further subjected to size-exclusion chromatography using a Superdex200pg 26/600 column in order to achieve a purity of 90% or more. First, the neutralized sample was concentrated to a volume of 13 mL or less using a Millipore^{™} stirred cell 250 mL concentrator and a Millipore^{™} disc 50 kDa. The concentrated sample was loaded onto a column equilibrated with PBS, pH 6.5, and eluted proteins were collected in tubes. After confirming purity by SEC-HPLC, the sample was further concentrated. The final concentrated protein was analyzed by UV quantification, and purity was confirmed by SEC-HPLC and SDS-PAGE analysis.

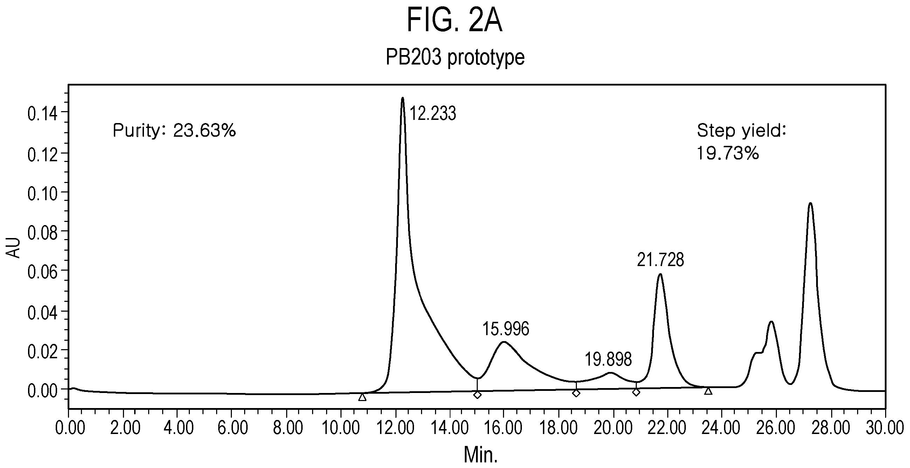

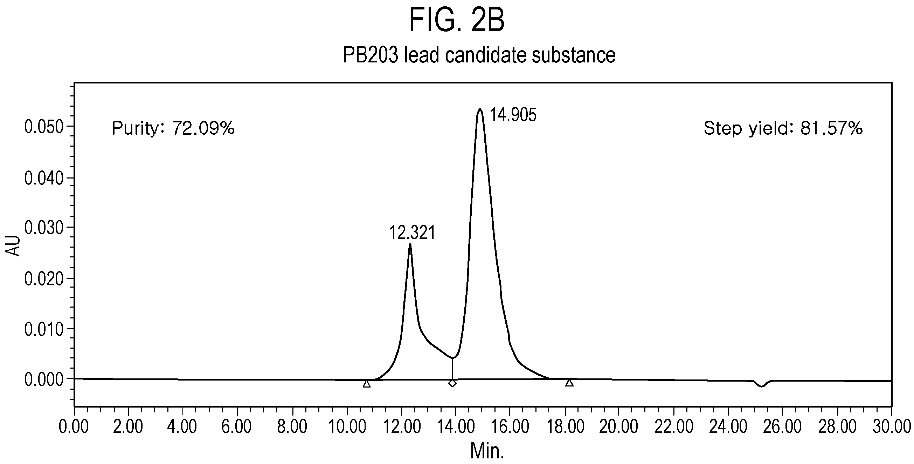

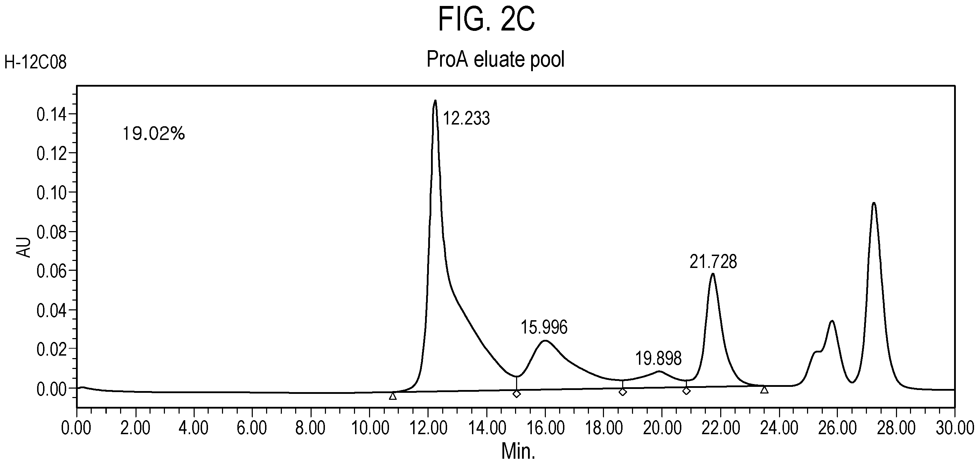

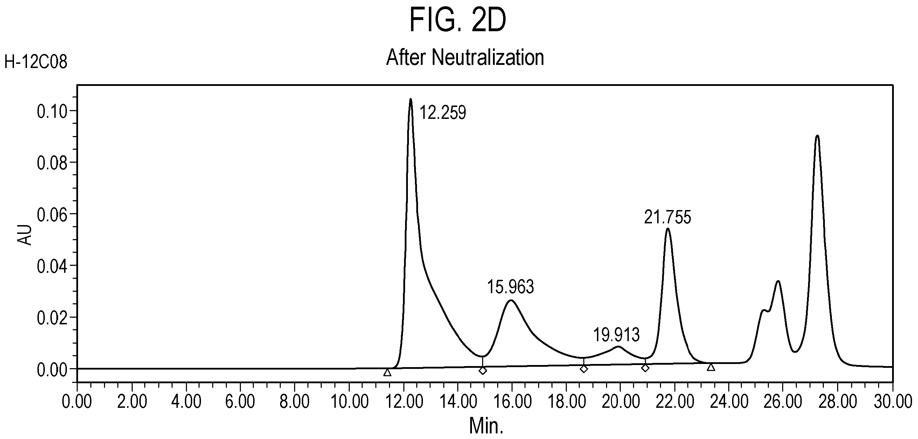

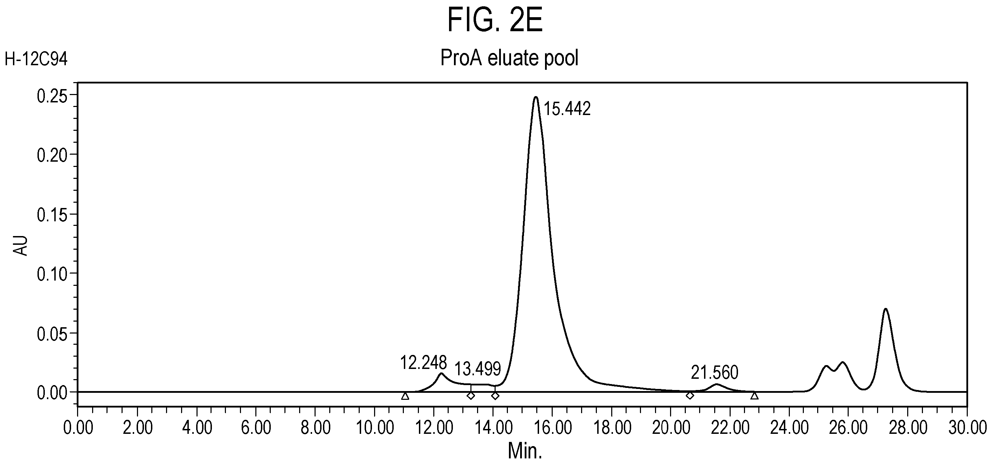

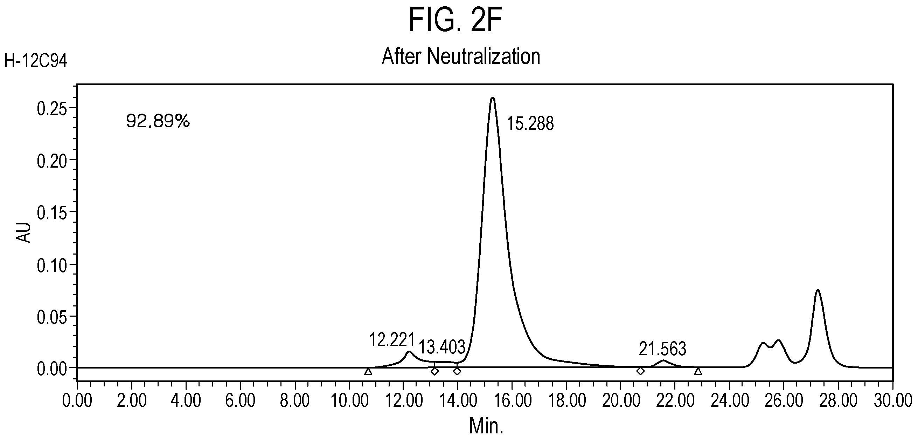

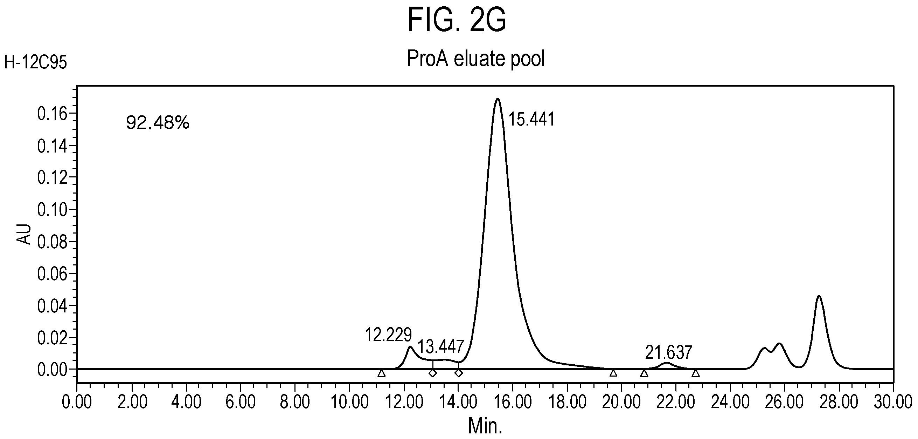

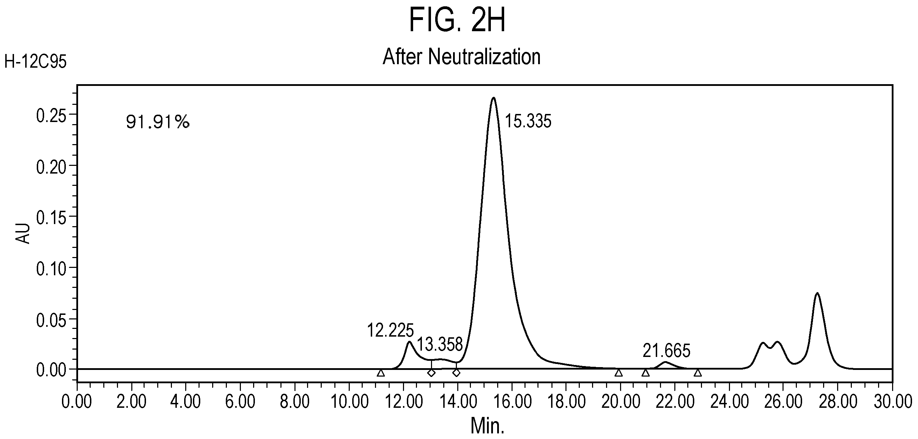

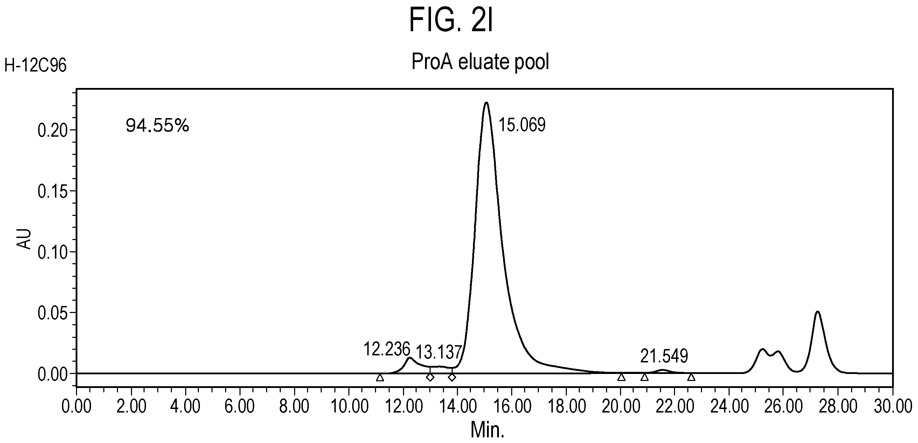

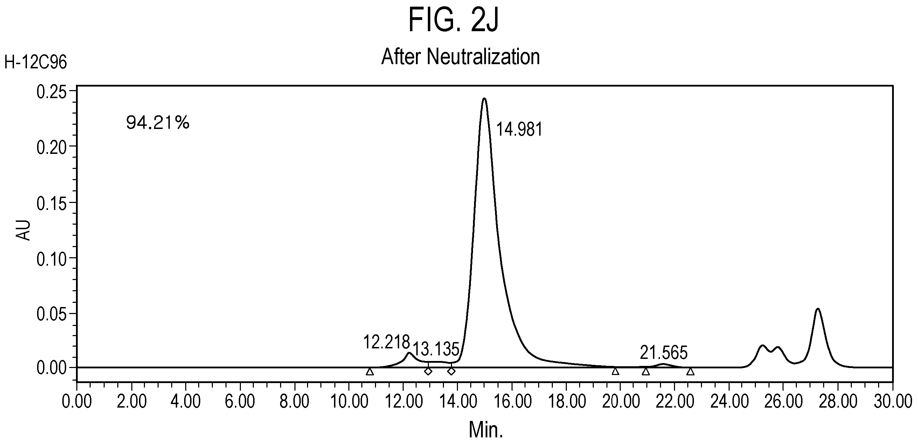

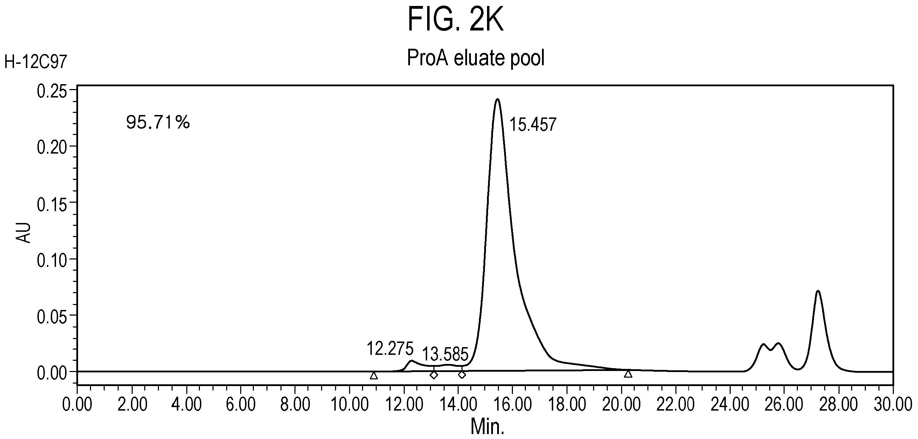

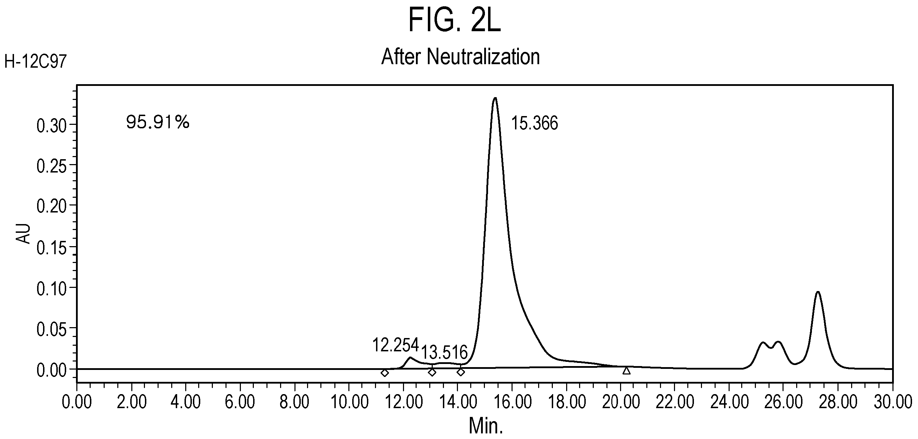

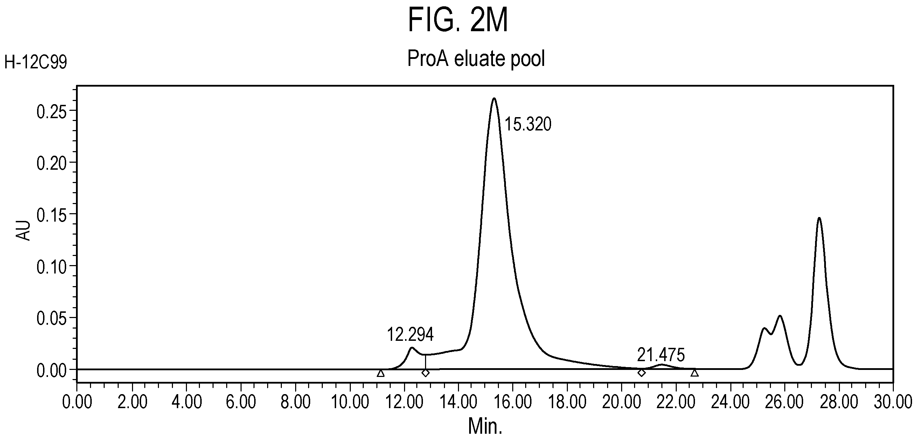

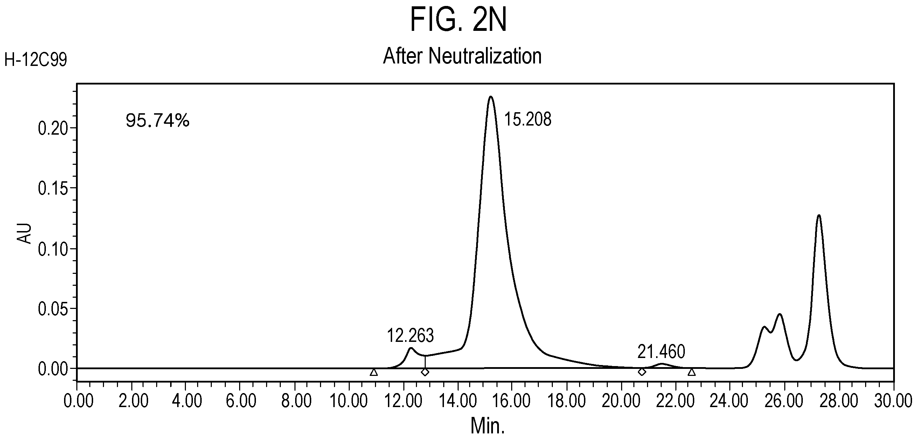

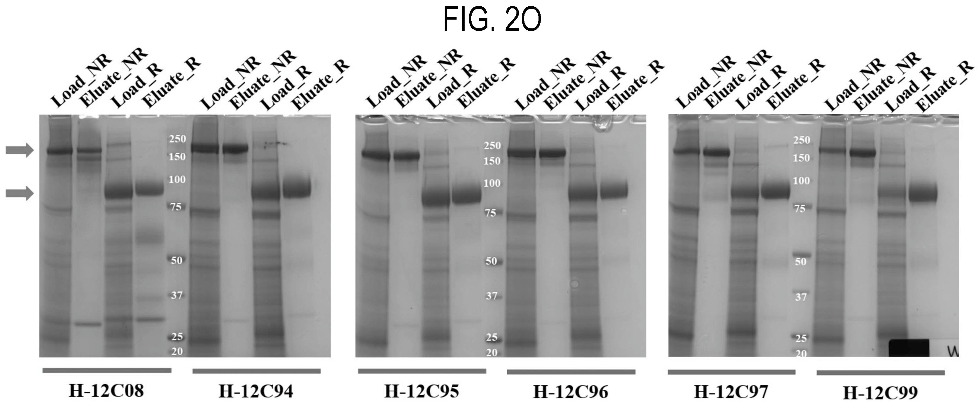

Through the above process, one or more of the following physicochemical parameters were measured for each conjugate: titer, elution amount, step yield, content of high molecular weight species (HMWS), content of low molecular weight species (LMWS), and functional monomer content. Here, "titer" refers to the concentration of the Fc-fusion protein present in the culture supernatant measured upon harvest of the cell culture; "elution amount" refers to the total amount of protein recovered after the primary purification step (affinity chromatography); and "yield" refers to a value obtained by dividing the total amount of protein recovered after the primary purification by the total amount of protein measured in the culture supernatant, and indicates the recovery rate of the protein recovered by the column during the primary purification. HMWS refers to protein species having a mass greater than the predicted functional unit of the protein, and LMWS refers to protein species having a mass smaller than the predicted functional unit of the protein; in both cases, they refer to fractions having abnormal protein folding. A functional monomer refers to a fraction having normal protein structure and function. FIGs. 2a to 2o show the productivity and purity of conjugates of anti-PD-L1 scFv and the modified fusion protein (modified VEGFR1-D2-D3). That is, it was confirmed that, compared to a control conjugate (H-12C08; A167 scFv-HG4SL-VEGF-Grab) prepared using an unmodified fusion protein, the conjugates H-12C94 to H-12C99, which are conjugates based on the modified fusion protein of the present disclosure, exhibited increased purity when purified under the same conditions (purity of about 20% vs. about 90%). It was confirmed that the conjugate prepared using the unmodified fusion protein generated a large amount of high-molecular-weight aggregates (FIGs. 2c and 2d), whereas in the conjugates prepared using the modified fusion protein, such aggregates were significantly reduced (FIGs. 2e to 2n). This indicates that the physicochemical properties of the conjugates of the present disclosure were improved by including the modified fusion protein, which in turn indicates an improved yield in drug manufacturing.

### EXAMPLE 5 - Freeze/Thaw Stability Test of Conjugates

Freeze/thaw stability of the produced conjugates was evaluated. To confirm stability upon freeze/thaw, the conjugate protein sample was frozen by standing in an ultralow-temperature freezer at -70°C for 1 hour, and then thawed by standing at room temperature for 30 minutes. This freeze/thaw procedure was repeated five times. At each cycle, 50 µL of the protein sample was collected and analyzed by SEC-HPLC to confirm changes in purity (Table 10: 2^{nd} run (2R); Table 11: 3^{rd} run (3R)).

**[TABLE 10]**

| Protein code | Control | 1^{st} F/T | 2^{nd} F/T | 3^{rd} F/T | 4^{th} F/T | 5^{th} F/T |
|---|---|---|---|---|---|---|
| H-32D01 | 92.01 | 86.54 | 83.92 | 83.65 | 83.11 | 83.48 |
| H-32D02 | 94.78 | 89.64 | 88.36 | 87.87 | 87.59 | 87.56 |
| H-32D09 | 96.17 | 96.46 | 95.09 | 94.52 | 94.22 | 93.81 |
| H-32D10 | 96.29 | 95.05 | 94.01 | 93.62 | 93.4 | 92.94 |

**[TABLE 11]**

| Protein code | Control | 1^{st} F/T | 2^{nd} F/T | 3^{rd} F/T | 4^{th} F/T | 5^{th} F/T |
|---|---|---|---|---|---|---|
| H-32D02 | 94.03 | 93.73 | 93.24 | 93.06 | 92.96 | 92.84 |
| H-32D04 | 93.57 | 92.78 | 92.28 | 92 | 91.81 | 91.79 |
| H-32D06 | 93.24 | 92.6 | 92.45 | 92.18 | 92.04 | 91.98 |
| H-32D08 | 95.89 | 94.9 | 94.5 | 94.05 | 93.91 | 93.82 |

Table 10 shows the freeze/thaw stability results obtained in the 2^{nd} run experiment (2R), and Table 11 shows the freeze/thaw stability results obtained in the 3^{rd} run experiment (3R). As shown in Table 10, in terms of freeze/thaw stability of the conjugates, there was no large difference depending on linker V2 or V3, although V3 showed slightly higher stability. As for the scFv, atezolizumab showed relatively higher stability than A167; however, as with the linkers, it is difficult to regard this as a meaningful difference. Overall, high freeze/thaw stability was observed for the conjugates based on the modified fusion protein of the present disclosure, regardless of the linker and scFv.

This was also observed in the comparative results of Fc variants employing the A167 scFv and the V3 linker. As shown in Table 11, for all variants, the purity decrease was confirmed to be within 2% even after five freeze/thaw cycles.

The above results were obtained in a general buffer rather than an optimized formulation, indicating the possibility that freeze/thaw stability may be further improved through future formulation studies.

Overall, the conjugates based on the modified fusion protein of the present disclosure exhibited high freeze/thaw stability.

### EXAMPLE 6 - Purity Analysis of Conjugate Proteins Using Size-Exclusion High-Performance Liquid Chromatography

Purity of the conjugate proteins was analyzed by connecting a Tosoh TSKgel G3000SWXL SEC column to a Waters Arc HPLC system, with PBS (pH 7.4) serving as a mobile phase. While flowing the mobile phase at 0.5 mL/min, 30 µg of a protein sample was injected into the column and detected at 280 nm for 30 minutes to measure one or more of the following: content of high molecular weight species (HMWS), content of low molecular weight species (LMWS), and functional monomer content.

### EXAMPLE 7 - Evaluation of Binding Affinity to VEGF-A, PlGF, and PD-L1

Binding affinities of each protein to VEGF-A and PlGF ligands were measured using an Octet^{®} RED96e (ForteBIO) instrument according to the reference supra (Kamat et al., 2017).

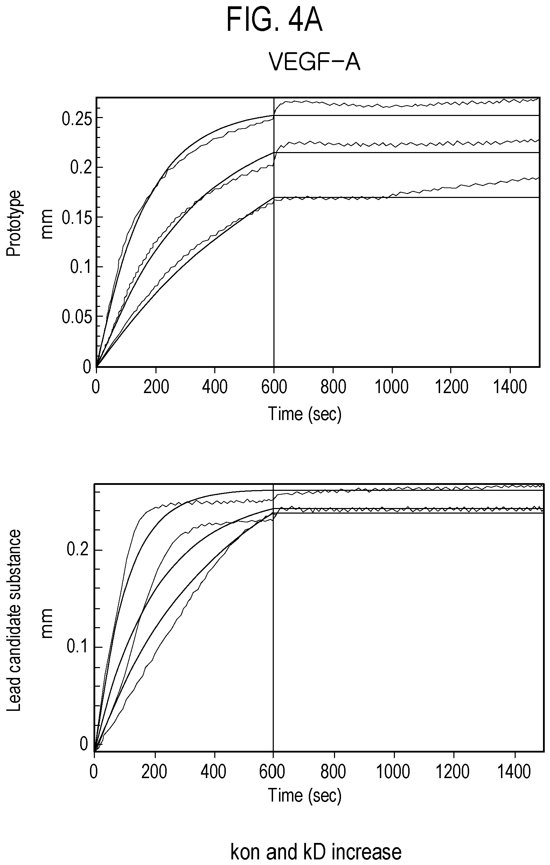

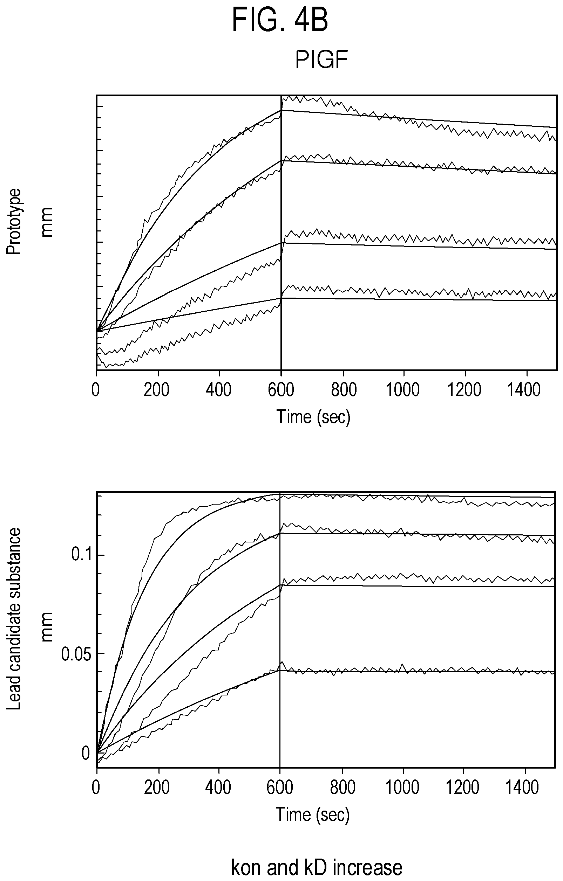

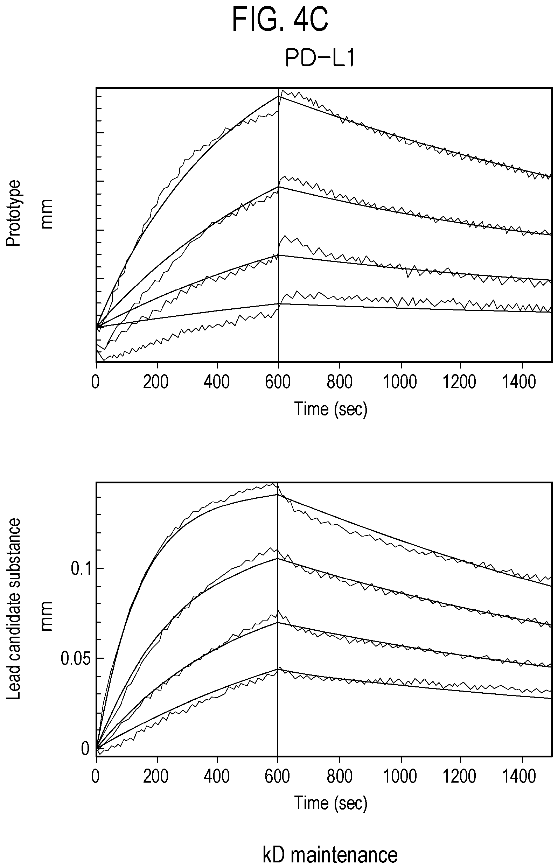

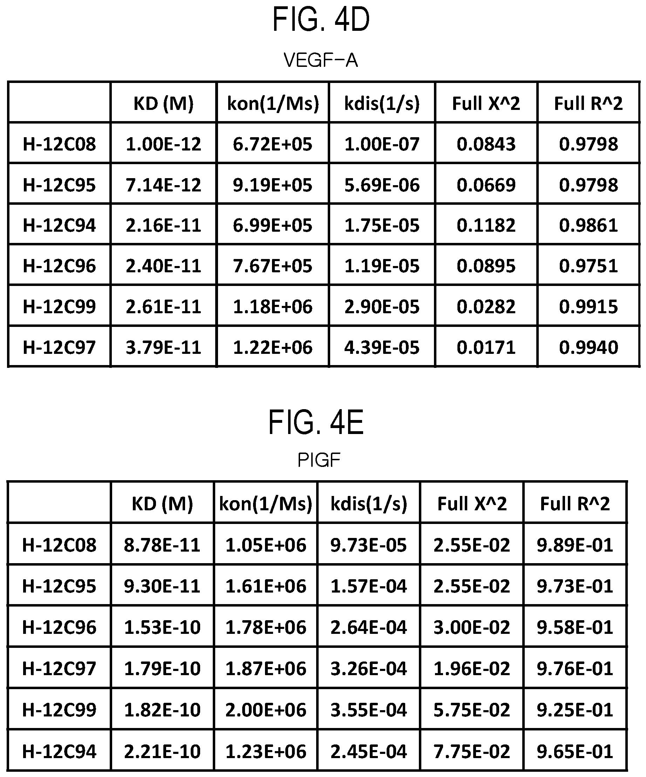

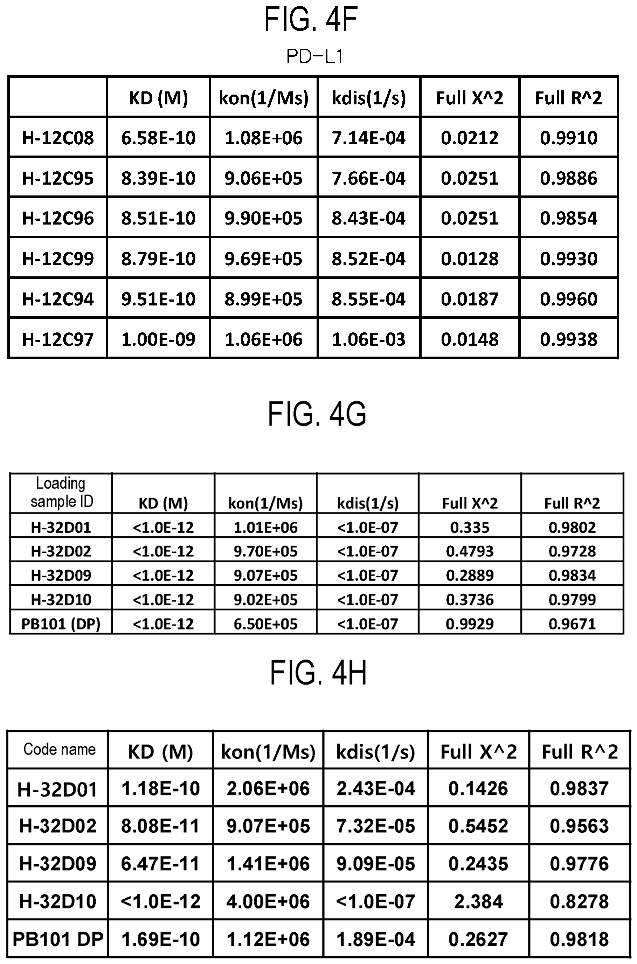

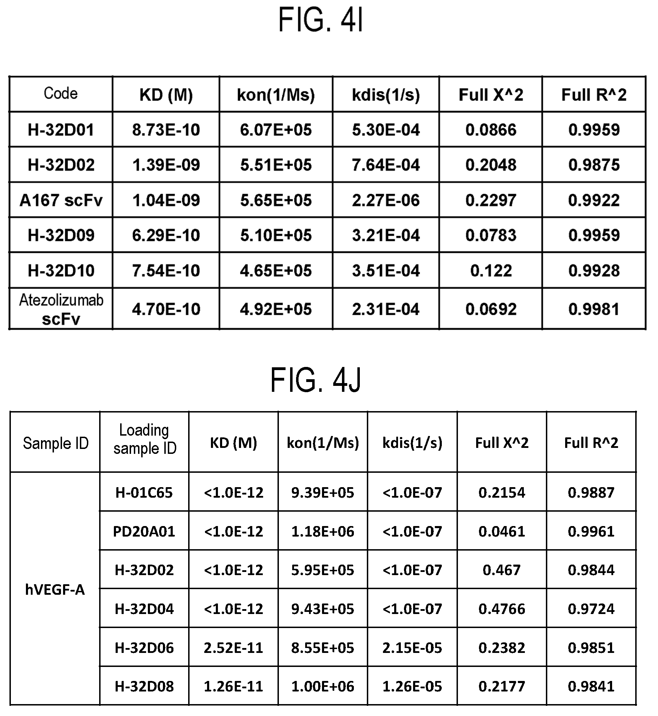

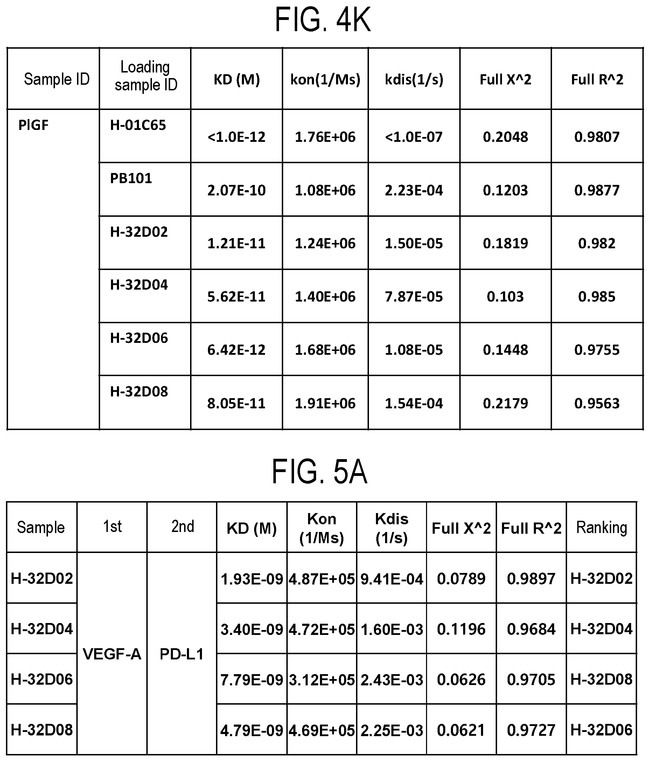

First, prior to binding measurement, an AHC sensor was equilibrated by immersion in 1× KB buffer (ForteBIO). After binding the conjugate protein PB203, which is the analyte, to the sensor at a concentration of 10 nM, the sensor was washed with 1× KB buffer to remove residual protein after binding. Thereafter, VEGF-A, PlGF, and PD-L1 were each flowed over the sensor at eight concentration points to measure binding. From the binding results, at least three concentrations satisfying Full X² (≤3) and Full R² (≥0.95), which are indicators for ensuring reliability, were selected and set for global fitting, and binding affinity (KD) as well as Kon and Kdis values were calculated. The results are shown in FIGs. 4a to 4k. As can be seen in FIGs. 4a to 4k, it was confirmed that the conjugates of the present disclosure exhibited an increased association rate to VEGF-A and PlGF compared with conjugates prepared using an unmodified fusion protein, and that the PD-L1 binding of the scFv was well maintained without reduction.

However, when preparing a conjugate of the modified fusion protein of the present disclosure and an scFv targeting a specific antigen, it cannot be predicted in advance whether the binding of the fusion protein to VEGF and/or PlGF and the binding of the scFv to the specific antigen will function without interfering with each other. In the present disclosure, it was confirmed that the binding of the modified fusion protein to VEGF and PlGF and the binding of the anti-PD-L1 scFv to PD-L1 were maintained without antagonism, which is an effect that is not readily predictable.

### EXAMPLE 8 - Stability Evaluation in Blood-Mimicking Environment

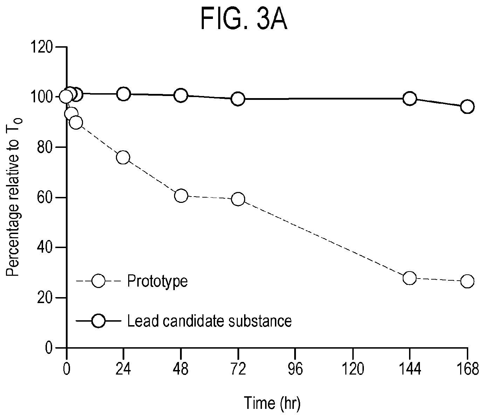

To indirectly analyze in vivo stability of PB203, the concentration of residual protein variants after a certain time was measured by an ELISA method through reaction with pooled serum collected from rats. PB203 was mixed with rat pooled serum to a final concentration of 10 µg/mL and stored in an incubator at 37°C at 168 hours, 144 hours, 72 hours, 48 hours, 24 hours, 4 hours, 2 hours, and 0 hours before the test initiation time, respectively.. The day before the assay, plates were coated with human VEGF-A (R&D Systems) by overnight incubation at 4°C. The plates were washed with a 0.1% PBS-T washing buffer to remove insufficiently bound human VEGF-A, and then treated with 0.1% PBS-T containing 5% skim milk to perform blocking at room temperature for 1 hour. The protein-serum mixtures prepared for each time point were diluted 50-fold in blocking buffer, and 100 µL was added to each well and reacted at room temperature for 2 hours. Thereafter, the reaction solution in the wells was removed and the plates were washed with wash buffer. A detection antibody, HRP Goat anti-human IgG Fc Cross-Absorbed Secondary antibody (Invitrogen), was reacted at room temperature for 1 hour, followed by washing to remove unreacted detection antibody. Then, 100 µL of 3,3',5,5'-tetramethylbenzidine (TMB) solution (Sigma) was added and reacted at room temperature for 10 minutes, and the reaction was stopped by adding stop solution (Sigma). Absorbance was measured at 450 nm using a plate reader. The remaining amount (%) was calculated relative to the absorbance at 0 hours and compared. The results are shown in FIGs. 3a to 3c.

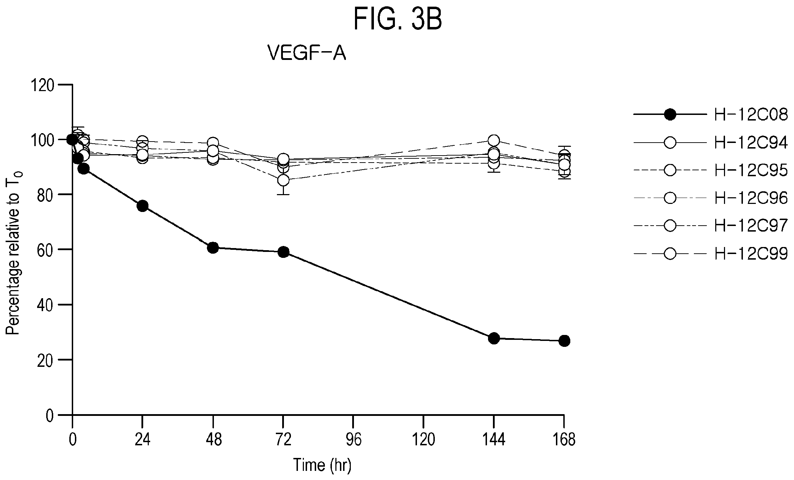

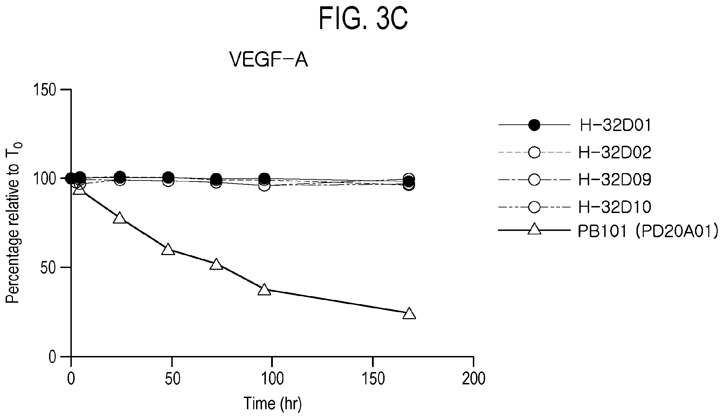

As can be seen in FIGs. 3a and 3b, it can be confirmed that H-12C94 to H-12C99, which are conjugates based on the modified fusion protein of the present disclosure, exhibit markedly superior serum stability in a blood-mimicking environment compared with a conjugate (H-12C08) prepared using an unmodified fusion protein. In addition, as can be seen in H-32D01, H-32D02, H-32D09, and H-32D10, it was confirmed that markedly superior serum stability was exhibited in the blood-mimicking environment regardless of use of the V2 linker or the V3 linker and regardless of the type of scFv (FIG. 3c).

### EXAMPLE 9 - Isoelectric Point Analysis

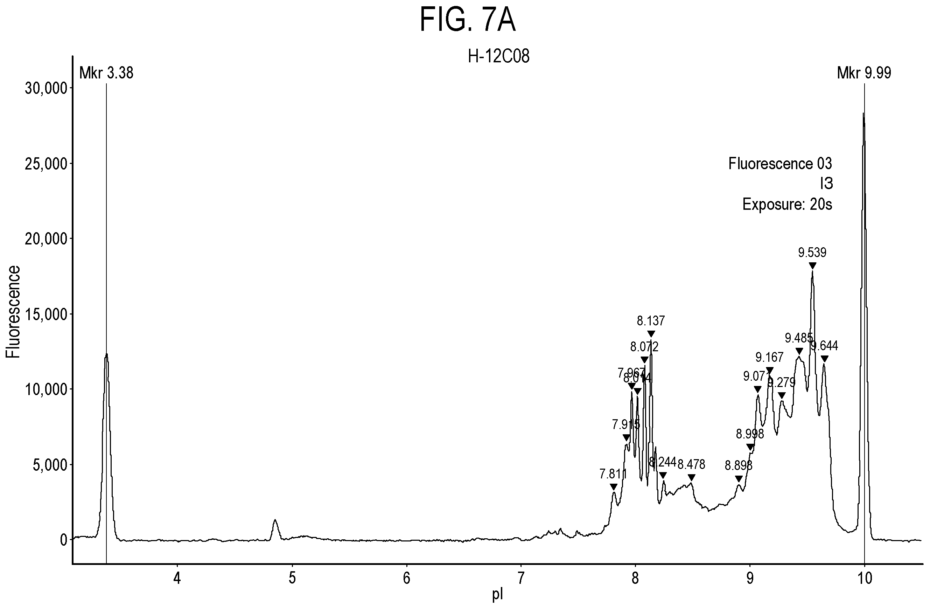

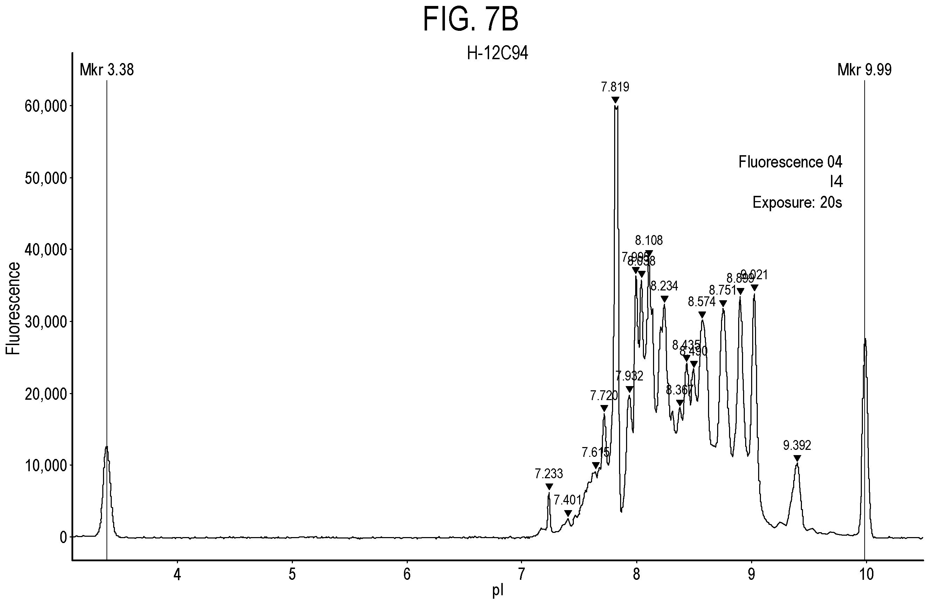

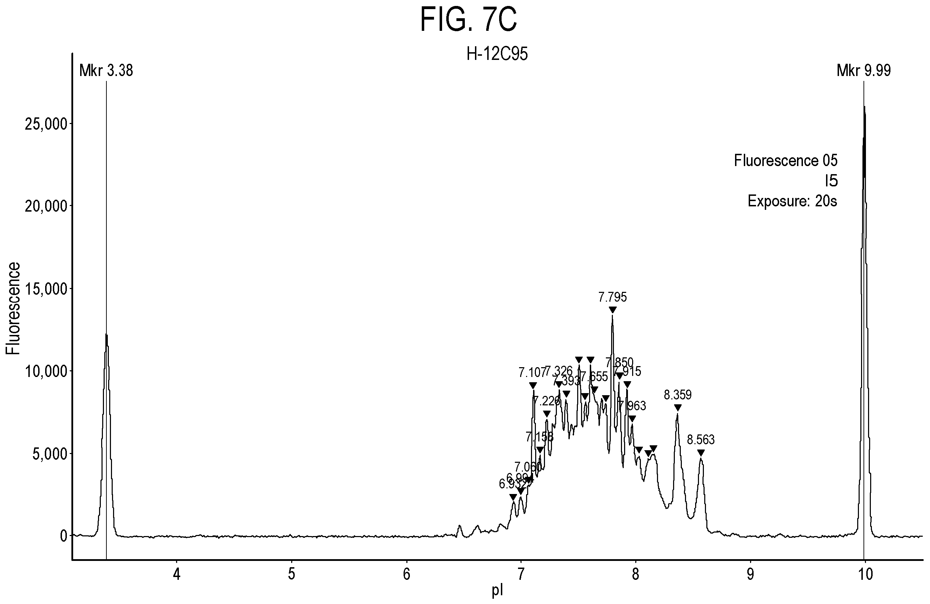

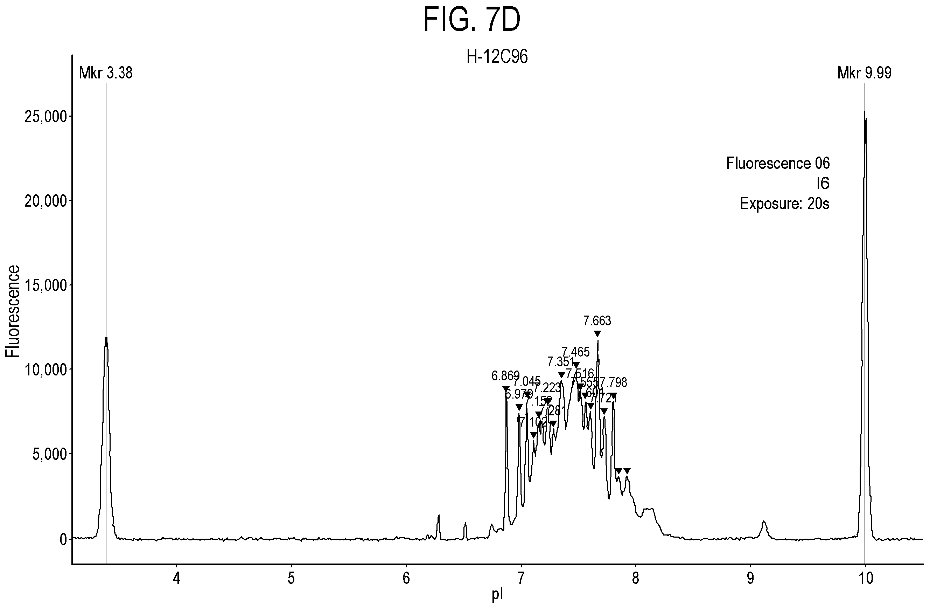

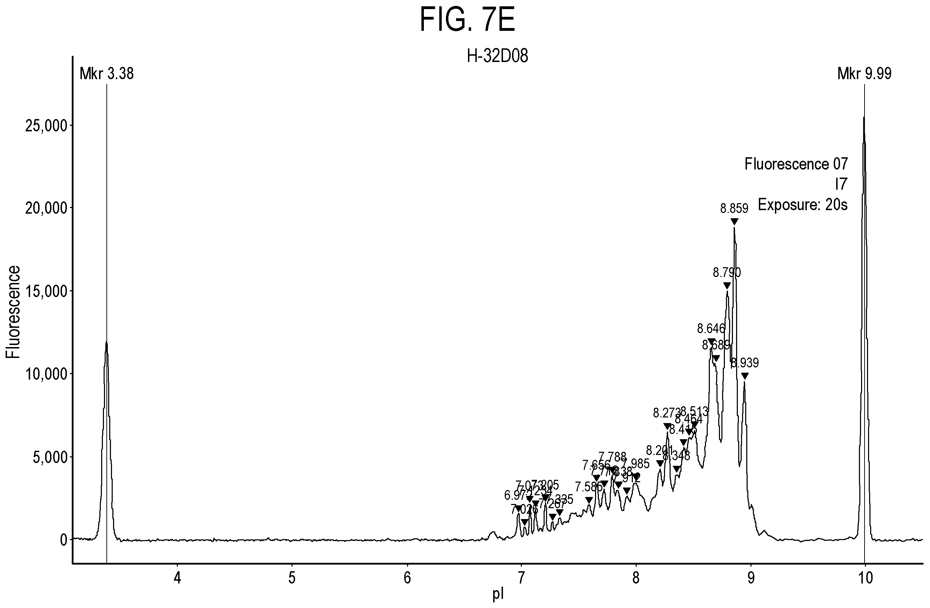

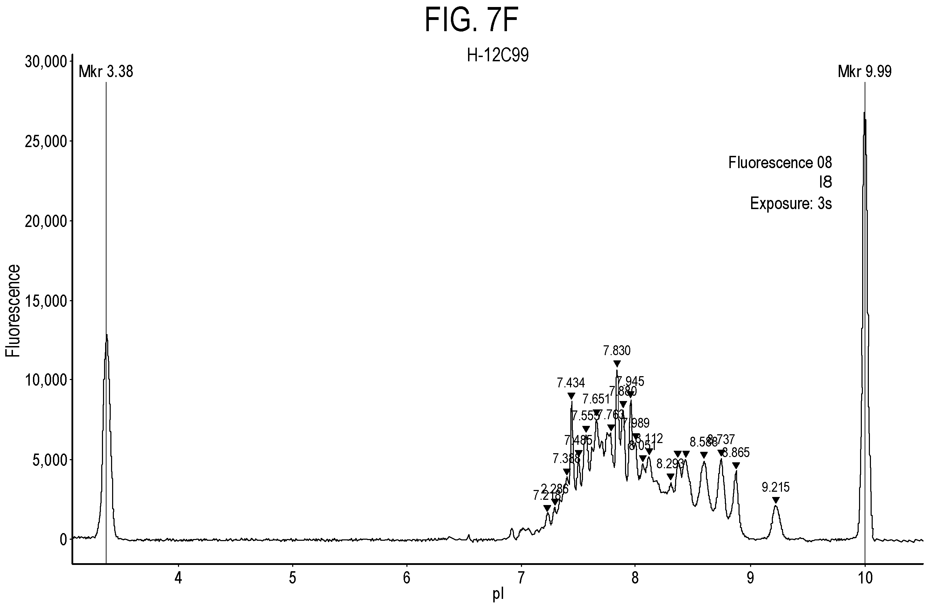

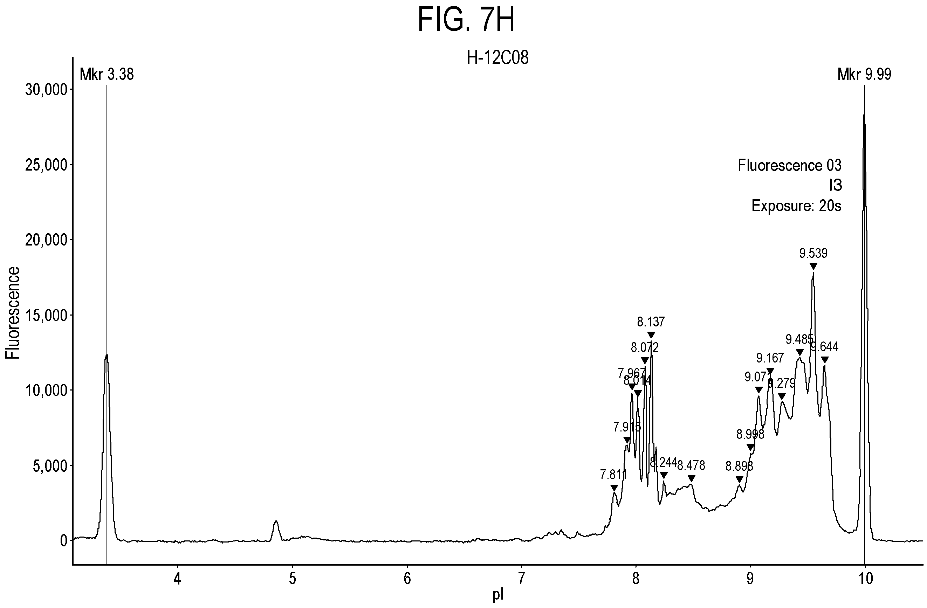

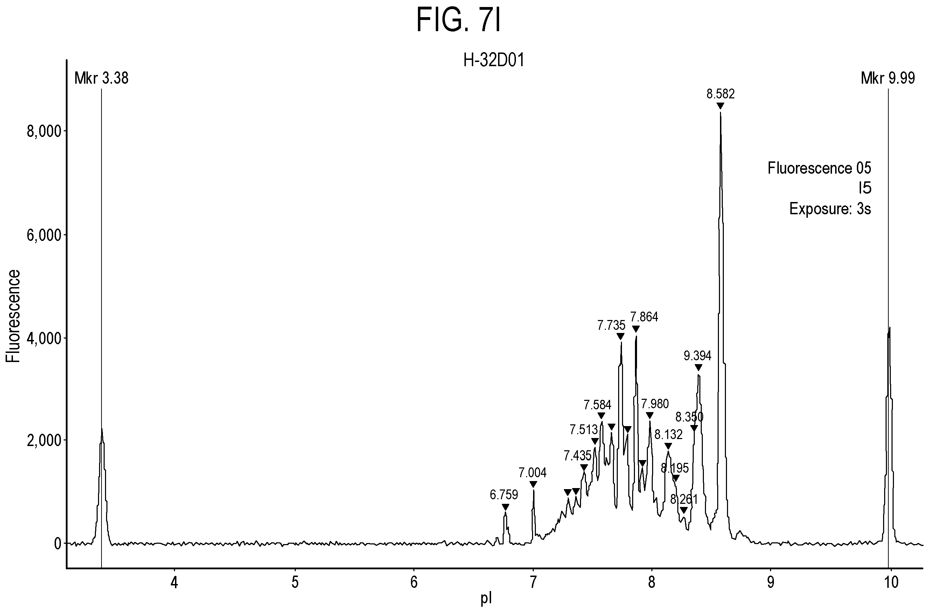

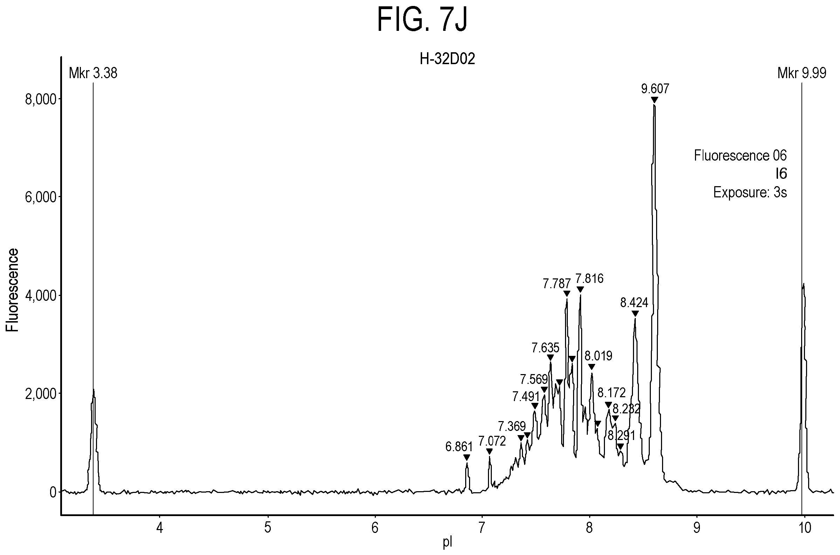

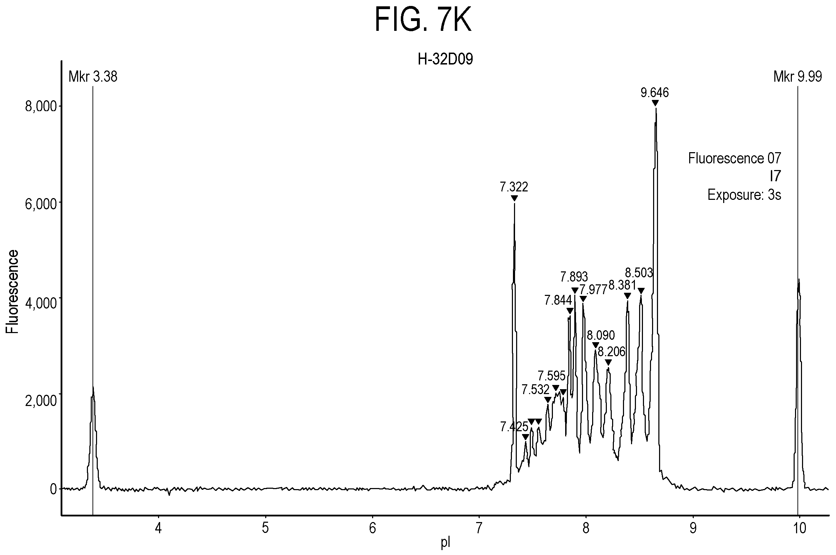

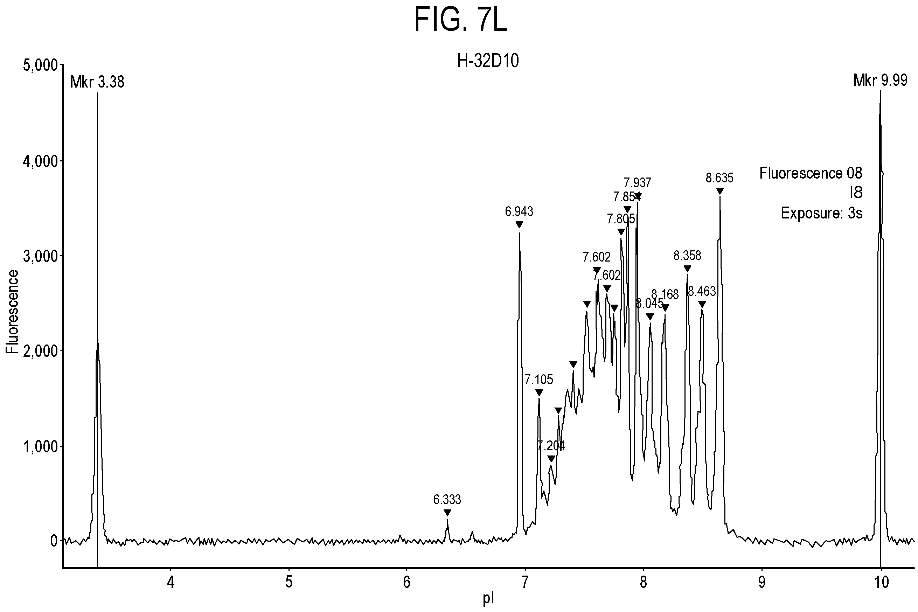

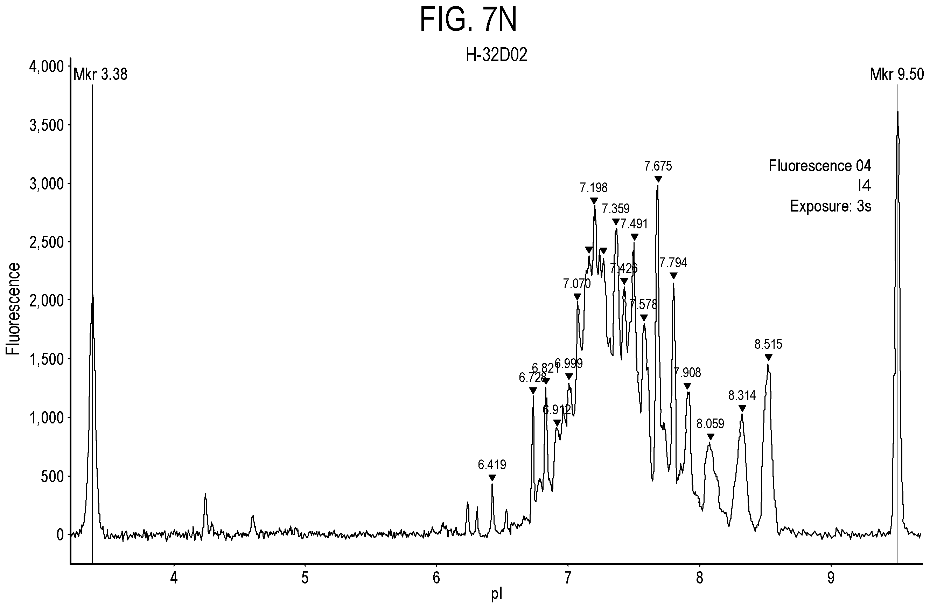

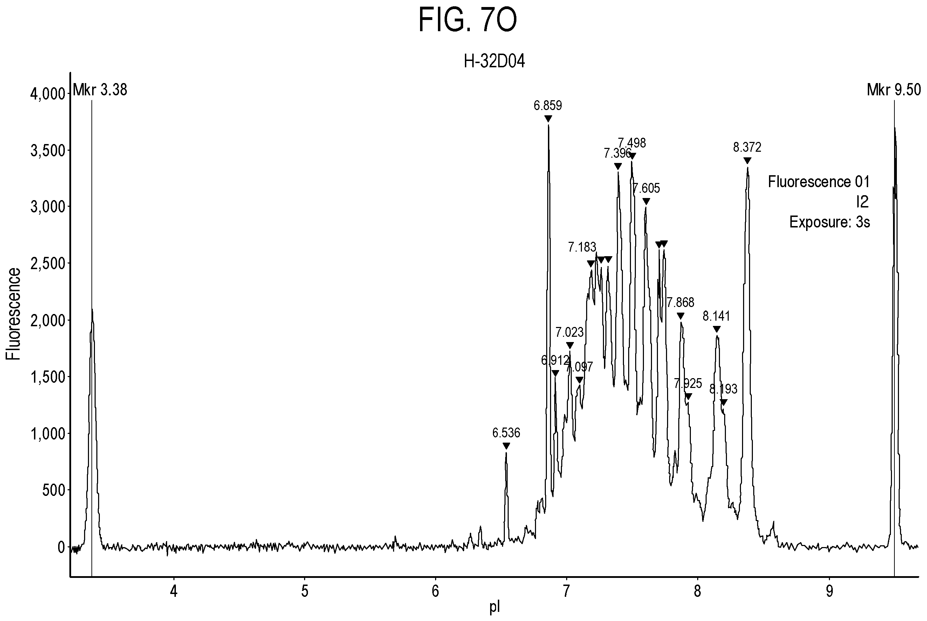

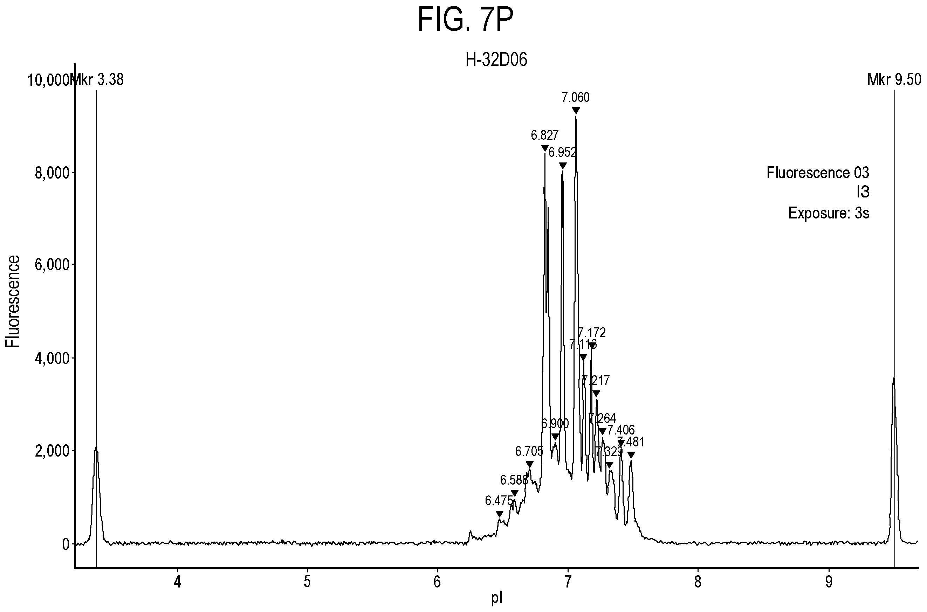

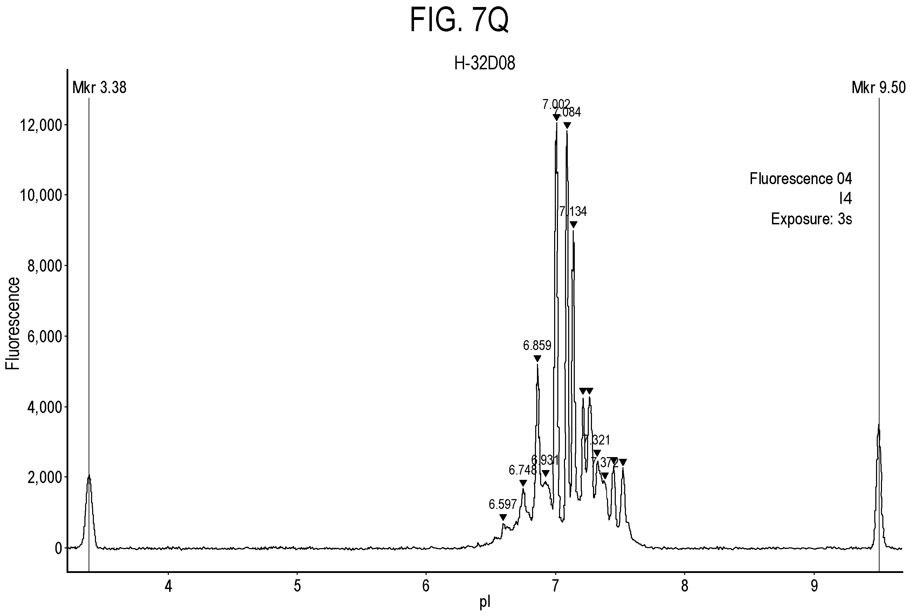

The isoelectric point (pI) of the PB203 protein was analyzed using an icIEF (image capillary isoelectric focusing) system. Forty microliters of the modified fusion protein diluted to a concentration of 2 mg/mL was mixed with 160 µL of a pre-prepared master mix solution (SERVALYT^{™} + low pI marker (3.38) (ProteinSimple) + high pI marker (9.5) (ProteinSimple) + 1% methylcellulose (ProteinSimple) + DW + 500 mM arginine (Sigma) + 200 mM iminodiacetic acid (Sigma) + 10 M urea (Sigma)). After centrifugation to remove precipitates and bubbles, the mixture was loaded into each well of the instrument (Maurice, ProteinSimple). Two milliliters each of catholyte solution and anolyte solution were placed in the OH⁻ and H⁺ positions of the cartridge, respectively, and the cartridge was mounted on the instrument. Thereafter, the pI was measured according to the instrument instructions. Since the measured pI appeared as multiple peaks over a specific range rather than a single peak, the pI distribution of the protein was defined by dividing it into three regions: acidic (pI 6-7), neutral (pI 7-8), and basic (pI >8), and the region showing the highest proportion was compared. The results are shown in FIGs. 7a to 7r.

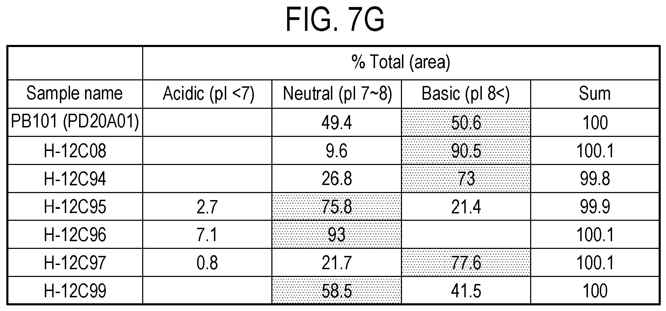

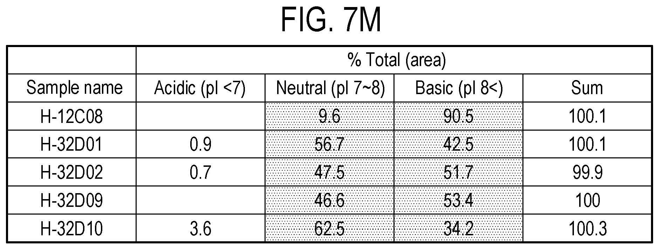

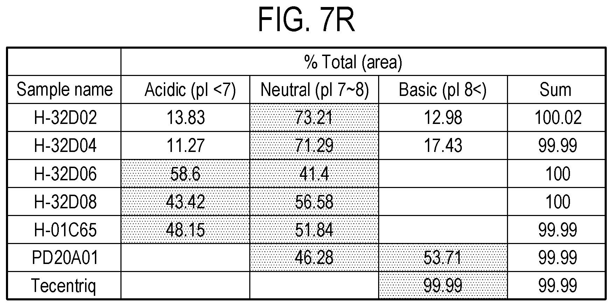

As can be seen in FIGs. 7g, 7m, and 7r, it was confirmed that the conjugates based on the modified fusion protein of the present disclosure, H-12C94 to H-12C99, exhibited an increased acidic/neutral ratio compared with the control conjugate prepared using an unmodified fusion protein (H-12C08; A167 + VEGF-Grab). In addition, as shown in FIGs. 7m and 7r, it was confirmed that the acidic/neutral ratio increased regardless of whether linker V2 or V3 was used, regardless of the type of scFv, and regardless of the presence or absence of Fc variations, compared with unmodified fusion proteins and conjugates prepared therefrom. This indicates that the conjugates of the present disclosure tend to carry a more negative charge in an in vivo environment, suggesting a reduced likelihood of nonspecific binding during systemic circulation.

### EXAMPLE 10 - Thermal Stability Analysis

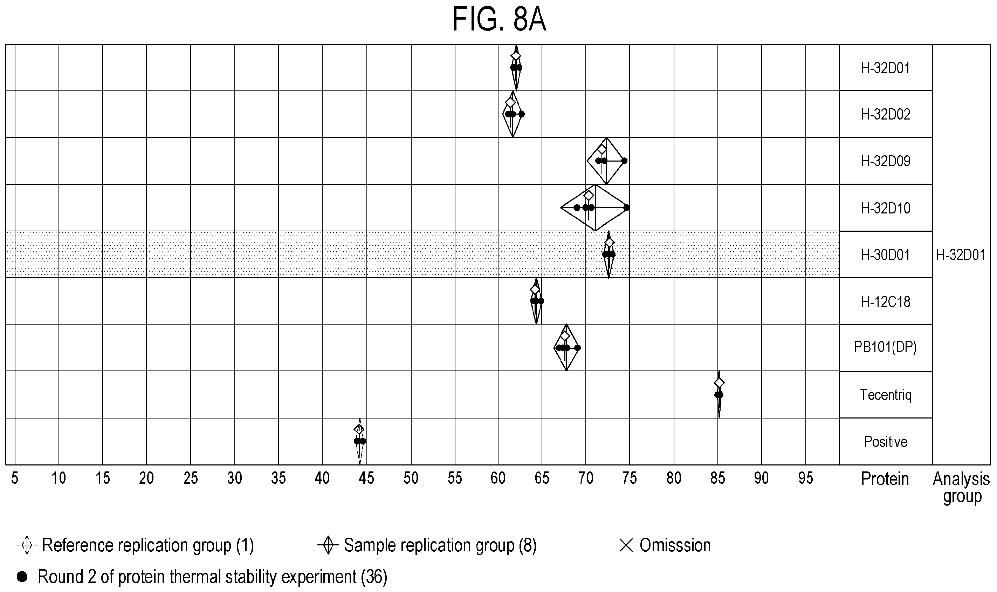

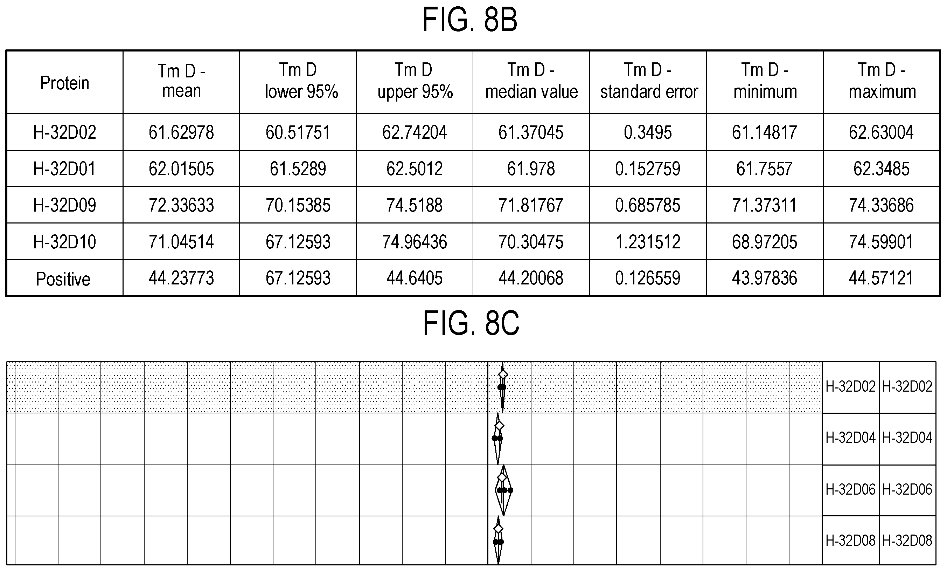

PB203 was prepared by dilution to a final concentration of 2 mg/mL in 1× PBS buffer. To 12.5 µL of the protein, 2.5 µL of 8× Protein Thermal Shift^{™} dye (Life Technologies) and 5.0 µL of Protein Thermal Shift^{™} buffer (Life Technologies) were added to prepare a final reaction mixture. The prepared 20 µL reaction mixture was loaded into PCR tubes, arranged on the tray of a QuantStudio^{™} real-time PCR instrument, and the melting temperature (Tm) was measured. All analyses were performed in four independent replicates. The results are shown in FIGs. 8a to 8d.

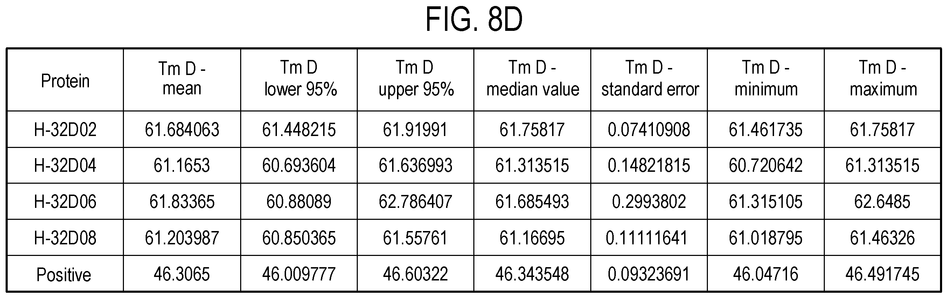

Specifically, FIG. 8b shows that the effect of linkers V2 and V3 on thermal stability was not significant, whereas thermal stability varied depending on the scFv used. In addition, FIG. 8d confirms that Fc variations were not correlated with thermal stability.

Since the temperature upon in vivo administration is within 40°C, protein therapeutics are generally evaluated for storage stability at 25°C and 40°C, assuming room temperature and physiological temperature. Under these criteria, the conjugates based on the modified fusion protein of the present disclosure were confirmed to exhibit overall high thermal stability of 60°C or higher.

This demonstrates that the conjugates based on the modified fusion protein of the present disclosure possess physicochemical properties suitable for development as pharmaceutical agents.

### EXAMPLE 11 - Analysis of VEGF-A Inhibitory Activity

To quantitatively analyze inhibitory activity against VEGF-A, a genetically engineered cell line (KDR/NFAT-RE HEK293, Promega) incorporating a luciferase reporter system based on VEGFR-2 (KDR) expression and VEGF-A/VEGFR-2 interaction was used. After dispensing 25 µL of cells at a density of 1.6 × 10⁶ cells/mL into a 96-well plate, 25 µL of a VEGF-A solution (33.3 ng/mL) and 25 µL of PB203 diluted to various concentrations were added and incubated for 6 hours at 37°C in a 5% CO₂ incubator. The plate was then cooled at room temperature for 15 minutes, after which 75 µL of Bio-Glo reagent (Promega; luciferase assay buffer + substrate mixture) was added. After incubation for 10 minutes in the dark, luminescence was measured using a plate reader. Data were analyzed using GraphPad Prism to calculate IC₅₀ values for each substance.

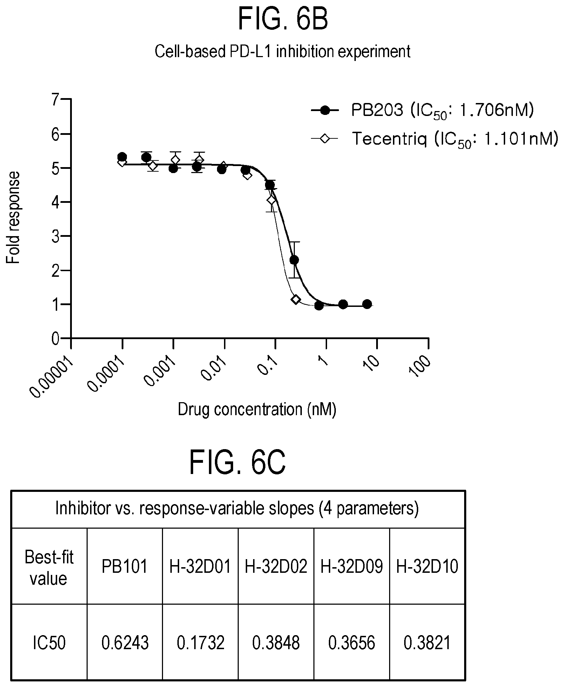

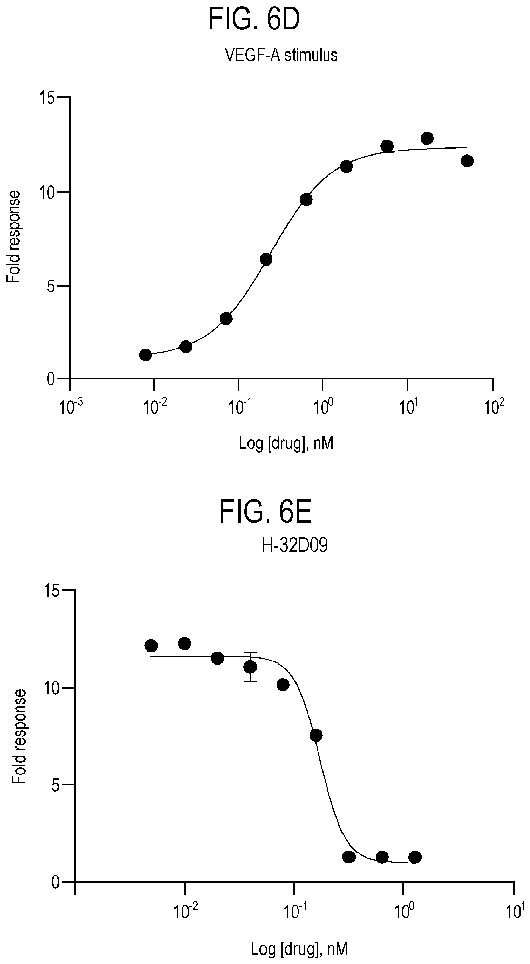

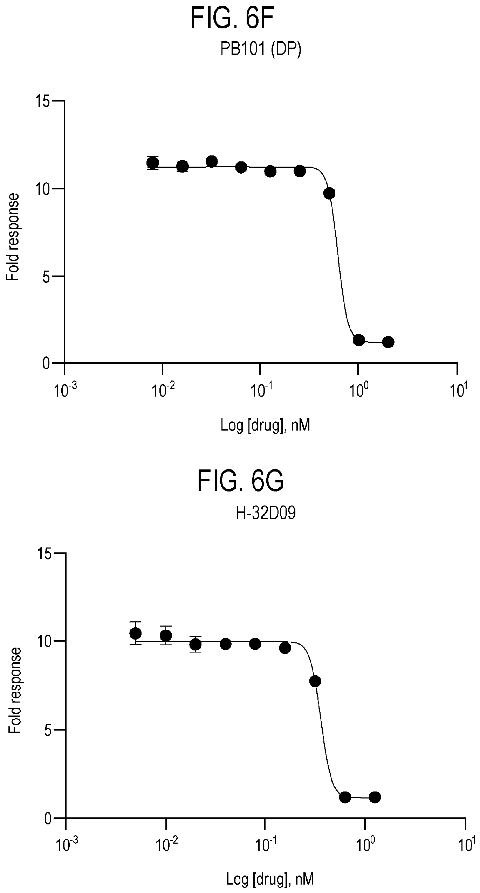

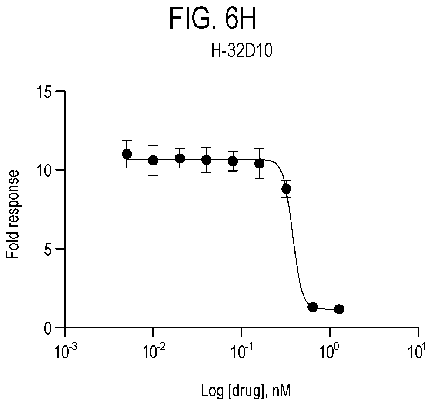

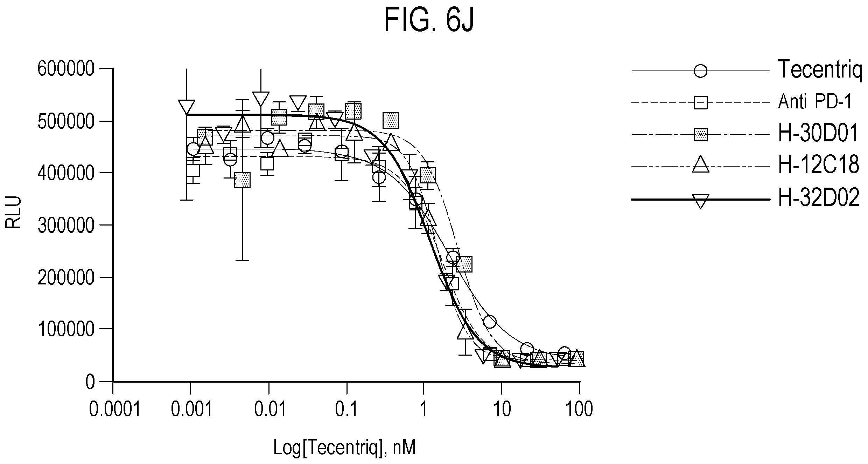

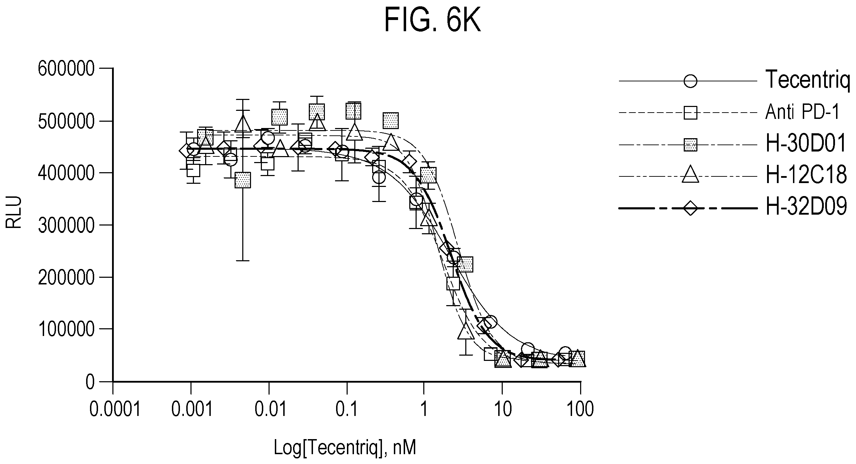

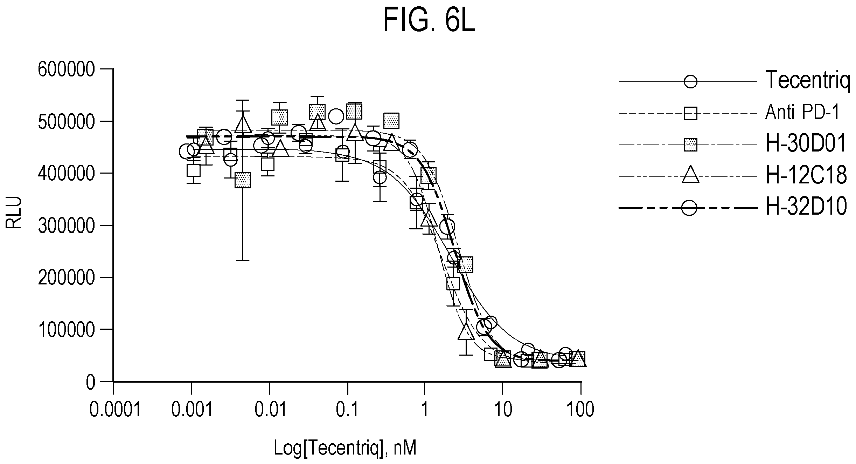

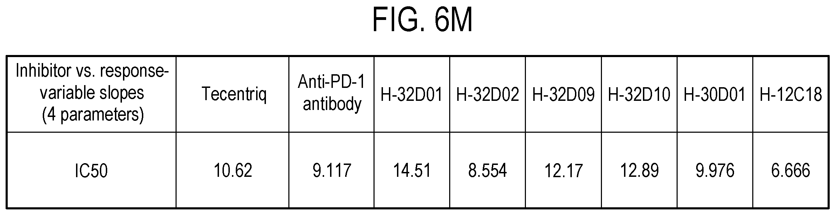

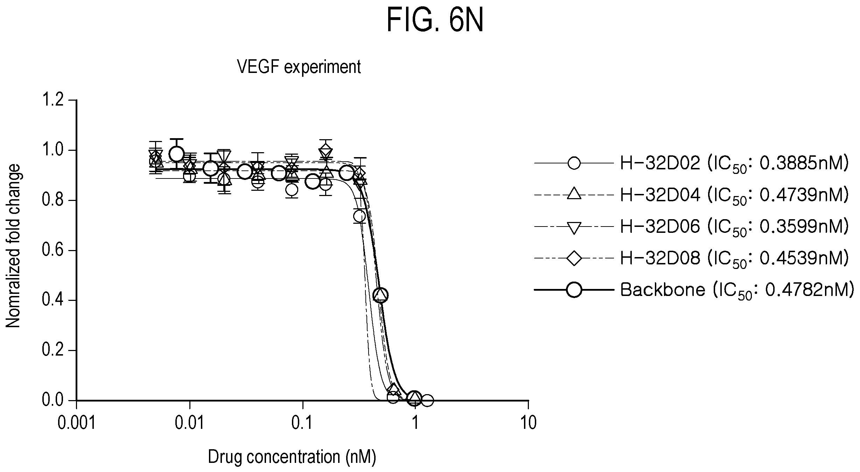

As shown in FIGs. 6c to 6l and FIG. 6n, it was confirmed that the conjugates based on the modified fusion protein of the present disclosure exhibited cell-level VEGF-A signal inhibition comparable to that of the modified fusion protein prior to conjugation, regardless of the type of linker or scFv used. In addition, this inhibitory activity was confirmed to be superior compared with the unmodified fusion protein VEGF-Grab (PB101).

### EXAMPLE 12 - Analysis of PD-1/PD-L1 Signal Inhibitory Activity

To quantitatively analyze inhibitory activity against PD-L1, a genetically engineered cell line (Jurkat PD-1/U2OS PD-L1, Eurofins Discovery) incorporating a luciferase reporter system based on PD-1 and PD-L1 expression and PD-1/PD-L1 interaction was used. Forty microliters of U2OS PD-L1 cells at a density of 1 × 10⁵ cells/mL were dispensed into a 96-well plate, followed by addition of 20 µL of diluted PB203 and control substances, and incubated for 1 hour at 37°C in a 5% CO₂ incubator. Then, 40 µL of Jurkat PD-1 cells at a density of 2.4 × 10⁵ cells/mL were additionally dispensed into the 96-well plate and reacted at room temperature for 2 hours. Thereafter, 10 µL of Detection reagent 1 was added to the plate and incubated for 15 minutes at room temperature in the dark. Subsequently, 40 µL of Detection reagent 2 was added and incubated for 3 hours at room temperature in the dark. Finally, luminescence was measured using a plate reader. Data were analyzed using GraphPad Prism to calculate IC₅₀ values for each substance.

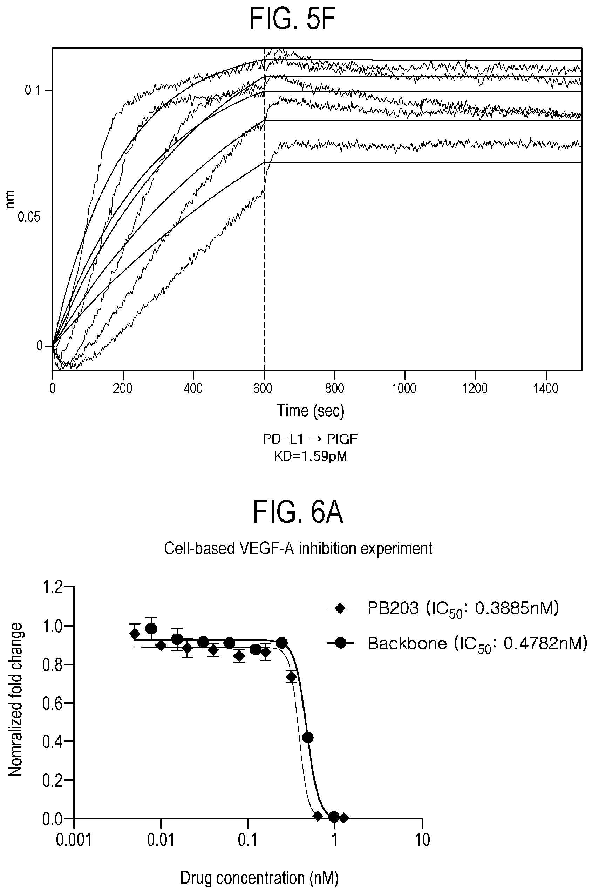

The inhibitory effects of the conjugates of anti-PD-L1 scFv and the modified fusion protein (modified VEGFR1-D2-D3) against VEGF-A and PD-L1 are shown in FIGs. 6a to 6e.

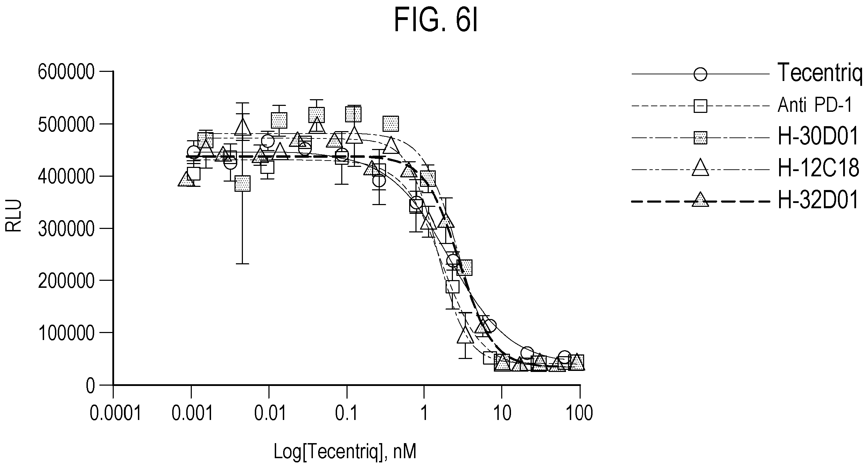

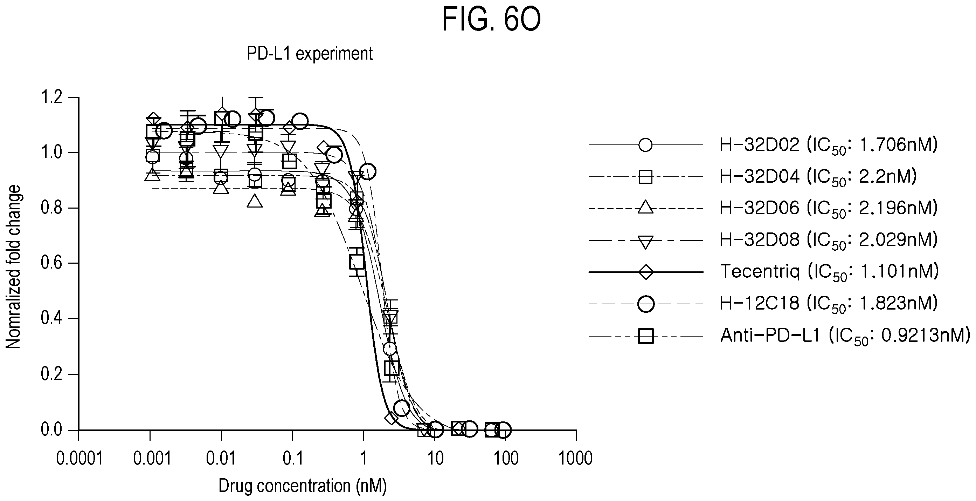

As shown in FIGs. 6i to 6n and FIG. 6o, it was confirmed that the conjugates (H-32D01, H-32D02, H-32D04, H-32D06, H-32D08, H-32D09, H-32D10, etc.) based on the modified fusion protein of the present disclosure exhibited cell-level PD-L1 signal inhibition comparable to that of anti-PD-1 antibodies prior to conjugation, regardless of the type of linker or scFv used.

This demonstrates that, despite their more complex structures, the conjugates based on the modified fusion protein of the present disclosure maintain each function without mutual interference through structural design.

### EXAMPLE 13 - Evaluation of Simultaneous Binding Ability to VEGF-A, PlGF, and PD-L1 Ligands

To evaluate binding affinity depending on the order of binding when two or more ligands among VEGF-A, PlGF, and PD-L1 are bound, an Octet^{®} RED96e instrument (ForteBIO) was used.

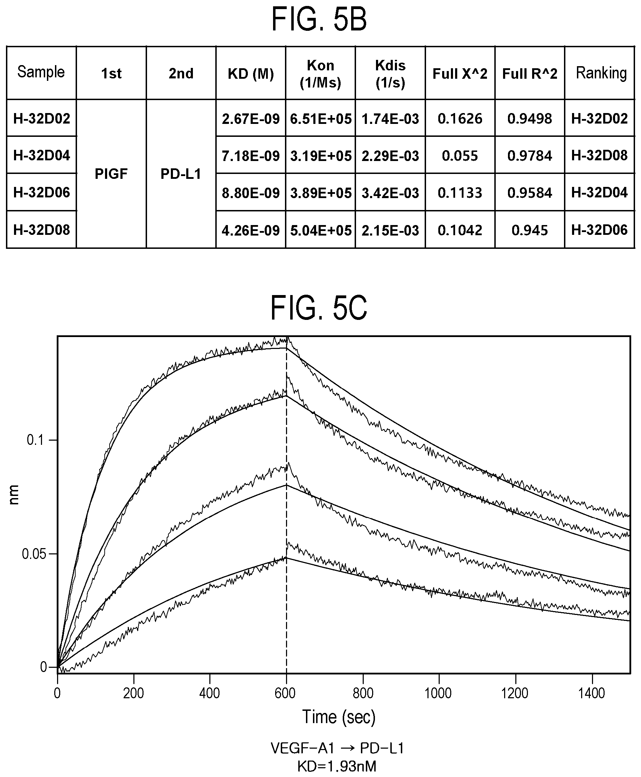

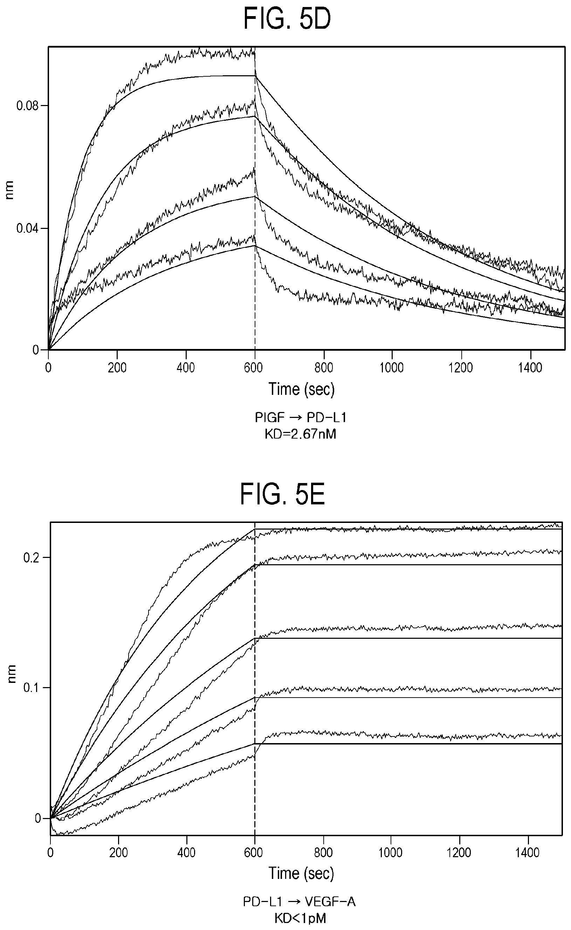

Prior to the binding assay, AHC sensors were equilibrated in 1x KB buffer (ForteBIO). The analyte protein PB203 was immobilized on the sensors at a concentration of 100 nM, followed by washing with 1x KB buffer to remove unbound protein. Subsequently, VEGF-A, PlGF, or PD-L1 was bound at 30 nM (high concentration), and then a different ligand from the previously bound ligand (e.g., VEGF-A → PD-L1, PlGF → PD-L1, PD-L1 → VEGF-A, PD-L1 → PlGF) was introduced in the presence of the previously bound ligand (30 nM) over eight concentration points (30, 15, 7.5, 3.75, 1.875, 0.937, 0.468, and 0 nM), and the binding signals were measured. Among the binding results, four or more concentration points meeting the reliability criteria, Full X² (3 or less) and Full R² ( 0.95 or more), were selected, and binding affinity and other parameters were calculated by global fitting. The resulting simultaneous binding affinities of the conjugate of anti-PD-L1 scFv and the modified VEGFR1-D2-D3 fusion protein to the targets are shown in FIGs. 5a to 5f. As a result of measuring simultaneous antigen binding affinity, it was confirmed that the affinity was maintained with little difference even when the antigen type was varied. Specifically, as shown in FIGs. 5a and 5b, the conjugates based on the modified fusion protein of the present disclosure maintained similar binding affinity to PD-L1 regardless of whether they were bound to VEGF-A or PlGF. In addition, as shown in FIGs. 5c to 5f, the conjugates based on the modified fusion protein of the present disclosure maintained binding affinity comparable to that observed upon binding to a single ligand, regardless of the order or type of ligand binding. This demonstrates that, despite their more complex structures, the conjugates based on the modified fusion protein of the present disclosure maintain each function without mutual interference through structural design.

### EXAMPLE 14 - Pharmacokinetic Evaluation Following Single Intravenous Administration in SD Rats

Seven-week-old male Sprague-Dawley (SD) rats were acclimated for one week prior to the study and then used for the experiment. PB203 was administered as a single intravenous dose at 3 mpk. Blood samples were collected via the jugular vein at designated time points (0, 0.083, 2, 8, 24, 48, 96, 120, 168, and 336 hours), followed by plasma separation.

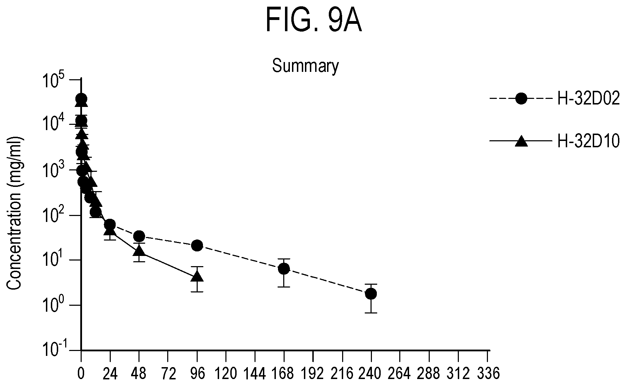

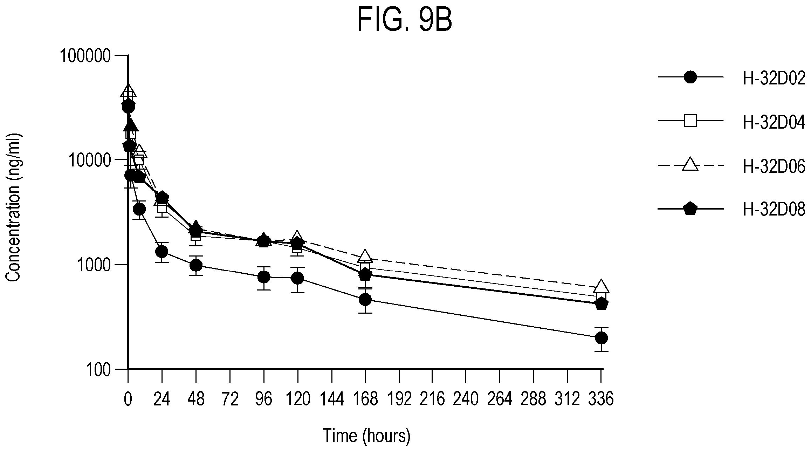

On the day prior to the assay, plates were coated with human PD-L1 (R&D Systems) by overnight incubation at 4°C. After washing with 0.1% PBS-T washing buffer to remove unbound human PD-L1, blocking was performed at room temperature for 1 hour using 0.1% PBS-T containing 5% skim milk. The prepared plasma samples were diluted 20-fold with blocking buffer, and 100 µL of each sample was added to each well and incubated at room temperature for 2 hours. The reaction solution was then removed, and the plates were washed with washing buffer. A detection antibody, HRP goat anti-human IgG Fc cross-adsorbed secondary antibody (Invitrogen), was reacted at room temperature for 1 hour, followed by washing to remove unreacted detection antibody. Then, 100 µL of 3,3',5,5'-tetramethylbenzidine (TMB) solution (Sigma) was added and reacted at room temperature for 10 minutes, after which a stop solution (Sigma) was added to terminate the reaction. Absorbance was measured at 450 nm using a plate reader. A quantitative standard was prepared using PB203 by serial dilution from 62.5 to 0.48, and a standard curve was generated based on the measured absorbance (OD450). Plasma concentrations of PB203 were calculated by back-calculation using the derived equation, and PK parameters were finally calculated using the time-dependent concentration results. First, to verify pharmacokinetic profiles according to scFv type, H-32D02 and H-32D10, which commonly used linker V3 and were conjugated with A167 or atezolizumab scFv, respectively, were evaluated. As shown in FIG. 9a, the A167 conjugate exhibited superior in vivo pharmacokinetic behavior, and Fc variants thereof were subsequently prepared. Four Fc variants containing wild-type Fc for the A167 conjugate were prepared and evaluated. As shown in FIG. 9b, the H-32D04, H-32D06, and H-32D08 conjugates exhibited superior in vivo pharmacokinetic profiles compared with the H-32D02 conjugate. The in vivo pharmacokinetic profiles among these three conjugates were similar to one another.

### EXAMPLE 15 - Antitumor Efficacy Study in Mouse Model

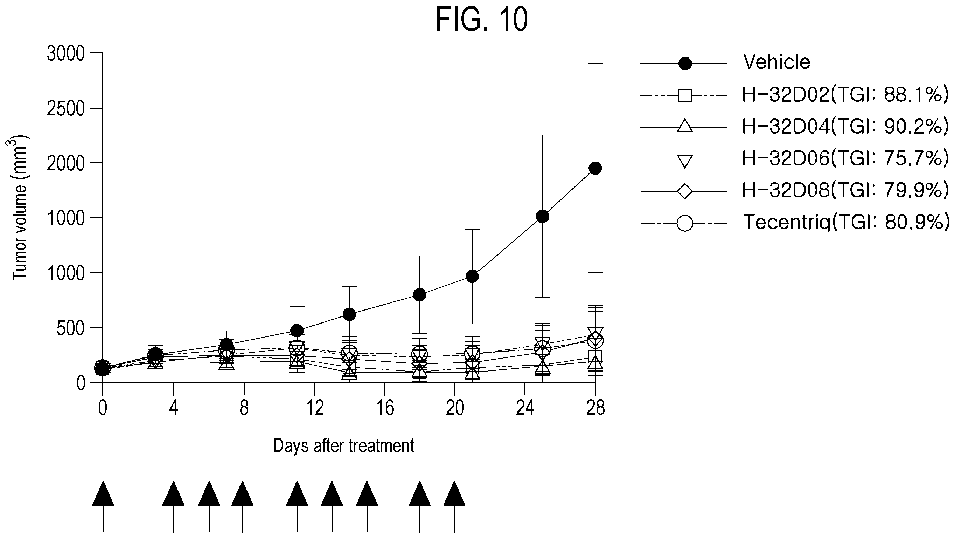

Genetically engineered mice (B6/JGpt-Cd274tm1(hCD274)/Gpt) were subcutaneously implanted with the genetically modified cell line MC-38-hPD-L1, and animals were randomized when tumor volume reached approximately 100 mm³. Thereafter, PB203 candidate substances and control drugs were administered intraperitoneally at a dose of 10 mpk three times per week, and tumor volume and weight were measured over three weeks. This study was conducted in compliance with animal ethics regulations. The results are shown in FIG. 10. As shown in FIG. 10, among the conjugates of the present disclosure, those containing wild-type Fc and Fc variants with suppressed ADCC/CDC activation exhibited tumor growth inhibition (TGI) superior by approximately 10% or more compared with the marketed antibody Tecentriq, and the other two Fc variants also showed high levels of tumor growth inhibition. In particular, H-32D04 exhibited complete regression in approximately 40% of the subjects, which is a superior result compared with Tecentriq, in which no complete regression was observed.

From the above description, a person or ordinary skill in the art to which the present disclosure pertains will be able to understand that the present disclosure may be implemented in other specific forms without departing from the technical spirit or essential features thereof. In this regard, the embodiments described above are to be understood in all respects as illustrative and not restrictive. The scope of the present disclosure should be construed not by the foregoing detailed description but by the meaning and scope of the claims set forth below, and all changes or modifications derived from the equivalent concepts thereof are to be included within the scope of the present disclosure.

## Claims

1. A conjugate, comprising: a programmed cell death protein ligand 1 (PD-L1)-targeting scFv; and a fusion protein comprising a VEGFR1 (vascular endothelial growth factor receptor 1) extracellular domain, a linker, and a multimerization domain, wherein the PD-L1-targeting scFv is fused to the N-terminus of the fusion protein.

2. The conjugate of claim 1,
wherein the fusion protein comprises a VEGFR1 extracellular domain, a linker, and a multimerization domain,
wherein the VEGFR1 extracellular domain comprises an immunoglobulin (Ig)-like domain D2 and an Ig-like domain D3 of VEGFR1, the linker is located between the Ig-like domain D3 and the multimerization domain, and
the fusion protein is a modified fusion protein having one or more of the following characteristics (a) to (c):
(a) one or more amino acid substitutions selected from K241E, L243S, R244V, and H246E on a β1-β2 loop of domain D3, an amino acid substitution of L258A, L258S, or L258D on a β2-β3 loop of domain D3, and one or more amino acid substitutions selected from K300G, Q302T, and K304S on a β5-β6 loop of domain D3,
wherein the β1-β2 loop of domain D3 comprises amino acid residues T236 to T247 of the amino acid sequence of VEGFR1 comprising SEQ ID NO: 41, the β2-β3 loop comprises amino acid residues T256 to V262 of the amino acid sequence of VEGFR1, and the β5-β6 loop comprises amino acid residues D299 to L308 of the amino acid sequence of VEGFR1;
(b) the linker has a length of about 14 to about 35 amino acids; and
(c) a disulfide bond is present in the fusion protein, and among amino acid residues on the β1-β2 loop of domain D3, L243 and one of amino acid residues located at positions -2, -1, 0, +1, +2, and +3 relative to Y329 of domain D3 are substituted with cysteine.

3. The conjugate of claim 2, wherein the characteristic (a) of the fusion protein comprises:
amino acid substitutions of K241E, L243S, R244V, and H246E on the β1-β2 loop of domain D3; an amino acid substitution of L258A, L258S, or L258D on the β2-β3 loop of domain D3; and amino acid substitutions of K300G, Q302T, and K304S on the β5-β6 loop of domain D3.

4. The conjugate of claim 2, wherein the modified fusion protein has an N-terminus of domain D2 that starts with an amino acid sequence EF.

5. The conjugate of claim 2, wherein in characteristic (b) of the modified fusion protein, the linker comprises a glycine-serine (GS) repeat sequence, and the GS repeat sequence is a 2 to 35 amino acids long amino acid sequence consisting of glycine (G) and serine (S).

6. The conjugate of claim 5, wherein one or more glycine (G) residues included in the GS repeat sequence are substituted with cysteine (C).

7. The conjugate of claim 2, wherein in characteristic (b) of the modified fusion protein, the linker comprises an amino acid sequence of a hinge region derived from an immunoglobulin, and the amino acid sequence of the hinge region may be modified.

8. The conjugate of claim 7, wherein the hinge region derived from an immunoglobulin is derived from human IgD and/or IgG.

9. The conjugate of claim 7, wherein in characteristic (b) of the modified fusion protein, a papain recognition site or a glycosylation site present in the hinge region derived from an immunoglobulin has an amino acid variation.

10. The conjugate of claim 2, wherein in characteristic (b) of the modified fusion protein, the linker sequentially comprises, from the N-terminus toward the C-terminus, (i) an amino acid sequence selected from the group consisting of CS, CSSG, CS(GGGGS), CS(GGGGS)₃, C(GSSG)₂, GS, GSSG, (GSSG)₂, GGGGS, (GGGGS)₄, GS(GGGGS), and GS(GGGGS)₃; and (ii) a hinge region derived from an immunoglobulin selected from the group consisting of human IgG1, IgG4, or IgD/G1,
wherein the amino acid sequence of the hinge region is modifiable.

11. The conjugate of claim 2, wherein in characteristic (b) of the modified fusion protein, the linker is an amino acid sequence selected from the group consisting of CSSGDATPTSPPSP, CSKVDKKVEPKSSDTPPTCPPCP, CSGGGGSAEPKAGDATPPTCPPCP, CSGGGGSGGGGSGGGGSAESKYGPPCPPCP, CSNTGSGGEEKKKEKEKEEQEERSSDTPPTCPPCP, CSNTGSGGEEKKKEKEKEEQEERSCDTPPTCPPCP, CGSSGGSSGEPKSDATPTCPPCP, and CSKVDKKVEPKSSDKTYTCPPCP.

12. The conjugate of claim 2, wherein the modified fusion protein:
(a) comprises amino acid substitutions of K241E, L243S, R244V, and H246E on the β-β2 loop of domain D3, an amino acid substitution of L258A, L258S, or L258D on the β2-β3 loop of domain D3, and amino acid substitutions of K300G, Q302T, and K304S on the β5-β6 loop of domain D3;
(b) has a linker that is an amino acid sequence selected from the group consisting of CSSGDATPTSPPSP, CSKVDKKVEPKSSDTPPTCPPCP, CSGGGGSAEPKAGDATPPTCPPCP, CSGGGGSGGGGSGGGGSAESKYGPPCPPCP, CSNTGSGGEEKKKEKEKEEQEERSSDTPPTCPPCP, CSNTGSGGEEKKKEKEKEEQEERSCDTPPTCPPCP, CGSSGGSSGEPKSDATPTCPPCP, and CSKVDKKVEPKSSDKTYTCPPCP; and
(c) contains cysteines substituted for L243 and an amino acid residue located at position +2 relative to Y329 of domain D3.

13. The conjugate of claim 2, wherein the modified fusion protein consists of an amino acid sequence selected from the group consisting of SEQ ID NOs: 44 to 58.

14. The conjugate of claim 2, wherein the multimerization domain is:
(a) an Fc region of an immunoglobulin;
(b) a CH3 region of IgG1 or IgG4;
(c) CH2 and CH3 regions of IgG1 or IgG4;
(d) an Fc region of an immunoglobulin comprising an amino acid sequence having at least 85% identity to the amino acid sequence of SEQ ID NO: 59; or
(e) an Fc region of an immunoglobulin comprising the amino acid sequence of SEQ ID NO: 60.

15. The conjugate of claim 14, wherein the multimerization domain comprises an IgG1 Fc region consisting of the amino acid sequence of SEQ ID NO: 60.

16. The conjugate of claim 2, wherein the multimerization domain has:
(i) one or more selected from the group consisting of a T20Q amino acid substitution, a D126E amino acid substitution, an L128M amino acid substitution, an M198L amino acid substitution, and a K217 amino acid deletion, or
(ii) one or more selected from the group consisting of an L4A amino acid substitution, an L5A amino acid substitution, an H38Q amino acid substitution, a K44Q amino acid substitution, a Y66F amino acid substitution, an A97G amino acid substitution, an A100S amino acid substitution, a P101S amino acid substitution, a R125Q amino acid substitution, a D126E amino acid substitution, an L128M amino acid substitution, a K179R amino acid substitution, a Q189E amino acid substitution, a P215L amino acid substitution, and a K217 amino acid deletion
based on SEQ ID NO: 60.

17. The conjugate of claim 2, wherein the modified fusion protein is in dimer or multimer form.

18. The conjugate of claim 1, wherein the PD-L1-targeting scFv is an atezolizumab scFv or an anti-PD-L1 scFv comprising the amino acid sequence of SEQ ID NO: 40.

19. A pharmaceutical composition comprising the conjugate according to any one of claims 1 to 18 as an active ingredient for preventing or treating chronic infection, tissue allograft, autoimmune disease, inflammatory disease, neoplastic disease, cancer, angiogenesis-related disease, or ocular disease,.

20. A nucleic acid molecule encoding the conjugate according to any one of claims 1 to 18.

21. A host cell comprising a nucleotide sequence encoding the conjugate according to any one of claims 1 to 18.

22. A vector comprising a nucleotide sequence encoding the conjugate according to any one of claims 1 to 18.

23. The vector of claim 22, which is a recombinant viral vector.

24. A pharmaceutical composition for delivering a viral vector to a subject, comprising the recombinant viral vector of claim 23, wherein the fusion protein encoded by the recombinant viral vector is expressed in the subject, and the pharmaceutical composition is for preventing or treating an autoimmune disease, an inflammatory disease, a neoplastic disease, cancer, an angiogenesis-related disease, or an ocular disease.

25. The pharmaceutical composition of claim 24, wherein the recombinant viral vector is a recombinant adeno-associated virus vector.

26. A method for preventing or treating one or more selected from the group consisting of chronic infection, tissue allograft, autoimmune disease, inflammatory disease, neoplastic disease, cancer, angiogenesis-related disease, and ocular disease, the method comprising administering, to a subject in need thereof, the conjugate according to any one of claims 1 to 18 and/or the recombinant viral vector of claim 23.

27. Use of the conjugate according to any one of claims 1 to 18 and/or the recombinant viral vector of claim 23 for preventing or treating one or more selected from the group consisting of chronic infection, tissue allograft, autoimmune disease, inflammatory disease, neoplastic disease, cancer, angiogenesis-related disease, and ocular disease.
